(19) Europäisches Patentamt
European Patent Office
Office européen des brevets

(11) EP 2 012 579 B1

(12) **FASCICULE DE BREVET EUROPEEN**

(45) Date de publication et mention de la délivrance du brevet:
**28.08.2013 Bulletin 2013/35**

(21) Numéro de dépôt: **07731968.9**

(22) Date de dépôt: **30.04.2007**

(51) Int Cl.:
*A01H 1/00* (2006.01) *A01H 1/04* (2006.01)
*A01H 4/00* (2006.01) *A01H 5/00* (2006.01)
*C12N 15/00* (2006.01) *C12N 9/88* (2006.01)

(86) Numéro de dépôt international:
**PCT/FR2007/051197**

(87) Numéro de publication internationale:
**WO 2007/125264 (08.11.2007 Gazette 2007/45)**

(54) **SYSTÈME GÉNÉTIQUE POUR LE CONTRÔLE DU DÉVELOPPEMENT DU TYPE FLORAL D'UNE PLANTE DICOTYLÉDONE, ET MISE EN OEUVRE DANS DES PROCÉDÉS DE DÉTECTION ET DE SÉLECTION**

GENETISCHES SYSTEM FÜR DIE KONTROLLE DER BLÜTENENTWICKLUNG EINER ZWEIKEIMBLÄTTRIGEN PFLANZE SOWIE DURCHFÜHRUNG VON NACHWEIS- UND SELEKTIONSVERFAHREN

GENETIC SYSTEM FOR CONTROLLING THE FLORAL DEVELOPMENT OF A DICOTYLEDON PLANT, AND IMPLEMENTATION IN DETECTION AND SELECTION PROCESSES

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **28.04.2006 FR 0651538**

(43) Date de publication de la demande:
**14.01.2009 Bulletin 2009/03**

(73) Titulaire: **INSTITUT NATIONAL DE LA RECHERCHE AGRONOMIQUE (INRA)**
**75341 Paris Cedex 07 (FR)**

(72) Inventeurs:
- **BENDAHMANE, Abdelhafid**
**91830 Le Coudray Montceaux (FR)**
- **BOUALEM, Adnane**
**03300 Cusset (FR)**
- **FERGANY, Mohamed**
**Le Caire (EG)**
- **DOGIMONT, Catherine**
**84270 Vedene (FR)**

(74) Mandataire: **Catherine, Alain et al**
**Cabinet HARLE et PHELIP**
**14-16, rue Ballu**
**75009 Paris (FR)**

(56) Documents cités:
**WO-A-87/01905 US-A1- 2003 154 521**

- **PAPADOPOULOU EKATERINA ET AL: "Effect of modified endogenous ethylene production on sex expression, bisexual flower development and fruit production in melon (Cucumis melo L.)" SEXUAL PLANT REPRODUCTION, vol. 18, no. 3, novembre 2005 (2005-11), pages 131-142, XP002408826 ISSN: 0934-0882**
- **PAPADOPOULOU EKATERINI ET AL: "INFLUENCE OF HETEROLOGOUS ACC SYNTHASE GENE ON SEX EXPRESSION OF MELON" HORTSCIENCE, AMERICAN SOCIETY OF HORTICULTURAL SCIENCE, ALEXANDRIA, VA, US, vol. 36, no. 3, 25 juillet 2001 (2001-07-25), page 455,POSTER8, XP008071541 ISSN: 0018-5345**
- **NOGUERA F J ET AL: "Development and mapping of a codominant SCAR marker linked to the andromonoecious gene of melon" THEORETICAL AND APPLIED GENETICS, vol. 110, no. 4, février 2005 (2005-02), pages 714-720, XP002408832 ISSN: 0040-5752**
- **SILBERSTEIN LEAH ET AL: "Linkage map of Cucumis melo including phenotypic traits and sequence-characterized genes." GENOME, vol. 46, no. 5, octobre 2003 (2003-10), pages 761-773, XP008071831 ISSN: 0831-2796**
- **YAMASAKI SEIJI ET AL: "Hormonal regulation of sex expression in plants." VITAMINS AND HORMONES. 2005, vol. 72, 2005, pages 79-110, XP008071798 ISSN: 0083-6729**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **RAM DANGAR ET AL: "Inheritance of gynoecism in bitter gourd (Momordica charantia L.)" JOURNAL OF HEREDITY, vol. 97, no. 3, mai 2006 (2006-05), pages 294-295, XP008071801 ISSN: 0022-1503**
- **YE BO-PING ET AL: "STUDIES ON A GYNOECIOUS-SPECIFIC ACC SYNTHASE GENE IN DIFFERENT SEXUAL PHENOTYPES OF CUCUMBER GENOME" ACTA BOTANICA SINICA, PLENUM/CHINA PROGRAM, NEW YORK,, US, vol. 42, no. 2, février 2000 (2000-02), pages 164-168, XP008071543 ISSN: 0095-4195**
- **WITKOWICZ JUSTYNA ET AL: "AFLP MARKER POLYMORPHISM IN CUCUMBER (CUCUMIS SATIVUS L.) NEAR ISOGENIC LINES DIFFERING IN SEX EXPRESSION" CELLULAR AND MOLECULAR BIOLOGY LETTERS, UNIVERSITY OF WROCAW. INSTITUTE OF BIOCHEMISTRY, WROCAW, PL, vol. 8, no. 2, 2003, pages 375-381, XP008072069 ISSN: 1425-8153**
- **MIBUS H ET AL: "Molecular characterization and isolation of the F/f gene for femaleness in cucumber (Cucumis sativus L.)" THEORETICAL AND APPLIED GENETICS, vol. 109, no. 8, novembre 2004 (2004-11), pages 1669-1676, XP002408828 ISSN: 0040-5752**
- **PRZYBECKI ZBIGNIEW ET AL: "THE ISOLATION OF CDNA CLONES FROM CUCUMBER (CUCUMIS SATIVUS L.) FLORAL BUDS COMING FROM PLANTS DIFFERING IN SEX" CELLULAR AND MOLECULAR BIOLOGY LETTERS, UNIVERSITY OF WROCAW. INSTITUTE OF BIOCHEMISTRY, WROCAW, PL, vol. 8, no. 2, 2003, pages 421-438, XP008071825 ISSN: 1425-8153**
- **BOUALEM ADNANE ET AL: "A Conserved Ethylene Biosynthesis Enzyme Leads to Andromonoecy in Two Cucumis Species", PLOS ONE, vol. 4, no. 7, July 2009 (2009-07), page Article No.: e6144, ISSN: 1932-6203**


Remarques:

Le dossier contient des informations techniques présentées postérieurement au dépôt de la demande et ne figurant pas dans le présent fascicule.

**Description**

## DOMAINE DE L'INVENTION

La présente invention se rapporte au domaine de la sélection de variétés de plantes, et en particulier à la sélection du type sexuel des plantes. Elle est relative à la détection génotypique du sexe des plantes par analyse du polymorphisme d'un gène A, ainsi qu'à des moyens de mise en oeuvre de cette détection et à des procédés d'obtention de plantes dont le phénotype sexuel est modifié.

## ART ANTERIEUR

La réalisation de plantes hybrides est d'un grand intérêt en agronomie et en agriculture. En effet, les plantes hybrides, grâce au phénomène d'hétérosis appelé également vigueur hybride, présentent une supériorité pour de nombreux caractères, par rapport à la moyenne de leurs deux parents. Cette supériorité peut s'illustrer par exemple par une meilleure vigueur, un meilleur rendement, une plus grande adaptation au milieu dans lequel l'hybride est cultivé, et une grande uniformité des hybrides par rapport à ses parents. Cette vigueur hybride est d'autant plus importante que les parents sont éloignés génétiquement.

La création de lignées pures et stables, futurs parents de l'hybride est un passage obligé pour la création de variété hybrides homogènes et reproductibles exprimant le plus d'hétérosis. Il est donc nécessaire de créer des lignées pures et stables, puis de croiser ces lignées pour obtenir des hybrides.

La création de lignées pures implique l'autofécondation d'une plante de sorte à obtenir des plantes présentant un même patrimoine génétique, fixé pour l'ensemble des caractères de productivité, régularité du rendement ou encore de résistance aux maladies, recherchées.

Pour créer des lignées pures, Il est donc nécessaire d'utiliser des plantes dont le type sexuel permet l'autofécondation, par exemple des plantes hermaphrodites.

Or, beaucoup de plantes dicotylédones, et en particulier les cucurbitaceae peuvent être monoïque, andromonoïque, gynoïques ou hermaphrodites.

Il a été montré que l'expression constitutive dans des plants de melons transgéniques d'une protéine ACS (1-aminocyclopropane-1-carboxylate synthase) du pétunia augmentait le nombre de fleurs hermaphrodites des plants andromonoïques mais n'entraînait pas de changement de sexe des plants (Papadopoulou et al., Sexual Plant reproduction, 2005, 18(3):131-142).

Chez le melon, l'existence des allèles A/a ainsi que les phénotypes associés est mentionnée dans les articles de Silberstein et al. (Genome. 2003, 46(5):761-73) et Noguera et al. (Theor Appl Genet. 2005, 110(4):714-20). Néanmoins ces documents ne fournissent aucune information sur la localisation précise et la structure de ces allèles.

Une première technique mise en oeuvre, pour l'obtention de lignées pures, consiste à réaliser un traitement chimique des plantes de sorte à obtenir des plantes aptes à s'autoféconder, par exemple des plantes hermaphrodites. Chez le melon (*cucumis melo*) par exemple, la pulvérisation d'inhibiteurs de la synthèse de l'éthylène tel que le nitrate d'argent ou le thiosulfate d'argent entraîne l'apparition temporaire d'étamines dans les fleurs femelles (Rudich *et al.*, 1969 ; Risser et al.,1979). De cette manière, la transformation des plantes gynoïques en plantes hermaphrodites est utilisée pour le maintien de lignées pures.

Cependant, la production de lignées pures par cette méthode est limitée par le coût des agents chimiques, leur durée d'action, et leurs effets phytotoxiques. De plus, de tels agents peuvent ne pas être efficaces en ce qui concerne la réalisation d'hybrides à partir de plantes dont la durée de floraison est longue, car de nouvelles fleurs apparues après traitement pourraient ne pas être affectées par le traitement chimique.

Il existe donc un besoin pour un système qui permettrait de contrôler le développement du type floral d'une plante dicotylédone, et d'obtenir une plante d'un type floral déterminé.

De plus, de très nombreux croisements sont nécessaires pour obtenir des hybrides intéressants, à partir de lignées pures, et à chaque croisement, les plants présentant le phénotype le plus prometteur sont retenus.

Lors du croisement des lignées pures entre elles, il est indispensable de pouvoir choisir le sens du croisement effectué, et d'éviter l'auto pollinisation des plantes qui conduirait à des plantes ne présentant pas la vigueur hybride recherchée.

Là encore, du fait de la diversité du type sexuel des plantes dicotylédones, il est nécessaire de séparer les fleurs mâles et les fleurs femelles d'un même plant pour éviter l'autopollinisation.

Une première technique, mise en oeuvre notamment pour le maïs, consiste à utiliser des moyens mécaniques pour réaliser une émasculation des plantes. Cependant cette technique s'avère extrêmement coûteuse puisqu'elle nécessite l'émasculation de chaque plante dont on veut éviter l'autopollinisation, pour chaque croisement effectué.

Une autre technique consiste à réaliser une émasculation chimique des plantes, bloquant la formation de pollen viable. Ainsi, chez le melon (*Cucumis melo),* le traitement de plantes monoïques par de l'éthrel (précurseur de l'éthylène) entraîne la disparition temporaire des fleurs mâles.

De tels agents chimiques, appelés gamétocides, utilisés pour provoquer une stérilité mâle transitoire présentent plusieurs inconvénients, comme un coût élevé ou une grande toxicité, comme cela a été rappelé ci-dessus.

Les techniques mécaniques ou chimiques de contrôle du type floral décrites ci-dessus s'avèrent donc très coûteuses, d'autant que de très nombreux croisements sont nécessaires pour obtenir des plants hybrides présentant les caractères recherchés et pouvant être commercialisés.

Pour faciliter la création de lignées pures et d'hybrides, Il existe donc également un besoin pour un système qui permettrait de contrôler le développement du type floral d'une plante dicotylédone, et d'obtenir une plante d'un type floral déterminé.

Une autre voie pour obtenir des plants capables d'autopollinisation utiles pour la création de lignées pures, ou non capables d'autopollinisation, pour la création d'hybrides, pourrait consister respectivement en une sélection d'individus exclusivement hermaphrodites, ou exclusivement femelles, présents dans une espèce. Cependant, une telle technique s'avèrerait extrêmement croûteuse elle aussi, puisqu'elle nécessiterait la culture d'un nombre très important de plantes, jusqu'au moment où il est possible d'en déterminer le type sexuel. Cette technique serait de plus aléatoire, car les mécanismes de détermination du sexe des fleurs dépendent notamment de facteurs environnementaux.

Il existe donc également un besoin pour un procédé qui permettrait de sélectionner des plantes dycotylédones par exemple hermaphrodites ou femelles, sans devoir pour autant les cultiver.

Ce procédé devrait permettre de sélectionner des plantes, particulièrement utiles pour la réalisation de lignées pures ou d'hybrides comme cela a été précisé ci-dessus.

## SOMMAIRE DE L'INVENTION

L'invention décrit, un système qui permet de contrôler le développement du type floral d'une plante dicotylédone. Les inventeurs ont en effet montré que deux éléments génétiques de contrôle, (A/a) et (G/g) possédant tous deux au moins deux allèles, (A) et (a) pour le premier élément génétique et (G) et (g) pour le second, participent au contrôle du déterminisme du sexe chez les cucurbitacées.

Les inventeurs ont également montré que physiologiquement, les deux allèles (A) et (a), se distinguent par des taux différents d'une nouvelle protéine.

L'invention décrit un système génétique pour le contrôle du développement du type floral d'une plante dicotylédone, ledit système comprenant la combinaison de deux éléments génétiques de contrôle, respectivement :

- un premier élément génétique de contrôle (A/a) présent dans une plante dicotylédone, sous la forme d'un allèle dominant (A), et d'un allèle récessif (a), dans lequel :

    - l'allèle dominant (A), consiste en un acide nucléique (NA) comprenant :

        (i) un polynucléotide régulateur (PA) fonctionnel dans une plante dicotylédone, et
        (ii) un acide nucléique dont l'expression est régulée par le polynucléotide régulateur (PA), ledit acide nucléique codant pour la protéine ACCS de séquence SEQ ID N°3,

    - l'allèle récessif (a), se distingue de l'allèle dominant (A) par :

        (i) un acide nucléique (NA) non présent dans la plante, ou
        (ii) un polynucléotide régulateur (Pa) non fonctionnel dans une plante dicotylédone, ou
        (iii) un acide nucléique codant pour une protéine ACCS non active, ou
        (iv) un polynucléotide régulateur (Pa) non fonctionnel dans une plante dicotylédone, et un acide nucléique codant pour une protéine ACCS non active, et

- un second élément génétique de contrôle (G/g) présent dans une plante dicotylédone, sous la forme d'un allèle dominant (G), et d'un allèle récessif (g), dans lequel :

    - l'allèle dominant (G), consistant en un acide nucléique (NG) dont l'expression conduit au développement d'une plante andromonoique ou monoique, et
    - l'allèle récessif (g), se distingue de l'allèle dominant (G) par :

        (i) un acide nucléique (NG) non présent dans la plante, ou
        (ii) la présence d'un acide nucléique (Ng) dont l'expression, dans une plante dicotylédone, conduit au développement d'une plante hermaphrodite ou gynoïque,

étant entendu que le premier élément génétique de contrôle a été artificiellement introduit dans ladite plante dicotylédone.

L'invention décrit egalement les polynucléotides régulateurs (PA) et (Pa) en tant que tels.

L'invention est également relative à des procédés d'obtention d'une plante transformée dont le phénotype sexuel a été modifié, ainsi que les parties d'une telle plante, notamment ses semences.

L'invention décrit encore la protéine ACCS telle que définie plus en détail ci-après, ou un fragment de cette protéine, ainsi que des anticorps dirigés contre la protéine ACCS.

L'invention décrit également des procédés pour détecter la présence des allèles (A) et (a) dans un échantillon.

## DESCRIPTION DETAILLEE DE L'INVENTION

Les inventeurs ont montré que deux éléments génétiques de contrôle, (A/a) et (G/g) possédant tous deux au moins deux allèles, (A) et (a) pour le premier élément génétique et (G) et (g) pour le second, participent au contrôle du déterminisme du sexe chez les cucurbitacées.

Il a en effet été démontré par les inventeurs que l'allèle (A) contrôle le caractère andromonoïque des plantes, et l'allèle (G) contrôle le caractère gynoïque des plantes, comme cela est illustré dans le tableau 1 ci-dessous.

**Tableau 1**

| Phénotype | génotype | Type de fleurs |
|---|---|---|
| Monoïque | AAGG ou Aa GG | Mâles et femelles |
| Andromonoïque | aaGG | Mâles et hermaphrodites |
| Hermaphrodite | aagg | Hermaphrodites |
| Gynoïque | AAgg ou Aagg | Femelles |

Le tableau 1 illustre la correspondance entre le génotype et le phénotype sexuel de fleurs de plantes dicotylédones.

Les inventeurs ont également montré que physiologiquement, les deux allèles (A) et (a), se distinguent par des taux différents d'une nouvelle protéine.

En particulier, Les inventeurs ont montré que physiologiquement, chez *Cucumis melo,* les deux allèles (A) et (a), se distinguent par des taux différents d'une nouvelle protéine, de type ACCS, impliquée dans le métabolisme de l'éthylène.

Or, différentes études ont montré que les gènes de la biologie florale des cucurbitacées codent pour des protéines impliquées dans la voie de biosynthèse ou de régulation de l'éthylène (Kamachi *et al.,* 1997 ; Kahana *et al.,* 2000).

Les inventeurs ont montré que l'allèle (a) se distingue physiologiquement de l'allèle (A) par un taux de protéine ACCS faible dans la plante, comparé à celui d'une plante portant un allèle (A).

Du point de vue génétique, les inventeurs ont montré que l'allèle (A) se distingue de l'allèle (a) par une différence au niveau de la séquence promotrice de la séquence codant pour la protéine ACCS. C'est cette différence qui, du point de vue des inventeurs, induit un taux de protéine distinct pour les deux allèles (A) et (a) et, un phénotype sexuel distinct.

Les inventeurs ont également montré dans l'exemple 1 que l'allèle (A) se distingue également de l'allèle (a) par une différence au niveau de la séquence de la protéine ACCS elle-même. En effet l'allèle (A) est associé à la présence d'un résidu alanine en position 57 de la séquence codant pour la protéique ACCS, et l'allèle (a) est associé à la présence d'un résidu valine en position 57 de cette même séquence.

Les inventeurs ont également montré dans l'exemple 2 que cette différence au niveau de la région promotrice, et dans la séquence de la protéine elle-même se traduit par une expression temporelle et spatiale distincte de la protéine ACCS pour les deux allèles.

Sans vouloir être liés à une quelconque théorie les inventeurs pensent que le système de contrôle objet de l'invention peut être généralisé à toute plante dicotylédone, pour laquelle la détermination du sexe est dépendante du taux d'éthylène comme cela a été rappelé ci-dessus.

En effet, les inventeurs ont montré dans l'exemple 3 que l'expression de l'allèle (A) ou de l'allèle (a) dans une plante dicotylédone n'appartenant pas à la famille des cucurbitacées permet d'obtenir respectivement un phénotype gynoïque ou hermaphrodite.

Enfin, les inventeurs ont identifié que l'allèle (A) est dominant sur l'allèle (a).

L'invention décrit un système génétique pour le contrôle du développement du type floral d'une plante dicotylédone, ledit système comprenant la combinaison de deux éléments génétiques de contrôle, respectivement :

- un premier élément génétique de contrôle (A/a) présent dans une plante dicotylédone, sous la forme d'un allèle dominant (A), et d'un allèle récessif (a), dans lequel :

- l'allèle dominant (A), consiste en un acide nucléique (NA) comprenant :

  (i) un polynucléotide régulateur (PA) fonctionnel dans une plante dicotylédone, et
  (ii) un acide nucléique dont l'expression est régulée par le polynucléotide régulateur (PA), ledit acide nucléique codant pour la protéine ACCS de séquence SEQ ID N°3,

- l'allèle récessif (a), se distingue de l'allèle dominant (A) par :

  (i) un acide nucléique (NA) non présent dans la plante, ou
  (ii) un polynucléotide régulateur (Pa) non fonctionnel dans une plante dicotylédone, ou
  (iii) un acide nucléique codant pour une protéine ACCS non active, ou
  (iv) un polynucléotide régulateur (Pa) non fonctionnel dans une plante dicotylédone, et un acide nucléique codant pour une protéine ACCS non active, et

- un second élément génétique de contrôle (G/g) présent dans une plante dicotylédone, sous la forme d'un allèle dominant (G), et d'un allèle récessif (g), dans lequel :

  - l'allèle dominant (G), consistant en un acide nucléique (NG) dont l'expression conduit au développement d'une plante andromonoique ou monoique, et
  - l'allèle récessif (g), se distingue de l'allèle dominant (G) par :

    (i) un acide nucléique (NG) non présent dans la plante, ou
    (ii) la présence d'un acide nucléique (Ng) dont l'expression, dans une plante dicotylédone, conduit au développement d'une plante hermaphrodite ou gynoïque,

étant entendu que le premier élément génétiques de contrôle a été artificiellement introduit dans ladite plante dicotylédone.

Le système de contrôle génétique, identifié par les inventeurs, permet de contrôler, et/ou de modifier le sexe des fleurs de plantes dicotylédones, et est donc très avantageux, par rapport aux systèmes de contrôle mécaniques, souvent coûteux, ou chimiques, souvent toxiques, tels que décrits dans la partie introductive.

Par « allèle », on entend au sens de la présente invention, l'une des formes d'un gène occupant un site ou locus sur une paire de chromosomes homologues. Les allèles d'un gène se rapportent au même trait génétique mais peuvent déterminer des phénotypes différents.

Un allèle dominant est un allèle dont le niveau d'expression phénotypique est beaucoup plus important que celui de l'allèle homologue (dit récessif). La dominance peut être complète ou partielle.

Un allèle récessif est un allèle ne s'exprimant dans le phénotype que lorsque la plante reçoit les allèles identiques de chacun de ses deux parents. En revanche, l'expression de l'allèle récessif est masquée si l'allèle homologue dominant est présent.

Ainsi, le système défini ci-dessus existe sous la forme de différents états correspondants chacun à un phénotype.

Lorsque le premier élément génétique de contrôle (A/a) présent dans une plante dicotylédone est sous forme d'allèle (A), la plante est de phénotype monoïque ou gynoïque.

Lorsque le premier élément génétique de contrôle (A/a) présent dans une plante est sous forme d'allèle (a), la plante est de phénotype hermaphrodite ou andromonoïque.

Lorsque le second élément génétique de contrôle (G/g) présent dans une plante dicotylédone est sous forme d'allèle (G), la plante est de phénotype monoïque ou andromonoïque.

Lorsque le second élément génétique de contrôle (G/g) présent dans une plante est sous forme d'allèle (g), la plante est de phénotype hermaphrodite ou gynoïque.

La correspondance entre allèles et phénotypes et résumée dans le tableau 1.

La suite de la description expose des variantes, ou modes de réalisation préférés des premier et second éléments génétiques de contrôle faisant partie du système de contrôle objet de l'invention.

**<u>Elément génétique de contrôle A/a, sous forme d'allèle dominant (A) du système selon l'invention</u>**

De manière générale, l'élément génétique de contrôle A/a, présent dans une plante sous la forme de l'allèle dominant (A), permet d'obtenir un taux plus élevé de protéine ACCS, par rapport au taux observé lorsque l'allèle (A) n'est pas présent dans ladite plante.

Dans la suite de la description, on considère qu'un « taux élevé » de protéine ACCS correspond au taux moyen de la protéine ACCS mesuré dans une plante comprenant l'allèle dominant (A) dans son génome, et qu'un « taux faible » de protéine ACCS correspond au taux moyen de protéine ACCS observé dans une plante ne comprenant pas l'allèle dominant (A) dans son génome.

L'allèle dominant (A), consiste en un acide nucléique (NA) comprenant :

(i) un polynucléotide régulateur (PA) fonctionnel dans une plante dicotylédone, et
(ii) un acide nucléique dont l'expression est régulée par le polynucléotide régulateur (PA), ledit acide nucléique codant pour la protéine ACCS de séquence SEQ ID N°3.

***- polynucléotide régulateur (PA) fonctionnel***

Un polynucléotide régulateur ou promoteur (PA) fonctionnel selon l'invention consiste en un acide nucléique qui permet l'expression de la protéine ACCS de séquence SEQ ID N°3 chez les plantes dicotylédones.

A titre d'exemple un tel promoteur comprend une séquence nucléotidique allant du nucléotide 1 au nucléotide 5906 de la séquence SEQ ID N°1.

Ainsi, l'invention décrit un système génétique de contrôle dans lequel le polynucléotide régulateur (PA) comprend ou consiste en une séquence nucléotidique allant du nucléotide 1 au nucléotide 5906 de la séquence SEQ ID N°1.

L'invention décrit également le polynucléotide régulateur (PA) en tant que tel, défini ci-dessus, ainsi que des fragments de cet acide nucléique, comme cela sera décrit plus en détails dans la partie intitulée « Acides nucléiques selon l'invention »

Un polynucléotide régulateur (PA) fonctionnel selon l'invention peut également consister en un promoteur connu pour diriger l'expression de la séquence d'acide nucléique codant pour la protéine ACCS de façon constitutive ou de façon tissu spécifique.

Un polynucléotide régulateur (PA) fonctionnel selon l'invention peut ainsi être choisi parmi des promoteurs tissu specifique tel que ceux des gènes de la famille des « MADS box » de la classe A B C D et E, tels que décrits par Theiβen et al., 2001 ou tout autre promoteur de gènes homéotiques.

Un polynucléotide régulateur (PA) fonctionnel selon l'invention peut ainsi être choisi parmi :

- le promoteur 35S du virus de la mosaïque du chou-fleur, ou le promoteur 19S ou avantageusement le promoteur constitutif double 35S (pd35S), décrits dans l'article de Kay et al., 1987 ;
- le promoteur actine du riz suivi de l'intron actine de riz (pAR-IAR) contenu dans le plasmide pAct1-F4 décrit par Mc Elroy et al., 1991 ;
- le promoteur constitutif EF-1α du gène codant pour le facteur d'élongation végétale décrit dans la demande PCT n°WO 90/02172 ou encore dans l'article de AXELOS et al. (1989) ;
- le superpromoteur chimérique PSP (NI et al., 1995) constitué de la fusion de trois copies de l'élément d'activité transcriptionnelle du promoteur du gène de la octopin synthase de *Agrobacterium tumefaciens* et de l'élément d'activation de la transcription du promoteur du gène de la mannopin synthase de *Agrobacterium tumefaciens ;* et
- le promoteur ubiquitine du tournesol (BINET et al., 1991) ; - le promoteur de l'ubiquitine 1 de maïs (CHRISTENSEN et al., 1996). le promoteur de l'ubiquitine 1 de maïs (Christensen et al., 1996)

Un polynucléotide régulateur (PA) fonctionnel selon l'invention peut également consister en un promoteur inductible.

Ainsi, l'invention décrit système de contrôle tel que défini ci-dessus, dans lequel le polynucléotide régulateur (PA) est sensible à l'action d'un signal inducteur, et de préférence, dans lequel le polynucléotide régulateur (PA) est un polynucléotide activateur inductible de la transcription ou de la traduction.

Lorsque le polynucléotide régulateur activateur de la transcription ou de la traduction est sensible, directement ou indirectement, à l'action d'un signal inducteur activateur, il s'agit d'un polynucléotide « activateur inductible » au sens de l'invention.

Selon l'invention, un polynucléotide régulateur du type « activateur inductible » est une séquence régulatrice qui n'est activée qu'en présence d'un signal externe. Un tel signal externe peut être la fixation d'un facteur de transcription, la fixation d'un facteur de transcription pouvant être induite sous l'effet du signal inducteur activateur auquel le polynucléotide régulateur est directement ou indirectement sensible.

Lorsqu'une telle construction d'acides nucléiques est utilisée dans un hôte cellulaire, l'expression du polynucléotide codant pour la protéine ACCS selon l'invention peut être induite en mettant en contact l'hôte cellulaire transformé avec le signal inducteur activateur auquel le polynucléotide régulateur activateur est, directement ou indirectement, sensible.

Lorsque l'on recherche l'absence d'expression du polynucléotide codant pour un polypeptide ACCS chez cet hôte cellulaire transformé, il suffit alors d'éliminer ou supprimer la présence du signal inducteur activateur auquel le polynucléotide régulateur de la transcription ou de la traduction est sensible.

L'homme du métier aura recours à ses connaissances générales techniques dans le domaine des polynucléotides régulateurs, en particulier ceux actifs chez les végétaux, pour définir les constructions répondant à la définition du mode de réalisation ci-dessus.

La séquence régulatrice capable de contrôler l'acide nucléique codant pour une protéine ACCS peut être une séquence régulatrice inductible par un métabolite particulier, tel que :

- une séquence régulatrice inductible par les glucocorticoïdes telle que décrite par AOYAMA et al. (1997) ou telle que décrit par McNELLYS et al. (1998);
- une séquence régulatrice inductible par l'éthanol, telle que celle décrite par SALTER et al. (1998) ou encore telle que décrite par CADDICK et al. (1998);
- une séquence régulatrice inductible par la tétracycline telle que celle commercialisée par la Société CLONTECH.
- une séquence de promoteur inductible par un pathogène ou par un métabolite produit par un pathogène.
- une séquence régulatrice de gènes de type PR, inductible par l'acide salicylique ou le BTH ou l'aliette (Gorlach et al., 1996, Molina et al., 1998) ;
- une séquence régulatrice de type récepteur Ecdysone (Martinez et al., 1999) inductible par le tebufeno3ide (référence produit RH5992, commercialisé par ROHM & HAAS) par exemple, appartenant à la famille des dibenzoylhydrazines.

### *-Acides nucléiques codant pour la protéine ACCS*

De préférence, l'acide nucléique codant pour la protéine ACCS comprend, de l'extrémité 5' vers l'extrémité 3', au moins :

(i) une séquence ayant au moins 95% d'identité avec le polynucléotide allant du nucléotide 5907 au nucléotide 6086 de la séquence SEQ ID N°1,
(ii) une séquence ayant au moins 95% d'identité avec le polynucléotide allant du nucléotide 6181 au nucléotide 6467 de la séquence SEQ ID N°1, et
(iii) une séquence ayant au moins 95% d'identité avec le polynucléotide allant du nucléotide 7046 au nucléotide 7915 de la séquence SEQ ID N°1.

## Elément génétique de contrôle A/a, sous forme d'allèle récessif (a) du système selon l'invention

De manière générale, l'élément génétique de contrôle (A/a) sous la forme de l'allèle récessif (a), lorsqu'il est présent dans une plante qui ne possède pas l'allèle dominant (A) dans son génome, ne permet pas d'obtenir un taux de protéine ACCS, aussi élevé que celui obtenu lorsque l'allèle (A) est présent.

On peut donc définir l'allèle (a) comme toute altération du génotype correspondant à l'allèle (A), ne permettant pas d'obtenir un taux d'ACCS aussi élevé que l'allèle (A).

L'allèle récessif (a), se distingue de l'allèle dominant (A) par :

(i) un acide nucléique (NA) non présent dans la plante, ou
(ii) un polynucléotide régulateur (Pa) non fonctionnel dans une plante dicotylédone, ou
(iii) un acide nucléique codant pour une protéine ACCS non active, ou
(iv) un polynucléotide régulateur (Pa) non fonctionnel dans une plante dicotylédone, et un acide nucléique codant pour une protéine ACCS non active.

### *- Polynucléotide régulateur (Pa) non fonctionnel*

Un polynucléotide régulateur (Pa), ou promoteur non fonctionnel selon l'invention est un acide nucléique qui :

(i) ne permet pas l'expression de la protéine ACCS de séquence SEQ ID N°3 dans une cellule hôte, ou
(ii) permet l'expression de cette protéine à un taux faible en comparaison du taux observé avec le polynucléotide régulateur (PA), ou
(iii) permet l'expression de la protéine ACCS au cours de la vie de la plante, pendant une durée inférieure, en comparaison de celle observée avec le polynucléotide régulateur (PA).

Pour comparer le niveau d'expression de plusieurs promoteurs, une technique simple, connue de l'homme du métier consiste à placer un gène marqueur de sélection sous le contrôle des promoteurs à tester. Un gène marqueur de sélection peut être par exemple le gène de résistance à l'herbicide BASTA, bien connu de l'homme du métier.

Une autre technique peut consister à mesurer le taux de la protéine ACCS obtenue lorsque la séquence codant pour cette protéine est sous le contrôle de différents promoteurs, en utilisant des anticorps dirigés à l'encontre cette protéine, et les procédés décrits dans la partie « polypeptides selon l'invention ».

A titre d'exemple, un polynucléotide régulateur (Pa) non fonctionnel comprend une séquence nucléotidique allant du nucléotide 1 au nucléotide 3650 de la séquence SEQ ID N°2.

Ainsi, dans le système de contrôle objet de l'invention un polynucléotide régulateur (Pa) non fonctionnel peut comprendre une séquence nucléotidique allant du nucléotide 1 au nucléotide 3650 de la séquence SEQ ID N°2.

L'invention décrit également le polynucléotide régulateur (Pa) en tant que tel, défini ci-dessus.

L'invention a également pour objet un acide nucléique comprenant la séquence SEQ ID N°2. Un tel acide nucléique comprend un polynucléotide régulateur (Pa) et un acide nucléique codant pour la protéine ACCS de séquence SEQ ID N°3.

Un polynucléotide (Pa) non fonctionnel peut également être tout polynucléotide dérivé de du polynucléotide (PA) tel que défini ci-dessus dont la séquence nucléotidique comprend une insertion, une substitution ou une délétion d'un ou plusieurs nucléotides, par rapport à la séquence nucléotidique du polynucléotide régulateur.

Ainsi, l'invention a également pour objet un acide nucléique comprenant une séquence nucléotidique portant au moins une altération choisie parmi une mutation, une insertion ou une délétion, par rapport à l'acide nucléique allant du nucléotide 1 au nucléotide 5906 de la séquence SEQ ID N°1, ledit acide nucléique altéré conduisant à une expression réduite de la protéine ACCS, lorsqu'il contrôle l'expression de ladite protéine, par rapport à l'expression de la protéine ACCS contrôlée par l'acide nucléique allant du nucléotide 1 au nucléotide 5906 de la séquence SEQ ID N°1.

L'invention décrit également un système de contrôle tel que défini ci-dessus, dans lequel le polynucléotide régulateur (Pa) est sensible à l'action d'un signal inducteur, et de préférence, dans lequel le polynucléotide régulateur (Pa) est un polynucléotide répresseur inductible de la transcription ou de la traduction.

Par polynucléotide régulateur « répresseur », on entend selon l'invention une séquence régulatrice dont l'activité constitutive peut être bloquée par un signal externe. Un tel signal externe peut être l'absence de fixation d'un facteur de transcription reconnu par le polynucléotide régulateur répresseur. L'absence de fixation du facteur de transcription peut être induite sous l'effet du signal inducteur répresseur auquel le polynucléotide régulateur répresseur est sensible.

Selon ce premier mode de réalisation particulier, l'expression de la séquence codant pour une protéine ACCS est constitutive dans l'hôte cellulaire choisi, en l'absence du signal inducteur répresseur auquel le polynucléotide régulateur répresseur est directement ou indirectement sensible.

La mise en contact de l'hôte cellulaire avec le signal inducteur répresseur a pour effet, grâce à une action directe ou indirecte sur le polynucléotide régulateur répresseur, d'inhiber et/ou de bloquer l'expression du polynucléotide codant pour la protéine ACCS.

Pour réaliser les constructions d'ADN selon l'invention comprenant un polynucléotide régulateur répresseur, l'homme du métier aura recours à ses connaissances générales techniques dans le domaine de l'expression de gènes chez les végétaux.

Un procédé d'obtention d'une plante transformée, mettant en oeuvre ce type de polynucléotide régulateur est décrit dans la partie intitulée « procédés d'obtention d'une plante transformée selon l'invention ».

***- acide nucléique codant pour une protéine A CCS non active***

Par « acide nucléique codant pour une protéine ACCS non active » , on entend au sens de la présente invention, un acide nucléique qui code pour une protéine qui diffère de la protéine ACCS de séquence SEQ ID N°3, par la substitution, la délétion, ou l'insertion d'un ou plusieurs acides aminés, et qui ne possède pas l'activité biologique de la protéine ACCS de séquence SEQ ID N°3.

***- protéine ACCS non active***

Par « protéine ACCS non active », on entend au sens de la présente invention, une protéine qui diffère de la protéine ACCS de séquence SEQ ID N°3, par la substitution, la délétion, ou l'insertion d'un ou plusieurs acides aminés, et qui ne possède pas l'activité biologique de la protéine ACCS de séquence SEQ ID N°3.

D'une manière générale, une protéine ACCS non active est une protéine dont l'expression est associée à un phénotype andromonoïque ou hermaphrodite.

A titre d'exemple, une protéine ACCS non active peut être une protéine qui ne permet pas de transformer la S-adénosyl méthionine en ACC (1-aminocyclopropane-1-carboxylate).

Les inventeurs ont montré dans l'exemple 1 qu'une protéine ACCS non active selon l'invention est par exemple une protéine ACCS de séquence SEQ ID N°3 dans laquelle le résidu alanine en position 57 est remplacé par un résidu valine.

**Elément génétique de contrôle G/g, sous forme d'allèle récessif (g) du système selon l'invention**

De manière générale, l'élément génétique de contrôle (G/g) sous la forme de l'allèle récessif (g), lorsqu'il est présent dans une plante conduit au développement d'une plante hermaphrodite ou gynoïque.

**Acides nucléiques selon l'invention**

Comme indiqué ci-dessus, il a été caractérisé selon l'invention deux variants alléliques (A) et (a) d'un premier élément génétique de contrôle (A/a)

Les inventeurs ont identifié l'acide nucléique de séquence SEQ ID N°1 comme étant un acide nucléique correspondant au variant allélique dominant (A) et l'acide nucléique de séquence SEQ ID N°2, comme correspondant au variant allélique récessif (a), d'un premier élément génétique de contrôle sous forme d'un gène (A/a).

Dans le système de contrôle objet de l'invention, l'un au moins des deux éléments génétiques de contrôle a été introduit artificiellement dans une plante.

Comme cela a été exposé ci-dessus, une telle introduction provoque un changement du sexe de la fleur de la plante, ce qui est un des buts recherchés selon l'invention.

En conséquence les séquences SEQ ID N°1 et SEQ ID N°2 font partie des objets de l'invention.

La présente invention décrit donc un acide nucléique comprenant un polynucléotide possédant au moins 95% d'identité en nucléotides avec une séquence nucléotidique choisie parmi SEQ ID N°1 et SEQ ID N°2, ou avec un fragment de l'une des séquences SEQ ID N°1 et SEQ ID N°2, à condition qu'un tel acide nucléique possède les caractéristiques fonctionnelles de l'allèle (A) ou de l'allèle (a) tels que définis ci-dessus.

Est également décrit un acide nucléique de séquence complémentaire à l'acide nucléique tel que défini ci-dessus.

Est également décrit un acide nucléique consistant en un polynucléotide possédant au moins 95% d'identité en nucléotides avec une séquence choisie parmi les séquences SEQ ID N°1 et SEQ ID N°2, ou avec un fragment de l'une des séquences SEQ ID N°1 ou SEQ ID N°2, ou un acide nucléique de séquence complémentaire, à condition qu'un tel acide nucléique possède les caractéristiques fonctionnelles de l'allèle (A) ou de l'allèle (a) tels que définis ci-dessus.

L'invention décrit aussi un acide nucléique comprenant au moins 12, de préférence au moins 15 et de manière tout à fait préférée au moins 20 nucléotides consécutifs de l'acide nucléique de séquence SEQ ID N°1 ou SEQ ID N°2, étant entendu qu'un tel acide nucléique englobe dans sa définition les « fragments » d'un acide nucléique selon l'invention tel que défini dans la présente description.

L'invention est également relative aux acides nucléiques comprenant ou consistant en les séquences SEQ ID N°1 ou 2.

L'invention décrit aussi un acide nucléique comprenant au moins 12, de préférence au moins 15 et de manière tout à fait préférée au moins 20 nucléotides consécutifs de l'acide nucléique de séquence SEQ ID N°1 ou SEQ ID N°2, étant entendu qu'un tel acide nucléique englobe dans sa définition les « fragments » d'un acide nucléique selon l'invention tel que défini dans la présente description.

L'allèle (A) défini par la séquence SEQ ID N°1 comprend, de l'extrémité 5' vers l'extrémité 3', respectivement :

a) une séquence non codante portant des éléments régulateurs de la transcription et/ou de la traduction de ce gène, localisée en amont du premier exon, du nucléotide en position 1 jusqu'au nucléotide en position 5906 de la séquence SEQ ID N°1;
b) une région dite « codante » qui comprend les trois exons et les deux introns du gène (A/a), cette région codante étant localisée du nucléotide en position 5907 jusqu'au nucléotide en position 7915 de la séquence SEQ ID N°1; et
c) une région non codante localisée en aval de la région codante, du nucléotide en position 7915 jusqu'au nucléotide en position 13380 de la séquence SEQ ID N°1.

L'allèle (a) défini par la séquence SEQ ID N°1 comprend, de l'extrémité 5' vers l'extrémité 3', respectivement :

a) une séquence non codante portant des éléments régulateurs de la transcription et/ou de la traduction de ce gène, localisée en amont du premier exon, du nucléotide en position 1 jusqu'au nucléotide en position 3650 de la séquence SEQ ID N°2; qui diffère sensiblement de la séquence non codante localisée en partie 5' de l'acide nucléique de séquence SEQ ID N°1,
b) une région dite « codante » qui comprend les trois exons et les deux introns du gène (A/a), cette région codante étant localisée du nucléotide en position 3651 jusqu'au nucléotide en position 5659 de la séquence SEQ ID N°2, qui diffère peu de la séquence codante de l'acide nucléique de séquence SEQ ID N°1, et code pour une même protéine ACCS de séquence SEQ ID N°3. et

c) une région non codante localisée en aval de la région codante, du nucléotide en position 5659 jusqu'au nucléotide en position 11137 de la séquence SEQ ID N°1, qui diffère peu de la séquence non codante localisée en partie 3' de l'acide nucléique de séquence SEQ ID N°1.

Les caractéristiques structurales des trois exons et des deux introns du gène A/a sont détaillées dans le tableau 2 ci-après. Les caractéristiques structurales des exons et des introns des allèles (A) et (a) du gène (A/a) sont très proches, de sorte que les exons des allèles (A) et (a) codent pour une même protéine de séquence SEQ ID N°3. Comme cela a été rappelé ci-dessus, la différence majeure entre les séquences nucléotidiques correspondant aux allèles (A) et (a) se trouve dans les séquences régulatrices amont correspondant à ces deux allèles. Ces deux séquences possèdent donc une région commune constituée de 3 exons et 2 introns, et une région non commune, comprenant des régions régulatrices disctinctes.

**TABLEAU 2**

| Séquences des exons du gène A/a | | | | |
|---|---|---|---|---|
| Exon n° | Position du nucléotide en 5' sur | | Position du nucléotide en 3' sur | |
| | SEQ ID N°1 (allèle A) | SEQ ID N°2 (Allèle a) | SEQ ID N°1 (Allèle A) | SEQ ID N°2 (Allèle a) |
| 1 | 5907 | 3651 | 6086 | 3830 |
| 2 | 6181 | 3924 | 6467 | 4209 |
| 3 | 7046 | 4790 | 7915 | 5659 |

L'invention décrit également un acide nucléique comprenant au moins 12 nucléotides consécutifs d'un polynucléotide exonique du gène A/a, tel que les polynucléotides 1 à 3 décrits dans le tableau 1 ci-dessus, qui sont inclus dans l'acide nucléique de séquence SEQ ID N°1 et SEQ ID N°2.

Un tel acide nucléique code pour au moins une partie de la protéine ACCS et peut notamment être inséré dans un vecteur recombinant destiné à l'expression du produit de traduction correspondant dans une cellule hôte ou dans une plante transformée avec ce vecteur recombinant, en vue de d'obtenir une plante de génotype (A).

Un tel acide nucléique peut aussi être utilisé pour la synthèse de sondes et d'amorces nuctéotidiques destinées à la détection ou à l'amplification de séquences nucléotidiques comprises dans le gène (A/a) dans un échantillon.

Le cas échéant les séquences décrites ci-dessus peuvent porter une ou plusieurs mutations, préférentiellement une ou plusieurs mutations de nature à induire la synthèse d'une protéine ACCS non active, et à modifier le type sexuel d'une plante portant un tel gène (A/a) muté. De telles séquences répondent à la définition d'acides nucléiques codant pour une protéine ACCS non active, définis de manière générale ci-dessus.

**TABLEAU 3**

| Séquences des Introns du gène (A/a) | | | | |
|---|---|---|---|---|
| Intron n° | Position du nucléotide en 5' sur | | Position du nucléotide en 3' sur | |
| | SEQ ID N°1 | SEQ ID N°2 | SEQ ID N°1 | SEQ ID N°2 |
| 1 | 6087 | 3831 | 6180 | 3923 |
| 2 | 6468 | 4210 | 7045 | 4789 |

Il est également décrit un acide nucléique comprenant au moins 12 nucléotides consécutifs d'un polynucléotide intronique du gène (A/a), tel que les polynucléotides 1 et 2 décrits dans le tableau 2 ci-dessus, qui sont inclus dans l'acide nucléique de séquence SEQ ID N°1 et SEQ ID N°2.

Un tel acide nucléique peut être utilisé comme sonde ou amorce oligonucléotidique pour détecter la présence d'au moins une copie du gène (A/a) dans un échantillon, ou encore pour amplifier une séquence cible déterminée au sein du gène (A/a).

Un tel acide nucléique peut aussi être utilisé pour amplifier une séquence cible déterminée au sein du gène (A/a) ou l'inhiber par une approche sens ou co-suppression, ou par l'utilisation d'ARN double brin (Wassenegger et al. 1996 ; Kooter et al. 1999) pour interférence. Un tel acide nucléique peut également être utilisé pour rechercher des variants alléliques fonctionnels du gène (A/a), qui pourront être utilisés dans une méthode de sélection de plantes possédant un type sexuel déterminé.

Il est précisé que, sur leur région commune, à savoir les 3 exons et les deux introns décrits ci-dessus, les séquences SEQ ID N°1 et SEQ ID N°2 ont un pourcentage d'identité en nucléotides supérieur à 95%, ce pourcentage étant en fait supérieur à 99%.

***Autres acides nucléiques selon l'invention, codant pour la protéine ACCS***

L'invention décrit également un acide nucléique comprenant un polynucléotide possédant au moins 95% d'identité en nucléotides avec la séquence nucléotidique débutant au nucléotide en position 5907 et se terminant au nucléotide en position 7915 de la séquence SEQ ID N°1 ainsi qu'un acide nucléique de séquence complémentaire.

L'invention concerne aussi un acide nucléique possédant au moins 95% d'identité en nucléotides avec la séquence nucléotidique débutant au nucléotide en position 5907 et se terminant au nucléotide en position 7915 de la séquence SEQ ID N°1, ainsi qu'un acide nucléique de séquence complémentaire.

L'invention décrit encore un acide nucléique comprenant la séquence nucléotidique débutant au nucléotide en position 5907 et se terminant au nucléotide en position 7915 de la séquence SEQ ID N°1 ou un acide nucléique de séquence complémentaire.

L'invention décrit également un acide nucléique consistant en la séquence nucléotidique débutant au nucléotide en position 5907 et se terminant au nucléotide en position 7915 de la séquence SEQ ID N°1 ou un acide nucléique de séquence complémentaire.

Un autre objet de l'invention consiste en un acide nucléique comprenant, au moins :

(i) une séquence ayant au moins 95% d'identité avec le polynucléotide allant du nucléotide 5907 au nucléotide 6086 de la séquence SEQ ID N°1,
(ii) une séquence ayant au moins 95% d'identité avec le polynucléotide allant du nucléotide 6181 au nucléotide 6467 de la séquence SEQ ID N°1, et
(iii) une séquence ayant au moins 95% d'identité avec le polynucléotide allant du nucléotide 7046 au nucléotide 7915 de la séquence SEQ ID N°1.

L'invention a encore pour objet un acide nucléique comprenant, de l'extrémité 5' vers l'extrémité 3' :

(i) une séquence allant du nucléotide 5907 au nucléotide 6086 de la séquence SEQ ID N°1 ,
(ii) une séquence allant du nucléotide 6181 au nucléotide 6467 de la séquence SEQ ID N°1, et
(iii) une séquence allant du nucléotide 7046 au nucléotide 7915 de la séquence SEQ ID N°1.

Un acide nucléique codant pour la protéine ACCS, peut comprendre en outre des séquences leader et terminateur, classiques pour l'homme du métier.

**Produits de transcription et de traduction du gène (A/a) et polypeptides selon l'invention.**

Il est encore décrit le polypeptide comprenant la séquence d'acides aminés SEQ ID N°3, également nommé « protéine ACCS » dans la présente description, ainsi qu'un polypeptide possédant au moins 95% d'identité en acides aminés avec la séquence SEQ ID N°3 ou un fragment ou un variant de celui-ci.

Un fragment d'une protéine ACCS selon l'invention comprend au moins 10, 50, 100, 200, 300, 400, 420, 430, 440 ou 445 acides aminés consécutifs d'un polypeptide de séquence SEQ ID N°3.

L'invention est également relative à un polypeptide comprenant une séquence en acides aminés ayant au moins 95% d'identité en acides aminés avec la séquence d'une protéine ACCS de séquence SEQ ID N°3.

Avantageusement, fait aussi partie de l'invention un polypeptide ayant au moins 96%, 97%, 98%, 99%, 99,1%, 99,2%, 99,3%, 99,4%, 99,5%, 99,6%, 99,7%, 99,8% ou 99,9% d'identité en acides aminés avec la séquence d'un polypeptide de séquence SEQ ID N°3, ou un fragment peptidique de ce dernier.

De manière générale, les polypeptides selon la présente invention se présentent sous une forme isolée ou purifiée.

Un polypeptide selon l'invention peut être obtenu par recombinaison génétique selon des techniques bien connues de l'homme du métier, par exemple des techniques décrites dans AUSUBEL et al. (1989).

Un polypeptide selon l'invention peut être également préparé par des techniques classiques de synthèse chimique, indifféremment en solution homogène ou en phase solide.

A titre illustratif, un polypeptide selon l'invention pourra être préparé par la technique en solution homogène décrite par HOUBEN WEIL (1974) ou encore par la technique de synthèse en phase solide décrite par MERRIFIELD (1965a; 1965b).

De préférence, les polypeptides variants d'un polypeptide selon l'invention conservent leur capacité à être reconnus par des anticorps dirigés contre les polypeptides de séquences SEQ ID N°3.

Un polypeptide codé par le gène (A/a) selon l'invention, tel qu'un polypeptide de séquence en acides aminés SEQ ID N°3, ou encore un variant ou un fragment peptidique de ce dernier est utile notamment pour la préparation d'anticorps destinés à la détection de la présence et/ou de l'expression d'un polypeptide de séquences SEQ ID N°3 ou d'un fragment peptidique de ce dernier dans un échantillon.

L'invention décrit encore un polypeptide de séquence SEQ ID N°8 ou un fragment polypeptidique de cette séquence tel que défini ci-dessus. Le polypeptide de séquence SEQ ID N°8 code pour une protéine ACCS « non active » selon la définition donnée ci-dessus et diffère de la séquence SEQ ID N°3 par la présence d'un résidu valine en position 57.

Outre la détection de la présence d'un polypeptide codé par le gène (A/a) ou encore d'un fragment peptidique d'un tel polypeptide dans un échantillon, des anticorps dirigés contre ces polypeptides sont utilisés pour quantifier la synthèse d'un polypeptide de séquences SEQ ID N°3 ou 8, par exemple dans des cellules d'une plante, et déterminer ainsi le sexe de la plante, sans pour autant devoir la cultiver.

Par "anticorps" au sens de la présente invention, on entendra notamment des anticorps polyclonaux ou monoclonaux ou des fragments (par exemple les fragments $F(ab)'_2$, F(ab)) ou encore tout polypeptide comprenant un domaine de l'anticorps initial reconnaissant le polypeptide ou le fragment de polypeptide cible selon l'invention.

Des anticorps monoclaux peuvent être préparés à partir d'hybridomes selon la technique décrite par KOHLER et MILSTEIN (1975).

La présente invention décrit également des anticorps dirigés contre un polypeptide tel que décrit ci-dessus ou un fragment ou un variant de ce dernier, tel que produit dans la technique du trioma ou encore la technique d'hybridome décrite par KOZBOR et al. (1983).

L'invention décrit également des fragments d'anticorps simple chaîne Fv (ScFv) tels que décrits dans le brevet US N°4,946,778 ou encore par MARTINEAU et al. (1998).

Les anticorps décrits selon l'invention comprennent également des fragments d'anticorps obtenus à l'aide de banques de phages telles que décrites par RIDDER et al. (1995) ou encore des anticorps humanisés tels que décrits par REINMANN et al. (1997) et LEGER et al. (1997). Les préparations d'anticorps selon l'invention sont utiles dans des tests de détection immunologiques destinés à l'identification de la présence et/ou de la quantité d'un polypeptide de séquences SEQ ID N°3, ou d'un fragment peptidique de celui-ci, présent dans un échantillon.

Un anticorps décrit selon l'invention pourra comprendre en outre un marqueur détectable isotopique ou non isotopique, par exemple fluorescent, ou encore être couplé à une molécule telle que la biotine, selon des techniques bien connues de l'homme du métier.

Ainsi, l'invention décrit en outre un procédé pour détecter la présence d'un polypeptide conforme à l'invention dans un échantillon, ledit procédé comprenant les étapes de:

a) mettre en contact l'échantillon à tester avec un anticorps tel que décrit ci-dessus;
b) détecter le complexe antigène/anticorps formé.

L'invention décrit également un nécessaire ou kit de diagnostic pour la détection de la présence d'un polypeptide conforme à l'invention dans un échantillon, ledit nécessaire comprenant:

a) un anticorps tel que défini ci-dessus;
b) le cas échéant, un ou plusieurs réactifs nécessaires à la détection du complexe antigène/anticorps formé.

Un autre objet de l'invention consiste en l'utilisation d'un acide nucléique ou d'un variant allélique d'un acide nucléique tel que défini ci-dessus dans des programmes de sélection de plantes pour l'obtention de plantes dont le type floral a été modifié.

***<u>Acides nucléiques comprenant, un polynucléotide régulateur (PA) fonctionnel</u>***

Un polynucléotide régulateur ou promoteur (PA) fonctionnel selon l'invention consiste en un acide nucléique qui permet l'expression de la protéine ACCS de séquence SEQ ID N°3 chez les plantes dicotylédones.

Un tel polynucléotide régulateur (PA) fonctionnel permet ainsi, lorsqu'il est introduit artificiellement dans une plante, de modifier le sexe des fleurs d'une telle plante, et en particulier permet d'obtenir des plantes femelles, non capables d'autopollinisation.

L'invention décrit donc également un acide nucléique comprenant un polynucléotide possédant au moins 95% d'identité en nucléotides avec la séquence nucléotidique débutant au nucléotide en position 1 et se terminant au nucléotide en position 5906 de la séquence SEQ ID N°1 ainsi qu'un acide nucléique de séquence complémentaire.

L'invention décrit aussi un acide nucléique possédant au moins 95% d'identité en nucléotides avec la séquence nucléotidique débutant au nucléotide en position 1 et se terminant au nucléotide en position 5906 de la séquence SEQ ID N°1, ainsi qu'un acide nucléique de séquence complémentaire.

L'invention décrit encore un acide nucléique comprenant la séquence nucléotidique débutant au nucléotide en position 1 et se terminant au nucléotide en position 5906 de la séquence SEQ ID N°1 ou un acide nucléique de séquence complémentaire.

L'invention décrit également un acide nucléique consistant en la séquence nucléotidique débutant au nucléotide en position 1 et se terminant au nucléotide en position 5906 de la séquence SEQ ID N°1 ou un acide nucléique de séquence complémentaire.

L'invention décrit également un acide nucléique comprenant au moins 12 nucléotides consécutifs d'un polynucléotide régulateur, tel que défini ci-dessus.

Un tel acide nucléique peut être utilisé comme sonde ou amorce oligonucléotidique pour détecter la présence d'au moins une copie de l'allèle (A) du gène (A/a) dans un échantillon, pour amplifier une séquence cible déterminée au sein du gène (A/a). Un tel acide nucléique peut également être utilisé pour rechercher des variants alléliques fonctionnels du gène (A/a), ou pourront être utilisés dans une méthode de sélection de plantes possédant un type sexuel déterminé.

Des procédés de détection mettant en oeuvre des acides nucléiques tels que décrits ci-dessus sont décrits dans la partie intitulée « Procédés de sélection selon l'invention »

Un tel acide nucléique peut aussi être utilisé pour inhiber une séquence cible déterminée au sein du gène (A/a) par une approche antisens ou co-suppression, ou par l'utilisation d'ARN double brin (Wassenegger et al. 1996 ; Kooter et al. 1999) pour interférence.

***<u>Acides nucleiques comprenant un polynucléotide régulateur Pa non fonctionnel</u>***

Un polynucléotide régulateur (Pa), ou promoteur non fonctionnel selon l'invention est un acide nucléique qui :

(i) ne permet pas l'expression de la protéine ACCS de séquence SEQ ID N°3 dans une cellule hôte, ou
(ii) permet l'expression de cette protéine à un niveau très faible en comparaison du niveau observé avec le polynucléotide régulateur (PA), ou
(iii) permet l'expression de la protéine ACCS au cours de la vie de la plante, pendant une durée inférieure, en comparaison de celle observée avec le polynucléotide régulateur (PA).

Un tel polynucléotide régulateur (Pa) non fonctionnel permet ainsi, lorsqu'il est introduit artificiellement dans une plante, par exemple en remplacement d'un polynucléotide (A) de modifier le sexe des fleurs d'une telle plante, et en particulier permet d'obtenir des plantes hermaphrodites, capables d'autopollinisation.

L'invention décrit donc également un acide nucléique comprenant un polynucléotide possédant au moins 95% d'identité en nucléotides avec la séquence nucléotidique débutant au nucléotide en position 1 et se terminant au nucléotide en position 3650 de la séquence SEQ ID N°2 ainsi qu'un acide nucléique de séquence complémentaire.

L'invention décrit aussi un acide nucléique possédant au moins 95% d'identité en nucléotides avec la séquence nucléotidique débutant au nucléotide en position 1 et se terminant au nucléotide en position 3650 de la séquence SEQ ID N°2, ainsi qu'un acide nucléique de séquence complémentaire.

L'invention décrit encore un acide nucléique comprenant la séquence nucléotidique débutant au nucléotide en position 1 et se terminant au nucléotide en position 3650 de la séquence SEQ ID N°2 ou un acide nucléique de séquence complémentaire.

L'invention décrit également un acide nucléique consistant en la séquence nucléotidique débutant au nucléotide en position 1 et se terminant au nucléotide en position 3650 de la séquence SEQ ID N°2 ou un acide nucléique de séquence complémentaire.

Un tel acide nucléique peut être utilisé comme sonde ou amorce oligonucléotidique pour détecter la présence d'au moins une copie de l'allèle (a) du gène (A/a) dans un échantillon, ou encore pour amplifier une séquence cible déterminée au sein du gène (A/a).

L'invention concerne enfin des acides nucléiques comprenant l'association un ou plusieurs acides nucléiques tels que définis ci-dessus, par exemple un acide nucléique codant pour une protéine ACCS fonctionnelle sous le contrôle d'un promoteur de type (PA) ou (Pa).

**<u>Définitions générales</u>**

Selon l'invention, toute technique classique de biologie moléculaire, de microbiologie et d'ADN recombinant connue de l'homme du métier peut être utilisée. De telles techniques sont décrites par exemple par SAMBROOK et al. (1989), GLOVER (1985), GAIT (1984), HAMES et HIGGINS (1984), BERBAL (1984) et AUSUBEL et al. (1994).

De manière préférée, tout acide nucléique et tout polypeptide selon l'invention se présente sous une forme isolée ou purifiée.

Le terme "isolé" au sens de la présente invention désigne un matériel biologique qui a été soustrait à son environnement originel (l'environnement dans lequel il est localisé naturellement). Par exemple, un polynucléotide présent à l'état naturel dans une plante n'est pas isolé. Le même polynucléotide séparé des acides nucléiques adjacents au sein desquels il est naturellement inséré dans le génome de la plante est isolé. Un tel polynucléotide peut être inclus dans un vecteur et/ou un tel polynucléotide peut être inclus dans une composition et demeurer néanmoins à l'état isolé du fait que le vecteur ou la composition ne constitue pas son environnement naturel.

Le terme " purifié " ne nécessite pas que le matériel soit présent sous une forme de pureté absolue, exclusive de la présence d'autres composés. Il s'agit plutôt d'une définition relative.

Un polynucléotide ou un polypeptide est à l'état purifié après purification du matériel de départ ou du matériel naturel d'au moins un ordre de grandeur, de préférence 2 ou 3 et préférentiellement quatre ou cinq ordres de grandeur.

Aux fins de la présente description, l'expression " séquence nucléotidique " peut être employée pour désigner indifféremment un polynucléotide ou un acide nucléique. L'expression " séquence nucléotidique " englobe le matériel génétique lui-même et n'est donc pas restreinte à l'information concernant sa séquence.

Les termes " acide nucléique ", "polynucléotide ", " oiigonucléotide " ou encore " séquence nucléotidique " englobent des séquences d'ARN, d'ADN, d'ADNc ou encore des séquences hybrides ARN/ADN de plus d'un nucléotide, indifféremment sous la forme simple brin ou sous la forme de duplex.

Le terme " nucléotide " désigne à la fois les nucléotides naturels (A, T, G, C) ainsi que des nucléotides modifiés qui comprennent au moins une modification telle que (i) un analogue d'une purine, (ii) un analogue d'une pyrimidine, ou (iii) un sucre analogue, de tels nucléotides modifiés étant décrits par exemple dans la demande PCT N°WO 95/04064.

Aux fins de la présente invention, un premier polynucléotide est considéré comme étant " complémentaire " d'un second polynucléotide lorsque chaque base du premier nucléotide est appariée à la base complémentaire du second polynucléotide dont l'orientation est inversée. Les bases complémentaires sont A et T (ou A et U), et C et G.

Selon l'invention, un premier acide nucléique ayant au moins 95% d'identité avec un second acide nucléique de référence, possédera au moins 95%, de préférence au moins 96%, 97%, 98%, 98,5%, 99%, 99,1%, 99,2%, 99,3%, 99,4%, 99,5%, 99,6%, 99,7%, 99,8% ou 99,9% d'identité en nucléotides avec ce second polynucléotide de référence, le pourcentage d'identité entre deux séquences étant déterminé comme décrit ci-dessous.

Le « pourcentage d'identité » entre deux séquences d'acides nucléiques, au sens de la présente invention, est déterminé en comparant les deux séquences alignées de manière optimale, à travers une fenêtre de comparaison.

La partie de la séquence nucléotidique dans la fenêtre de comparaison peut ainsi comprendre des additions ou des délétions (par exemple des « gaps ») par rapport à la séquence de référence (qui ne comprend pas ces additions ou ces délétions) de manière à obtenir un alignement optimal entre les deux séquences.

Le pourcentage d'identité est calculé en déterminant le nombre de positions auxquelles une base nucléique identique est observée pour les deux séquences comparées, puis en divisant le nombre de positions auxquelles ii y a identité entre les deux bases nucléiques par le nombre total de positions dans la fenêtre de comparaison, puis en multipliant le résultat par cent afin d'obtenir le pourcentage d'identité en nucléotides des deux séquences entre elles.

L'alignement optimal des séquences pour la comparaison peut être réalisé de manière informatique à l'aide d'algorithmes connus.

De manière tout à fait préférée, le pourcentage d'identité de séquence est déterminé à l'aide du logiciel CLUSTAL W (version 1.82) les paramètres étant fixés comme suit : (1) CPU MODE = ClustalW mp ; (2) ALIGNMENT = « full » ; (3) OUTPUT FORMAT = « aln w/numbers » ; (4) OUTPUT ORDER = « aligned » ; (5) COLOR ALIGNMENT = « no » ; (6) KTUP (word size) = « default » ; (7) WINDOW LENGTH = « default » ; (8) SCORE TYPE = « percent » ; (9) TOPDIAG = « default » ; (10) PAIRGAP = « default » ; (11) PHYLOGENETIC TREE/TREE TYPE = « none » ; (12) MATRIX = « default » ; (13) GAP OPEN = « default » ; (14) END GAPS = « default » ; (15) GAP EXTENSION = « default » ; (16) GAP DISTANCES = « default » ; (17) TREE TYPE = « cladogram » et (18) TREE GRAP DISTANCES = « hide ».

Un acide nucléique possédant au moins 95% d'identité en nucléotides avec un acide nucléique selon l'invention englobe les " variants " d'un acide nucléique selon l'invention.

Par " variant " d'un acide nucléique selon l'invention, on entend un acide nucléique qui diffère de l'acide nucléique de référence par une ou plusieurs substitutions, additions ou délétions d'un nucléotide, par rapport à l'acide nucléique de référence. Un variant d'un acide nucléique selon l'invention peut être d'origine naturelle, tel qu'un variant allélique qui existe naturellement. Un tel acide nucléique variant peut être également un acide nucléique non naturel obtenu, par exemple, par des techniques de mutagenèse.

En général, les différences entre l'acide nucléique de référence et l'acide nucléique " variant " sont réduites de telle sorte que l'acide nucléique de référence et l'acide nucléique variant ont des séquences nucléotidiques très similaires et, dans de nombreuses régions, identiques. Les modifications nucléotidiques présentes dans un acide nucléique variant peuvent être silencieuses, ce qui signifie qu'elles n'affectent pas la séquence d'acides aminés qui peut être codée par cet acide nucléique variant.

Les modifications de nucléotides dans l'acide nucléique variant peuvent aussi résulter en des substitutions, additions ou délétions d'un ou plusieurs acides aminés dans la séquence du polypeptide qui peut être codé par cet acide nucléique variant.

De manière tout à fait préférée, un acide nucléique variant selon l'invention comportant une phase de lecture ouverte, code pour un polypeptide qui conserve la même fonction ou la même activité biologique que le polypeptide codé par l'acide nucléique de référence.

De manière tout à fait préférée, un acide nucléique variant selon l'invention et qui comporte une phase de lecture ouverte, code pour un polypeptide qui conserve la capacité d'être reconnu par des anticorps dirigés contre le polypeptide codé par l'acide nucléique de référence.

Font partie des « variants » d'un acide nucléique codant la protéine ACCS les acides nucléiques des gènes orthologues à la protéine ACCS inclus dans le génome de plantes, et possédant une identité en nucléotides d'au moins 95% avec un acide nucléique codant la protéine ACCS.

Par " fragment " d'un acide nucléique selon l'invention, on entend une séquence nucléotidique d'une longueur réduite par rapport à l'acide nucléique de référence, le fragment d'acide nucléique possédant une séquence nucléotidique identique à la séquence nucléotidique de l'acide nucléique de référence sur la partie commune. De tels fragments d'un acide nucléique selon l'invention possèdent au moins 12, 15, 18, 20, 25, 30, 35, 40, 45, 50, 60, 70, 80, 90, 100, 150, 200, 300, 400, 500, 1000, 2000 ou 3000 nucléotides consécutifs de l'acide nucléique de référence, la longueur maximale en nucléotides d'un fragment d'un acide nucléique selon l'invention étant bien entendu limitée par la longueur maximale en nucléotides de l'acide nucléique de référence.

## SONDES ET AMORCES

Les acides nucléiques selon l'invention, et en particulier les séquences nucléotidiques SEQ ID N°1 et SEQ ID N°2, leurs fragments d'au moins 12 nucléotides, les polynucléotides régulateurs (PA) et (Pa), ainsi que les acides nucléiques de séquence complémentaire, sont utiles pour la détection de la présence d'au moins une copie d'une séquence nucléotidique du gène (A/a) ou encore d'un fragment ou d'un variant allélique de cette dernière dans un échantillon.

En particulier, les sondes et amorces ci-dessus dérivés des séquence SEQ ID N°1, et en particulier dérivées du polynucléotide régulateur (PA) peuvent être utilisées pour détecter la présence de l'allèle (A) dans une plante dicotylédone.

De même, les sondes et amorces ci-dessus dérivées des séquence SEQ ID N°2, et en particulier dérivées du polynucléotide régulateur (Pa) non fonctionnel peuvent être utilisées pour détecter la présence de l'allèle (a) dans une plante dicotylédone.

Sont égalememnt décrites les sondes et amorces nucléotidiques hybridant, dans des conditions d'hybridation de forte stringence, avec un acide nucléique choisi parmi les séquences SEQ ID N°1 et SEQ ID N°2, ou avec un polynucléotide régulateur (PA) ou (Pa).

L'invention a donc également pour objet un acide nucléique, utilisable en tant que sonde ou amorce, hybridant spécifiquement avec un acide nucléique tel que défini ci-dessus.

Les conditions d'hybridation ci-dessous sont mises en oeuvre pour l'hybridation d'un acide nucléique, sonde ou amorce, de 20 bases de longueur.

Le niveau et la spécificité d'hybridation dépend de différents paramètres, tels que :

a) la pureté de la préparation de l'acide nucléique sur lequel la sonde ou l'amorce doit s'hybrider ;
b) la composition en bases de la sonde ou de l'amorce, les paires de base G-C possédant une plus grande stabilité thermique que les paires de bases R-T cu A-U ;
c) la longueur de la séquence de bases homologues entre la sonde ou l'amorce et l'acide nucléique ;
d) la force ionique : le taux d'hybridation augmente avec l'accroissement de la force ionique et la durée du temps d'incubation ;
e) la température d'incubation ;
f) la concentration de l'acide nucléique sur lequel la sonde ou l'amorce doit s'hybrider ;
g) la présence d'agents dénaturants tels que des agents favorisant la rupture des liaisons hydrogène, comme le formamide ou l'urée, qui accroissent la stringence de l'hybridation ;
h) le temps d'incubation, le taux d'hybridation augmentant avec la durée de l'incubation ;
i) la présence d'agents d'exclusion de volume, tels que le dextran ou le sulfate de dextran, qui augmentent le taux d'hybridation du fait qu'ils accroissent les concentrations effectives de la sonde ou l'amorce et de l'acide nucléique qui doit s'hybrider, au sein de la préparation.

Les paramètres définissant les conditions de stringence dépendent de la température à laquelle 50% des brins appariés se séparent (Tm).

Pour les séquences comprenant plus de 360 bases, Tm est définie par la relation:

Tm= 81,5 + 0,41 (%G+C)+16,6 Log(concentration en cations) - 0,63 (% formamide)-(600/nombre de bases) (SAMBROOK et al., (1989), pages 9.54-9.62).

Pour les séquences de longueur inférieure à 30 bases, Tm est définie par la relation: Tm= 4(G+C) + 2(A+T).

Dans des conditions de stringence appropriées, dans lesquelles les séquences aspécifiques n'hybrident pas, la température d'hybridation est approximativement de 5 à 30°C, de préférence de 5 à 10°C en-dessous de Tm.

Par « conditions d'hybridation de forte stringence » selon l'invention, on entend des conditions d'hybridation telles que l'on se place à une température d'hybridation de 5°C au-dessous du Tm.

Les conditions d'hybridation ci-dessus décrites peuvent être adaptées en fonction de la longueur et de la composition en bases de l'acide nucléique dont l'hybridation est recherchée ou du type de marquage choisi, selon les techniques connues de l'homme du métier.

Les conditions convenables d'hybridation peuvent par exemple être adaptées selon l'enseignement contenu dans l'ouvrage de HAMES et HIGGINS (1985) ou encore dans l'ouvrage de AUSUBEL et al. (1989).

A titre illustratif, les conditions d'hybridation utilisées pour un acide nucléique de 200 bases de longueur sont les suivantes :

__Préhybridation:__

mêmes conditions que pour l'hybridation
durée: 1 nuit.

__Hybridation:__

5 x SSPE (0.9 M NaCl, 50 mM phosphate de sodium pH 7.7, 5 mM EDTA)
5 x Denhardt's (0.2% PVP, 0.2% Ficoll, 0.2% SAB)
100 μg/ml ADN de sperme de saumon
0.1% SDS
durée: 1 nuit.

__Lavages:__

2 x SSC, 0.1 % SDS 10 min 65°C
1 x SSC, 0.1 % SDS 10 min 65°C
0.5 x SSC, 0.1 % SDS 10 min 65°C
0.1 x SSC, 0.1 % SDS 10 min 65°C.

Les sondes ou les amorces nucléotidiques selon l'invention comprennent au moins 12 nucléotides consécutifs d'un acide nucléique selon l'invention, en particulier d'un acide nucléique de séquences SEQ ID N°1 ou SEQ ID N°2 ou de sa séquence complémentaire, d'un acide nucléique ayant 95% d'identité en nucléotides avec une séquence choisie parmi les séquences SEQ ID N°1 ou 2 ou de sa séquence complémentaire ou encore d'un acide nucléique hybridant dans des conditions d'hybridation de forte stringence avec une séquence choisie parmi les séquences SEQ ID N°1 ou 2 ou de sa séquence complémentaire.

De préférence, des sondes ou amorces nucléotidiques selon l'invention auront une longueur d'au moins 15, 18, 20, 25, 30, 35, 40, 45, 50, 60, 100, 150, 200, 300, 400, 500, 1000, 2000, ou 3000 nucléotides consécutifs d'un acide nucléique selon l'invention.

Alternativement, une sonde ou une amorce nucléotidique selon l'invention consistera et/ou comprendra les fragments d'une longueur de 15, 18, 20, 25, 30, 35, 40, 45, 50, 60, 900, 150, 200, 300, 400, 500, 1000, 2000 ou 3000 nucléotides consécutifs d'un acide nucléique selon l'invention.

A titre d'exemple, le couple d'amorces défini par la séquence SEQ ID N°5, correspondant à une amorce sens, et SEQ ID N°6 correspondant à une amorce antisens permet d'amplifier un fragment de l'acide nucléique de séquence SEQ ID N°1 ou un fragment de l'acide nucléique de séquence SEQ ID N°2

Une amorce ou une sonde nucléotidique selon l'invention peut être préparée par toute méthode adaptée bien connue de l'homme du métier, y compris par clonage et action d'enzymes de restriction ou encore par synthèse chimique directe selon des techniques telles que la méthode au phosphodiester de NARANG et al. (1979) ou de BROWN et al. (1979), la méthode au diéthylphosphoramidites de BEAUCAGE et al. (1980) ou encore la technique sur support solide décrite dans le brevet européen n°EP 0 707 592. Chacun des acides nucléiques selon l'invention, y compris les sondes et amorces oligonucléotidiques décrites ci-dessus, peut être marqué, si désiré, en incorporant une molécule détectable, c'est-à-dire un marqueur détectable, par des moyens spectroscopiques, photochimiques, biochimiques, immunochimiques ou encore chimiques.

Par exemple, de tels marqueurs peuvent consister en des isotopes radioactifs ($^{32}P$ $^{3}H$, $^{35}S$), des molécules fluorescentes (5-bromodéoxyuridine, fluorescéine, acétylaminofluorène) ou encore des ligands tels que la biotine.

Le marquage des sondes est fait de préférence par incorporation de molécules marquées au sein des polynucléotides par extension d'amorces, ou bien par rajout sur les extrémités 5' ou 3'.

Des exemples de marquage non radioactif de fragments d'acides nucléiques sont décrits notamment dans le brevet français n°FR 78 10 975 ou encore dans les articles de URDEA et al. (1988) ou SANCHEZ PESCADOR et al. (1988).

De manière avantageuse, les sondes selon l'invention peuvent avoir des caractéristiques structurelles de nature à permettre une amplification du signal, telles que les sondes décrites par URDEA et al; (1991) ou encore dans le brevet européen n°EP 0 225 807 (Chiron).

Les sondes oligonucléotidiques selon l'invention peuvent être utilisées notamment dans des hybridations de type Southern à tout acide nucléique codant pour la protéine ACCS, en en particulier les acides nucléiques de séquences SEQ ID N°1 ou 2, ou encore dans des hybridations à l'ARN lorsque l'expression du transcrit correspondant est recherchée dans un échantillon.

Les sondes selon l'invention peuvent aussi être utilisées pour la détection de produits d'amplification PCR ou encore pour la détection de mésappariements.

Des sondes ou amorces nucléotidiques selon l'invention peuvent être immobilisées sur un support solide. De tels supports solides sont bien connus de l'homme du métier et comprennent des surfaces des puits de plaques de micro-titration, des billes de polystyrène, des billes magnétiques, des bandes de nitrocellulose ou encore des microparticules telles que des particules de latex.

En conséquence, l'invention décrit encore un acide nucléique utilisable en tant que sonde ou amorce nucléotidique caractérisé en ce qu'il comprend au moins 12 nucléotides consécutifs d'un acide nucléique tel que défini ci-dessus, en particulier d'un acide nucléique de séquences nucléotidiques SEQ ID N°1 et SEQ ID N°2.

L'invention décrit également un acide nucléique utilisable en tant que sonde ou amorce nucléotidique caractérisé en ce qu'il consiste en un polynucléotide d'au moins 12 nucléotides consécutifs d'un acide nucléique selon l'invention, de manière tout à fait préférée d'un acide nucléique de séquences choisies parmi les séquences nucléotidiques SEQ ID N°1 et SEQ ID N°2.

Comme décrit ci-dessus, un tel acide nucléique peut en outre être caractérisé en ce qu'il est marqué par une molécule détectable.

Un acide nucléique utilisable en tant que sonde ou amorce nucléotidique pour la détection ou l'amplification d'une séquence génomique, de l'ARNm ou de l'ADNc du gène (A/a) peut en outre être caractérisé en ce qu'il est choisi parmi les séquences suivantes :

a) les séquences nucléotidiques hybridant, dans des conditions d'hybridation de forte stringence, avec un acide nucléique de séquence SEQ ID N°1 ou SEQ ID N°2; et

b) les séquences comprenant au moins 12 nucléotides consécutifs d'un acide nucléique de séquence SEQ ID N°1 ou SEQ ID N°2.

**<u>Vecteurs, cellules et plantes selon l'invention.</u>**

Dans le système de contrôle divulgué dans la présente description pour permettre qu'au moins un des éléments génétiques de contrôle soit artificiellement introduit dans la plante dicotylédone, les acides nucléiques, et les polynucléotides régulateurs définis ci-dessus doivent être introduits dans des vecteurs, puis dans des cellules.

Ainsi, l'invention décrit également des vecteurs, des cellules et des plantes transformées, qui comprennent les polynucléotides régulateurs (PA) et (Pa), les acides nucléiques codant pour des protéines ACCS actives ou non actives, ainsi que les acides nucléiques correspondant aux allèles (G) et (g) tels que décrits ci-dessus, et les amorces définies ci-dessus.

**Vecteurs**

Un acide nucléique tel que défini ci-dessus, nommé ci-après acide nucléique d'intérêt, peut être inséré dans un vecteur approprié.

Par " vecteur " au sens de la présente invention, on entendra une molécule d'ADN ou d'ARN circulaire ou linéaire qui est indifféremment sous forme simple brin ou double brin.

Un vecteur recombinant selon l'invention est de préférence un vecteur d'expression, ou plus spécifiquement un vecteur d'insertion, un vecteur de transformation ou un vecteur d'intégration.

Il peut s'agir notamment d'un vecteur d'origine bactérienne ou virale.

Dans tous les cas, l'acide nucléique d'intérêt est placé sous le contrôle d'une ou plusieurs séquences contenant des signaux de régulation de son expression dans la plante considérée, soit que les signaux de régulation soient tous contenus dans l'acide nucléique d'intérêt, comme c'est le cas dans les constructions d'acides nucléiques décrites dans la section précédente, soit que l'un, plusieurs d'entre eux, ou encore tous les signaux de régulation soient contenus dans le vecteur receveur dans lequel l'acide nucléique d'intérêt a été inséré.

Un vecteur recombinant selon l'invention comprend avantageusement des séquences d'initiation et d'arrêt de la transcription appropriées.

En outre, les vecteurs recombinants selon l'invention peuvent inclure une ou plusieurs origines de réplication fonctionnelles dans les cellules hôtes dans lesquelles leur expression est recherchée, ainsi que, le cas échéant, des séquences nucléotidiques marqueurs de sélection.

Les vecteurs recombinants selon l'invention peuvent aussi inclure un ou plusieurs des signaux de régulation de l'expression tels que définis ci-dessus dans la description.

Les vecteurs bactériens préférés selon l'invention sont par exemple les vecteurs pBR322 (ATCC n°37 017) ou encore les vecteurs tels que pAA223-3 (Pharmacia, Uppsala, Suède) et pGEM1 (Promega Biotech, Madison, WI, Etats-Unis).

On peut encore citer d'autres vecteurs commercialisés tels que les vecteurs pQE70, pQE60, pQE9 (Quiagen), psiX174, pBluescript SA, pNH8A, pMH16A, pN3H18A, pMH46A, pWLNEO, pSV2CAT, pOG44, pXTI et pSG (Stratagene).

Il peut s'agir également de vecteurs du type *Baculovirus* tel que le vecteur pVL1392/1393 (Pharmingen) utilisé pour transfecter les cellules de la lignée Sf9 (ATCC N°CRL 1711) dérivée de *Spodoptera frugiperda.*

De manière préférée, et pour l'application principale des vecteurs de l'invention consistant à obtenir une expression stable, et préférentiellement inductible, d'une séquence codant pour une protéine ACCS dans une plante, on aura recours à des vecteurs spécialement adaptés pour l'expression de séquences d'intérêt dans des cellules de plantes, tels que les vecteurs suivants:

- vecteur pBIN19 (BEVAN et al.), commercialisé par la Société CLONTECH (Palo Alto, Californie, USA);
- vecteur pBI 101 (JEFFERSON, 1987), commercialisé par la Société CLONTECH ;
- vecteur pBI121 (JEFFERSON, 1987), commercialisé par la Société CLONTECH;
- vecteur pEGFP; Yang et al. (1996), commercialisé par la Société CLONTECH;
- vecteur pCAMBIA 1302 (HAJDUKIIEWICZ et al., 1994)
- vecteurs intermédiaires et superbinaires dérivés des vecteurs pSB12 et pSB1 décrits par Japan Tobacco (EP 672 752 et Ishida et al., 1996).

A titre d'exemple, dans le système de contrôle divulgué dans la présente description, le gène (A/a), sous la forme de l'allèle (A), peut être introduit artificiellement dans la plante dicotylédone en utilisant l'acide nucléique de séquence SEQ ID N°7, qui comprend, de l'extrémité 5' vers l'extrémité 3' :

- une partie de la séquence d'un vecteur pEC2, localisée du nucléotide en position 1 jusqu'au nucléotide en position 633 de la séquence SEQ ID N°7,
- la séquence du site de restriction NotI, localisée du nucléotide en position 634 jusqu'au nucléotide en position 641 de la séquence SEQ ID N°7
- la séquence d'un acide nucléique comprenant le gène (A/a), sous la forme de l'allèle (A), localisé du nucléotide en position 642 jusqu'au nucléotide en position 14020 de la séquence SEQ ID N°7
- la séquence du site de restriction NotI, localisée du nucléotide en position 14021 jusqu'au nucléotide en position 14028 de la séquence SEQ ID N°7
- une partie de la séquence d'un vecteur pEC2, localisée du nucléotide en position 14029 jusqu'au nucléotide en position 16177 de la séquence SEQ ID N°7.

Ainsi, la séquence SEQ ID N°7 comprend un vecteur pEC2 linéarisé, dans lequel la séquence d'un acide nucléique comprenant le gène (A/a), sous la forme de l'allèle (A) a été insérée, en utilisant le site de restriction NotI du vecteur.

**Cellules**

Les méthodes les plus répandues pour introduire des acides nucléiques dans des cellules bactériennes peuvent être utilisées dans le cadre de cette invention. Ce peut être la fusion de cellules réceptrices avec des protoplastes bactériens contenant l'ADN, l'électroporation, le bombardement par projectiles, l'infection par des vecteurs viraux, etc. Les cellules bactériennes sont souvent utilisées pour amplifier le nombre de plasmides contenant le construit comprenant la séquence nucléotidique objet de l'invention. Les bactéries sont mises en culture et les plasmides sont ensuite isolés selon des méthodes bien connus de l'homme du métier (se reporter aux manuels de protocoles déjà cités), incluant les kits de purification de plasmides vendus dans le commerce comme par exemple EasyPrepI de Pharmacia Biotech ou QIAexpress Expression System de Qiagen. Les plasmides ainsi isolés et purifiés sont ensuite manipulés pour produire d'autres plasmides qui seront utilisés pour transfecter les cellules végétales.
Pour permettre l'expression d'un acide nucléique d'intérêt selon l'invention placé sous le contrôle d'une séquence régulatrice appropriée, les acides nucléiques ou les vecteurs recombinants définis dans la présente description doivent être introduits dans une cellule hôte. L'introduction des polynucléotides selon l'invention dans une cellule hôte peut être réalisée *in vitro,* selon les techniques bien connues de l'homme du métier.
L'invention décrit aussi une cellule hôte transformée par un acide nucléique selon l'invention ou par un vecteur recombinant tel que défini ci-dessus.
Une telle cellule hôte transformée est préférentiellement d'origine bactérienne, fongique ou végétale.
Ainsi, peuvent être notamment utilisées des cellules bactériennes de différentes souches de *Escherichia coli* ou encore d'*Agrobacterium tumefaciens.*
Avantageusement, la cellule hôte transformée est une cellule de plante ou encore un protoplaste de plante.
Parmi les cellules susceptibles d'être transformées selon le procédé de l'invention, on peut citer à titre d'exemples des cellules de plantes dicotylédone, de préférence appartenant à la famille des *cucurbitaceae,* dont les membres sont détaillées ci-après dans la partie intitulée « plantes selon l'invention ».
Les plantes hybrides obtenues par le croisement de plantes selon l'invention, font aussi partie de l'invention.
Préférentiellement, il s'agit d'une cellule ou d'un protoplaste d'une plante appartenant à l'espèce *cucumis melo.*
L'invention a également pour objet l'utilisation d'un acide nucléique d'intérêt, pour fabriquer une plante transformée dont le phénotype sexuel est modifié.
L'invention est également relative à l'utilisation d'un vecteur recombinant tel que défini dans la présente description pour fabriquer une plante transformée dont le phénotype sexuel est modifié.
L'invention concerne aussi l'utilisation d'un hôte cellulaire transformé par un acide nucléique d'intérêt, pour fabriquer une plante transformée dont le phénotype sexuel est modifié.
L'invention concerne aussi une plante transformée comprenant une pluralité de cellules hôtes telles que définies ci-dessus.

**Plantes transformées selon l'invention**

L'invention concerne aussi un organisme multicellulaire végétal transformé, caractérisé en ce qu'il comprend une cellule hôte transformée ou une pluralité de cellules hôtes transformées par au moins l'un des acides nucléiques tels que définis ci-dessus ou encore par un vecteur recombinant comprenant un tel acide nucléique.
La plante transformée peut contenir une pluralité de copies d'un acide nucléique codant pour la protéine ACCS, dans les situations dans lesquelles une surexpression de la protéine ACCS est recherchée. Une surexpression de la protéine ACCS est recherchée notamment lorsque l'on souhaite obtenir des plantes produisant des fleurs femelles, non capables d'autopollinisation.
L'invention est donc également relative à une plane transformée telle que définie ci-dessus dont les fleurs sont exclusivement femelles ou hermaphrodites.
Les plantes transformées selon l'invention, comprennent toutes au moins un élément choisi parmi les acides nucléiques, et les polynucléotides régulateurs définis ci-dessus artificiellement introduit dans leur génome.
Les plantes hybrides obtenues par le croisement de plantes transformées selon l'invention font également partie de l'invention.
L'invention concerne aussi toute partie d'une plante transformée telle que définie dans la présente description, telle que la racine, mais aussi les parties aériennes comme la tige, la feuille, la fleur et surtout la graine ou le fruit.
L'invention décrit encore une semence ou une graine de plante produit par une plante transformée telle que définie ci-dessus.
Typiquement, une telle semence transformée ou un tel grain transformé comprend une ou plusieurs cellules comprenant dans leur génome une ou plusieurs copies des premiers et seconds éléments génétiques de contrôle tels que définis ci-dessus, artificiellement introduits dans ladite plante dicotylédone permettant la synthèse de la protéine ACCS à un taux élevé ou à un taux faible, le cas échéant de manière contrôlée et inductible.

Selon un mode de réalisation préférentiel d'une plante transformée selon l'invention, on cherche à exprimer de manière contrôlée la protéine ACCS, ce qui implique que la plante transformée ne contient, en tant que copie fonctionnelle d'un polynucléotide codant pour la protéine ACCS, uniquement la ou les copies qui ont été artificiellement introduites dans leurs cellules, et préférentiellement dans leur génome, alors que les séquences du gène (A/a) codant pour ACCS, retrouvées naturellement dans la plante sauvage portent au moins une mutation provoquant un défaut dans l'expression du gène (A/a).

On décrit des plantes transformées dicotylédones, de préférence appartenant à la famille cucurbitaceae, et en particulier aux genres choisis parmi :

*Abobra, Acanthosicyos, Actinostemma, Alsomitra, Ampelosicyos, Anacaona, Apatzingania, Apodanthera, Bambekea, Benincasa, Biswarea, Bolbostemma, Brandegea, bryonia, Calycophysum, Cayaponia, Cephalopentandra, Ceratosanthes, Chalema, Cionosicyos, Citrullus, Coccinia, Cogniauxia, Corallocarpus, Cremastopus, Ctenolepis, Cucumella, Cucumeropsis, Cucumis, Cucurbita" Cucurbitella, Cyclanthera, Cyclantheropsis, Dactyliandra, Dendrosicyos, Dicoelospermum, Dieterlea, Diplocyclos, Doyerea, Ecballium, Echinocystis, Echinopepon, Edgaria, Elateriopsis, Eureiandra, Fevillea, Gerrardanthus, Gomphogyne, Gurania, Guraniopsis, Gymnopetalum, Gynostemma, Halosicyos, Hanburia, Helmontia, Hemsleya, Herpetospermum, Hodgsonia, Ibervillea, Indofevillea, Kedrostis, Lagenaria" Lemurosicyos, Luffa, Marah, Melancium, Melothria, Melothrianthus, Microsechium, Momordica, Muellerargia, Mukia, Myrmecosicyos, Neoalsomitra, Nothoalsomitra, Odosicyos, Oreosyce, Parasicyos, Penelopeia, Peponium, Peponopsis, Polyclathra, Posadaea, Praecitrullus, Pseudocyclanthera, Pseudosicydium, Psiguria, Pteropepon, Pterosicyos, Raphidiocystis, Ruthalicia, Rytidostylis, Schizocarpum, Schizopepon, Sechiopsis, Sechium, Selysia, Seyrigia, Sicana, Sicydium, Sicyos, Sicyosperma, Siolmatra, Siraitia,* Solena, *Tecunumania, Telfairia, Thladiantha, Trichosanthes, Tricyclandra, Trochomeria, Trochomeriopsis, Tumamoca, Vaseyanthus, Wilbrandia, Xerosicyos, Zanonia, Zehneria, Zombitsia, ou Zygosicyos.*

De préférence, les plantes transformées appartiennent au genre *cucumis,* et à l'espèce *Cucumis Melo.*

## Procédés de détection selon l'invention

L'identification du système de contrôle du développement du type floral par les inventeurs, a permis de mettre au point des procédés de détection du phénotype sexuel des plantes extrêmement simples, qui sont détaillés ci-après.

Procédé de détection de la présence d'un allèle (A) ou (a), ladite méthode comprenant les étapes suivantes :

1) mettre en contact une sonde ou une pluralité de sondes nucléotidiques telles que définies ci-dessus avec l'échantillon à tester ; et
2) détecter le complexe éventuellement formé entre la ou les sondes et l'acide nucléique présent dans l'échantillon.

Procédé de détection de la présence d'un allèle (G) ou (g), ladite méthode comprenant les étapes de :

1) mettre en contact une sonde ou une pluralité de sondes nucléotidiques telles que définies ci-dessus avec l'échantillon à tester ; et
2) détecter le complexe éventuellement formé entre la ou les sondes et l'acide nucléique présent dans l'échantillon.

Ces deux procédés de détection permettent de sélectionner des plantes dont les phénotypes et génotypes ont été résumés dans le tableau 1.

La détection du complexe entre un acide nucléique et une sonde peut se faire par toute technique connue de l'homme du métier, et en particulier en utilisant des sondes ou amorces marquées, telles que décrites dans la partie « Sondes et amorces selon l'invention ».

Ces procédés sont particulièrement avantageux car ils évitent de devoir cultiver une plante dicotylédone, pour connaître son phénotype sexuel. Il est ainsi possible de réaliser la détection du phénotype sexuel d'un très grand échantillon de plantes, à moindre coût.

De plus ces procedes permettent de selectionner de caracteres phenotypiques à expression tardives tel l'apparition du phenotype sexuel des fleurs sur des plantes à un stade très jeune (plantule avec les premieres feuilles. L'application ci dessus permet un gain de temps et d'espace considerable.

Les inventeurs ont montré (exemple 1) que l'allèle (A) et l'allèle (a) sont associés à un polymorphisme nucléotidique simple (SNP). Ainsi, l'allèle (A) de séquence SEQ ID n°1 comprend, de la position 6074 à la position 6077, une séquence AGCT, se traduisant par la présence dans la protéine ACCS de SEQ ID N°3, d'un résidu alanine en position 54. L'allèle (a) de séquence SEQ ID n°2 comprend, de la position 3817 à la position 3820, une séquence AGTT, se traduisant par la présence dans la protéine ACCS d'un résidu valine en position 54.

Les inventeurs ont montré que parmi les deux séquences identifiées ci-dessus, seule la séquence AGCT en position 6074 à 6077 de la séquence SEQ ID N°1 correspondant à l'allèle (A) et présentant un résidu cytosine en position 6076 constitue un site de restriction pour l'enzyme Alu I. En conséquence, la méthode dite de « digestion enzymatique des fragments amplifiés » (En anglais : Cleaved Amplified Polymorphic Sequence Marker ou CAPS) connue de l'homme du métier peut être utilisée pour identifier la présence de l'allèle (A) ou de l'allèle (a) dans une plante.

Dans cette méthode, une étape d'amplification par PCR est réalisée, en utilisant un couple d'amorces particulier répondant aux critères suivants :

- le couple d'amorces encadre la région du SNP,
- le couple d'amorces permet d'amplifier l'allèle (A) et l'allèle (a),
- le couple d'amorces permet d'observer après digestion enzymatique par Alu I un nombre de fragments de restrictions différent selon que l'allèle (A) ou l'allèle (a) a été amplifié.

A titre d'exemple un tel couple d'amorces comprend les séquences SEQ ID N°9 et SEQ ID N°10.

Dans une seconde étape, les produits issus de la PCR sont mis en présence de l'enzyme de restriction Alu I, dans des conditions permettant un clivage.

Les produits de PCR ainsi digérés ou non digérés par l'enzyme, en fonction de leur séquence nucléotidique sont ensuite discriminés par des techniques usuelles, par exemple en fonction de leur taille.

En appliquant cette méthode, l'homme du métier peut aisément discriminer une plante portant l'allèle (A) d'une plante portant l'allèle (a) puisque les produits PCR issus d'une plante portant l'allèle (A) seront digérés par l'enzyme de restriction au niveau du SNP. Ces produits de PCR pourront aisément être distingués des produits PCR issus d'une plante portant l'allèle (a), non digérés par l'enzyme de restriction au niveau du SNP.

L'invention a donc également pour objet un procédé de détection de la présence d'un allèle (A) ou (a), ladite méthode comprenant les étapes suivantes :

- amplifier par PCR l'ADN d'un échantillon à analyser en utilisant les amorces de séquence SEQ ID N°11 et SEQ ID N°12,
- digérer le produit obtenu par l'enzyme de restriction Alu I, et
- détecter les fragments de restriction obtenus.

Pour détecter les fragments de restrictions obtenus, l'homme du métier procédera par exemple à une électrophorèse de manière à pouvoir détecter les fragments en fonction de leur taille.

Pour les plantes portant l'allèle (A) 4 fragments de restrictions sont obtenus. Leur taille est de 327, 197, 137 et 116 pb.

Pour les plantes portant l'allèle (a) 3 fragments de restrictions sont obtenus, leur taille est de 524, 137 et 116 pb.

Le procédé permet donc une détection facile du phénotype sexuel d'une plante.

## Procédés de sélection selon l'invention

Les procédés de détection ci-dessus peuvent être mis en oeuvre dans les procédés de sélection détaillés ci-après.

L'invention a pour objet un procédé de sélection du type floral d'une plante appartenant au genre cucurbitaceae, caractérisé en ce qu'il comprend une étape consistant à :

a) déterminer la présence des allèles (A) et (a), dans une plante d'intérêt appartenant à la famille des cucurbitaceae, par exemple en utilisant les acides nucléiques tels que définis ci-dessus, ou un anticorps dirigé contre la protéine ACCS et
b) sélectionner positivement la plante qui possède l'allèle (A) ou l'allèle (a) dans son génome.

L'invention décrit également un procédé de sélection du type floral d'une plante appartenant au genre cucurbitaceae, caractérisé en ce qu'il comprend une étape consistant à :

a) déterminer la présence des allèles (G) et (g), dans une plante d'intérêt appartenant à la famille des cucurbitaceae, par exemple en utilisant les acides nucléiques tels que définis ci-dessus, et
b) sélectionner positivement la plante qui possède l'allèle (G) ou l'allèle (g) dans son génome.

La détermination de la présence des allèles (A), (a), (G) et (g) peut être avantageusement effectuée en mettant en oeuvre les procédés de détection ci-dessus.

L'homme du métier peut aisément combiner les procédés de sélection définis ci-dessus, en se référant au tableau 1, donnant la correspondance entre génotype et phénotype, pour obtenir des plantes de phénotype uniquement femelle ou hermaphrodites, par exemple.

**Procédés d'obtention d'une plante transformée selon l'invention**

L'invention concerne tout d'abord un procédé pour l'obtention d'une plante transformée visant à insérer l'allèle (A) dans une plante ne comprenant pas cet allèle.

L'invention décrit ainsi un procédé pour l'obtention d'une plante transformée, appartenant à la famille cucurbitaceae, portant des fleurs femelles, caractérisé en ce qu'il comporte les étapes suivantes :

a) transformation d'au moins une cellule végétale d'une plante d'intérêt ne portant pas l'allèle (A), dans son génome, par une séquence nucléotidique (NA); ou un vecteur recombinant comprenant un tel acide nucléique ;
b) sélection des cellules transformées obtenues à l'étape a) ayant intégré dans leur génome, l'acide nucléique (NA) ;
c) Régénération d'une plante transformée à partir des cellules transformées obtenues à l'étape b).

Ce type de procédé est particulièrement utile en ce qu'il permet de d'insérer l'allèle (A) dans le génome d'une plante, qui présentera ainsi un phénotype monoïque ou gynoïque.

L'invention décrit egalement un procédé de transformation de plantes visant à supprimer l'allèle (A) dans une plante, ou a remplacer l'allèle (A) par un allèle (a) de sorte à obtenir une plante de phénotype hermaphrodite.

L'invention décrit donc un procédé pour l'obtention d'une plante transformée, appartenant à la famille cucurbitaceae, portant des fleurs hermaphrodites, caractérisé en ce qu'il comporte les étapes suivantes :

a) le remplacement dans une plante, de l'allèle (A), par un allèle (a),
b) sélection des cellules transformées obtenues à l'étape a) ayant intégré dans leur génome l'allèle (a).
c) Régénération d'une plante transformée à partir des cellules transformées obtenues à l'étape b),
d) croisement de plantes obtenues à l'étape c) pour l'obtention d'une ne portant plus d'allèle (A).

Dans un premier mode de réalisation, du procédé ci-dessus, l'étape a) consiste à transformer une plante comportant l'allèle (A) dans son génome, par un acide nucléique de type « antisens » tel que défini ci-dessus, et en sélectionnant les plantes ne comportant plus l'allèle (A).

Un résultat identique peut être obtenu en mettant en oeuvre des phénomènes de recombinaison homologue visant à remplacer tout ou partie de l'acide nucléique (NA) par un acide nucléique de structure altérée, qui ne permet pas d'obtenir un phénotype correspondant à l'allèle (A).

Un tel acide nucléique de structure altérée, peut consister en un polynucléotide régulateur (Pa), ou un acide nucléique codant pour une protéine ACCS altérée.

L'invention décrit ainsi un procédé pour l'obtention d'une plante transformée, appartenant à la famille cucurbitaceae, portant des fleurs hermaphrodites, caractérisé en ce qu'il comporte les étapes suivantes :

a) transformation d'au moins une cellule végétale d'une plante d'intérêt comportant un allèle (A) par un polynucléotide régulateur (Pa) ou par un acide nucléique codant pour une protéine ACCS altérée; ou un vecteur recombinant comprenant un tel acide nucléique ;
b) sélection des cellules transformées obtenues à l'étape a) ayant intégré dans leur génome au moins une copie d'un polynucléotide régulateur (Pa) ou un acide nucléique codant pour une protéine ACCS altérée.
c) Régénération d'une plante transformée à partir des cellules transformées obtenues à l'étape b),
d) croisement de plantes obtenues à l'étape c) pour l'obtention d'une ne portant plus d'allèle (A).

Ce type de procédé est particulièrement utile en ce qu'il permet d'obtenir des plantes ne comprenant plus d'allèle (A), et qui sont de type andromonoïque ou hermaphrodite.

L'invention concerne également un procédé de transformation de plantes visant à insérer l'allèle (G).

L'invention décrit ainsi un procédé pour l'obtention d'une plante transformée, appartenant à la famille cucurbitaceae, portant des fleurs fernelles, caractérisé en ce qu'il comporte les étapes suivantes :

a) transformation d'au moins une cellule végétale d'une plante d'intérêt ne comprenant pas l'allèle (G), dans son génome, par une séquence nucléotidique (NG); ou un vecteur recombinant comprenant un tel acide nuciéique ;
b) sélection des cellules transformées obtenues à l'étape a) ayant intégré dans leur l'acide nucléique (NG) ;

c) Régénération d'une plante transformée à partir des cellules transformées obtenues à l'étape b).

L'invention concerne également un procédé de transformation de plantes visant à remplacer l'allèle (G) par l'allèle (g).

L'invention décrit ainsi un procédé pour l'obtention d'une plante transformée, appartenant à la famille cucurbitaceae, portant des fleurs hermaphrodites, caractérisé en ce qu'il comporte les étapes suivantes :

a) le remplacement dans une plante, de l'allèle (G), par un allèle (g),
b) sélection des cellules transformées obtenues à l'étape a) ayant intégré dans leur génome l'allèle (g).
c) Régénération d'une plante transformée à partir des cellules transformées obtenues à l'étape, b),
d) croisement de plantes obtenues à l'étape c) pour l'obtention d'une ne portant plus d'allèle (G).

Les procédés ci-dessus peuvent être combinés entre eux en se fondant sur le tableau 1, de sorte à obtenir des plantes exclusivement femelles ou exclusivement hermaphrodites, dont l'intérêt industriel a été discuté ci-dessus.

Pour simplifier les procédés pour l'obtention d'une plante transformée exclusivement femelle ou exclusivement hermaphrodite, il est possible de réaliser une étape préalable dans les procédés définis ci-dessus, au cours de laquelle des mutations des gènes (A/a) et (G/g) présents naturellement dans la plante sont effectuées, par exemple par insertion au hasard du transposon *Mutator* dans une population de plantes de phénotype sauvage, puis en détectant chez les mutants obtenus, ceux d'entre ces mutants qui sont de génotype (aagg), par exemple à l'aide des sondes ou des amorces nucléotidiques décrites dans les exemples.

Selon ce mode de réalisation préférentiel, la plante transformée selon l'invention est caractérisée en ce qu'elle possède un génotype (aagg) et présente des fleurs exclusivement hermaphrodites.

Dans un mode de réalisation des procédés d'obtention d'une plante transformée définis ci-dessus, le polynucléotide (NA) lorsqu'il est utilisé comprend un polynucléotide régulateur (PA) activateur inductible.

L'invention décrit donc également un procédé pour l'obtention de graines de plantes dont le développement produit des plantes portant des fleurs femelles, comportant les étapes suivantes :

a) cultiver une plante d'intérêt ne portant pas l'allèle (A) tel que défini dans la revendication 1 dans son génome, transformée par une séquence nucléotidique (NA) comprend un polynucléotide régulateur (PA) activateur inductible ; ou par un vecteur recombinant comprenant cet acide nucléique ;
en l'absence d'un signal inducteur auquel le polynucléotide activateur inductible est sensible.
b) mettre en contact la plante transformée définie en a) avec le signal activateur inductible auquel le polynucléotide activateur inductible est sensible,
c) récupérer les graines matures, dont le développement produit exclusivement des plantes portant des fleurs femelles.

Dans un autre mode de réalisation, un polynucléotide régulateur (Pa) répresseur inductible est utilisé pour remplacer le polynucléotide régulateur naturellement présent dans la plante, et permettre de faire baisser le taux de protéine ACCS à un moment déterminé.

***-Procédés préférés d'obtention d'une plante portant exclusivement des fleurs femelles***

De manière tout à fait préférée, on décrit un procédé d'obtention d'une plante portant exclusivement des fleurs femelles, caractérisé en ce qu'il consiste à :

- détecter les allèles (A), (a), (G) et (g) en mettant en oeuvre les procédés de détection ci-dessus, et
- obtenir une plante comprenant au moins une copie de l'allèle (A) et aucune copie de l'allèle (G), en mettant en oeuvre les procédés de sélection ou les procédés d'obtention d'une plante transformée tels que définis ci-dessus.

Les plantes obtenues selon le procédé ci-dessus portent exclusivement des fleurs femelles, et sont donc particulièrement intéressantes d'un point de vue industriel, puisqu'elles ne sont pas capables d'auto pollinisation. Ces plantes peuvent donc être utilisées dans des procédés de sélection en vue d'obtenir des plantes hybrides.

***-Procédés préférés d'obtention d'une plante portant exclusivement des fleurs hermaphrodites***

De manière tout à fait préférée, on décrit un procédé d'obtention d'une plante portant exclusivement des fleurs hermaphrodites, caractérisé en ce qu'il consiste à :

- détecter les allèles (A), (a), (G) et (g) en mettant en oeuvre les procédés de détection définis ci-dessus, et
- obtenir une plante ne comprenant aucune copie de l'allèle (A) et aucune copie de l'allèle (G), en mettant en oeuvre les procédés de sélection ou les procédés d'obtention d'une plante transformée tels que définis ci-dessus.

Les plantes obtenues selon le procédé ci-dessus portent exclusivement des fleurs hermaphrodites, et sont donc particulièrement intéressantes d'un point de vue industriel, puisqu'elles ne sont capables d'auto pollinisation. Ces plantes peuvent donc être utilisées dans des procédés de création de lignées pures.

**<u>Procédés de transformation des plantes selon l'invention</u>**

Les méthodes les plus répandues pour introduire des acides nucléiques dans des cellules végétales peuvent être utilisées dans le cadre de cette invention.

La transformation de cellules végétales peut être réalisée par diverses méthodes telles que, par exemple, le transfert des vecteurs susmentionnés dans les protoplastes végétaux après incubation de ces derniers dans une solution de polyéthylèneglycol en présence de cations divalents (Ca 2+), l'électroporation (Fromm et al. 1985), l'utilisation d'un canon à particules, ou la micro-injection cytoplasmique ou nucléaire (Neuhaus et al, 1987).

Une des méthodes de transformation de cellules végétales pouvant être utilisée dans le cadre de l'invention est l'infection des cellules végétales par un hôte cellulaire bactérien comprenant le vecteur contenant la séquence d'intérêt. L'hôte cellulaire peut être *Agrobacterium tumefaciens* (An *et al.* 1986), ou A. *rhizogenes* (Guerche *et al.* 1987).

De manière préférentielle, la transformation des cellules végétales est réalisée par le transfert de la région T du plasmide circulaire extrachromosomique inducteur de tumeurs Ti d'A. *tumefaciens,* en utilisant un système binaire (Watson et al., 1994). Pour ce faire, deux vecteurs sont construits. Dans un de ces vecteurs, la région d'ADN-T a été éliminée par délétion, à l'exception des bords droit et gauche, un gène marqueur étant inséré entre eux pour permettre la sélection dans les cellules de plantes. L'autre partenaire du système binaire est un plasmide Ti auxiliaire, plasmide modifié qui n'a plus d'ADN-T mais contient toujours les gènes de virulence *vir* nécessaires à la transformation de la cellule végétale. Ce plasmide est maintenu dans *Agrobacterium.*

Selon un mode préféré, la méthode décrite par Ishida et al. (1996) peut être appliquée pour la transformation des dicotylédones. Selon un autre protocole, la transformation est réalisée selon la méthode décrite par Finer et al. (1992) utilisant le canon à particules de tungstène ou d'or.

**<u>Exemples</u>**

<u>Exemple 1 Identification d'un polymorphisme nucléotidique simple (SNP)</u>

L'analyse de la séquence d'un fragment d'ADN contenant le promoteur de la région du gène (A/a) (2kb en amont du codon d'initiation) révèle un taux important de polymorphisme dans la région régulatrice en 5' et dans les séquences introniques, alors que seule une mutation ponctuelle dans la séquence codante de la protéine a été identifiée (figure 1A). Cette mutation ponctuelle au niveau de l'acide nucléique se traduit au niveau de la séquence polypeptidique de la protéine ACCS, par le remplacement d'un résidu alanine en position 57 par un résidu valine (figure 1 B). En utilisant des méthodes de prédiction de substitution d'acides aminés, sur la base d'homologies de séquences et de propriétés physiques, il a été recherché si la mutation ponctuelle identifiée dans l'allèle a est dommageable pour la fonction protéique.

Le logiciel SIFT (Ng PC and Henikoff S., 2001)prédit que la substitution Ala57Val est grandement dommageable pour la fonction A. De plus, des analyses de structures cristallines de l'ACC synthase de pomme et de tomate (Capitani et al., 2002 ; Huai et al., 2001) mettent en exergue le rôle important de cet acide aminé.

Suite à l'identification de ce polymorphisme nucléotidique simple (ou SNP), des analyses d'haplotypes et d'associations dans une collection de 30 germplasmes de melon ont révélé que ce SNP est totalement associé au phénotype sexuel. Toutes les entrées monoïquesportent une alanine en position 57 alors que toutes les entrées andromonoïques portent une valine. Aucune exception n'a été trouvée ni aucun autre type de changement d'acide aminé à cette position.

Enfin, ce polymorphisme nucléotidiques simple a été analysé dans le but d'identifier des enzymes de restriction qui pourraient être utilisées pour développer une méthode dite de « digestion enzymatique des fragments amplifiés » (En anglais : Cleaved Amplified Polymorphic Sequence Marker ou CAPS). Il apparaît que la substitution du nucléotide C en un nucléotide T conduit à une perte d'un site de restriction Alu I dans l'allèle a.

<u>Exemple 2 Expression spatiale et temporelle de l'élément génétique de contrôle (A/a)</u>

Pour étudier l'expression de l'élément génétique de contrôle A/a sous la forme de l'allèle A, des hybridations *in situ* ont été effectuées en utilisant des sondes spécifiques de l'allèle A sur des plantes, et plus précisément sur des méristèmes floraux de plantes mâles, femelles et hermaphrodites, de génotype *AA* GG, aa GG, *AA* gg et *aa gg.* Dans les méristèmes floraux A l'expression est localement forte et le signal d'hybridation est détecté de manière spécifique dans les primordia des carpelles des fleurs femelles et hermaphrodites des plantes monoïques, andromonoïques, gynoïques et hermaphrodites. En se referant aux différents stades de développement de la fleur décrit pour le concombre (Bai et al., 2004), il apparaît que chez le melon le gène (A/a) est exprimé à un stade précoce du développement des méristèmes floraux avant qu'une distinction morphologique puisse être effectuée entre les fleurs mâles et les fleurs femelles.

Dans les fleurs mâles et hermaphrodites, aucune expression n'est détectée dans les anthères. Ces données indiquent que l'expression de l'allèle (A) dans les carpelles des fleurs femelles empêche le développement des étamines. Du fait que, dans les fleurs hermaphrodites, l'allèle récessif (a) présente le même profil d'expression que l'allèle A dans les fleurs femelles, on peut conclure que la fonction du gène A depend de sa tissu-spécificité d'expression ainsi que de la nature de la protéine ACCS synthétisée.

<u>Exemple 3 Transgénèse chez Arabidopsis thaliana</u>

Les effets potentiels du gène A/a, et de la protéine ACCS sur le phénotype sexuel floral et l'architecture de la fleur de plantes n'appartenant pas aux cucurbitacées ont été étudiés par transformation *d'Arabidopsis thaliana* par *Agrobacterium.* Les plantes transgéniques d' *Arabidopsis* portantnt l'allèle de melon A ou a présentent un phenotype au niveau de l'architecture florale et des siliques. (figures 2A et 2B). En effet les siliques des transformants *d'Arabidopsis* sont plus courtes que celles d'une plante Arabidopsis sauvage et l'architecture des fleurs des transformants d'Arabidopsis est tres affectée. Ces résultats permettent d'étendre l'utilisation du gène de melon (A/a) à des plantes dicotylédones n'appartement pas à la famille des cucurbitacées.

**<u>Références</u>**

An et al., (1986) Plant Physiol. 81, 86-91
Aoyama T et al., (1997) The Plant Journal, vol.11 (3):605-612.
Ausubel et al., (1997) Current protocols in molecular biology
Bai et al., 2004 Planta 220 : 230-240
Beaucage et al. (1981), Tetrahedron Lett., 22:1859-1862.
Berbal, 1984 .
Bevan et al., Nucleic Acids Research, vol. 12:8711-8721.
Brown et al. (1979), Methods Enzymol., 68:109-151.
Causse et al. (1995) Molecular Breeding 1: 259-272.
Capitani et al., 2002 J. Biol. Chem 51 : 49735-49742
Christensen et al. (1996), Transgenic. Res., 5:213
Finer et al. (1992) Plant Cell Report, 11, 323-328
FROMM M. et al. (1990), Biotechnology, 8:833-839
GAIT (ed.), (1984). Nucleic Acid Hybridization.
GORLACH J, VOLRATH S, KNAUF-BEITER G, HENGY G, BECKHOVE U, KOGEL KH, OOSTENDORP M, STAUB T, WARD E, KESSMANN H, RYALS J. (1996) Benzothiadiazole, a novel class of inducers of systemic acquired résistance, activates gene expression and disease résistance in wheat. Plant Cell 8:629-43
GLOVER (ed.), 1985. DNA Cloning: A Practical Approach, Volumes I and II Oligonucleotide Synthesis, MRL Press, Ltd., Oxford, U.K.
Guerche et al. (1987), Mol Gen. Genet 206, 382
HAMES and HIGGINS, 1985. Nucleic Acid Hybridization: a practical approach, Hames & Higgins Ed. IRL Press, Oxford.
HAJDUKIEWICZ, P. SVAB. Z. AND MALIGA P. Plant Mol. Biol. 25 (6), 989-994 (1994).
Huai et al., 2001 J ; Biol. Chem 41 : 38210-38216
Ishida et al. (1996) Nature biotechnology 14, 745-750
JEFFERSON, 1987, Plant Molecular Biology Reporter, vol.5:387-405.
Kay et al., (1987) Science 236, 4805
Kahana, A., Silberstein, L., Kessler, N., Goldstein, R. S. and Perl-Treves, R. (2000) expression of ACC oxidase genes differs among sex genotypes and sex phases in cucumber. Plant Mol Biol. Nov ; 41 (4): 517-528.
Kamachi, S., Sekimoto, H., Kondo, N. & Sakai, S., (1997). Cloning of a cDNA for a 1-aminocyclopropane-1-carboxylate synthase that is expressed durig development of female flowers at the apices of Cucumis sativus L. The Plant Cell Physiol., 38:1197-206.
Kohler G and Milstein C;, (1975), Nature, volume 256:495
KOOTER, JM., MATZKE, MA., AND MEYER, P. (1999) Listening to the silent genes: transgene silencing, gene regulation and pathogen control. Trends Plant Sci. 4, 430-437
Kozbor et al., (1983), Hybridoma, vol.2 (1):7-16.
Leger OJ et al., (1997), Hum Antibodies, vol.8 (1):3-16
Martineau P et al., (1998), J. Mol. Biol. vol.280(1):117-127.
Martinez, A., Sparks, C., Hart, CA., tompson, J., and Jepson, I. (1999) Ecdysone agonist inducible transcription in transgenic tobacco plants. Plant J. 19:97-106
McNELLIS T W, 1998, The Plant Journal, vol.14 (2): 247-257
Molina A, Hunt MD, Ryals JA (1998) Impaired fungicide activity in plants blocked in disease résistance signal transduction. Plant Cell 10:1903-14 NARANG et al. (1979), Methods Enzymol., 68: 90-98.
Neuhaus et al., (1987).Theor. Appl. Genet. 75(1), 30-36
Ng PC and Henikoff S. (2001) Genome Res. 11 (5):863-874
Reinmann KA et al. (1997), Aids Res. Hum retroviruses, vol.13 (11):933-943. Ridder R. et al., (1995), Biotechnology (NY), vol.13 (3):255-260.
SALTER MG et al., 1998, vol.16 (1): 127-132
Risser, G.and Rode, J.C., 1979. Induction par le nitrate d'argent de fleurs staminées chez des plantes gynoïques de melon (Cucumis melo L.). Annales de l'Amélioration des Plantes, 29:349-352.
Rudich, J., Halevy, A.H. and Kedar, N., 1969. Increase of femaleness of three cucurbits by treatment with Ethrel, an ethylene-releasing compound. Planta, 86:69-76.
Sambrook et al., (2001), Molecular Cloning A-laboratory manual
Sanchez Pescador, (1988), J. Clin. Microbiol., 26 (10):1934-1938.
Urdea et al. (1988), Nucleic Acids Research, 11:4937-4957.
WASSENEGGER, M., AND PÉLISSIER, T. (1998) A model for RNA-mediated gene silencing in higher plants Plant Mol. Biol. 37, 349-362
Watson et al. (1994) ADN recombinant, Ed. De Boek Université, 273-292 YANG F. MOSS LG, PHILIPS GN JR. Nat. Biotechnol. 1996 Oct. 14(10):1246-51.
YANG TT, CHENG L. KAIN SR, Nucleic acids Res. 1996, Novembre 15; 24(22):4592-3.

## Revendications

**1.** Acide nucléique comprenant, de l'extrémité 5' vers l'extrémité 3', au moins :

(i) une séquence ayant au moins 95% d'identité avec le polynucléotide allant du nucléotide 5907 au nucléotide 6086 de la séquence SEQ ID N°1,
(ii) une séquence ayant au moins 95% d'identité avec le polynucléotide allant du nucléotide 6181 au nucléotide 6467 de la séquence SEQ ID N°1, et
(iii) une séquence ayant au moins 95% d'identité avec le polynucléotide allant du nucléotide 7046 au nucléotide 7915 de la séquence SEQ ID N°1.

**2.** Acide nucléique, tel que défini dans la revendication 1, de séquence SEQ ID N°1.

**3.** Acide nucléique sous forme d'allèle (a), tel que défini dans la revendication 1, de séquence SEQ ID N°2.

**4.** Acide nucléique codant un polypeptide possédant au moins 95 % d'identité en acides aminés avec la séquence SEQ ID N°3.

**5.** Acide nucléique comprenant une séquence nucléodique allant du nucléotide 1 au nucléotide 5906 de la séquence SEQ ID N°1.

**6.** Acide nucléique comprenant, de l'extrémité 5' vers l'extrémité 3', au moins :

(i) une séquence allant du nucléotide 1 au nucléotide 5906 de la séquence SEQ ID N°1,
(ii) une séquence allant du nucléotide 5907 au nucléotide 6086 de la séquence SEQ ID N°1,
(iii) une séquence allant du nucléotide 6181 au nucléotide 6467 de la séquence SEQ ID N°1, et
(iv) une séquence allant du nucléotide 7046 au nucléotide 7915 de la séquence SEQ ID N°1,

et portant, par rapport à la séquence SEQ IDN°1 , une mutation de nature (i) à conduire à une expression réduite de la protéine ACCS de séquence SEQ ID N°3 ou (ii) à induire la synthèse d'une protéine ACCS non active.

**7.** Acide nucléique comprenant une séquence allant du nucléotide 1 au nucléotide 3650 de la séquence SEQ ID N°2.

**8.** Vecteur recombinant comprenant un acide nucléique tel que défini dans l'une quelconque des revendications 1 à 7.

**9.** Cellule hôte comprenant un vecteur recombinant selon la revendication 8.

**10.** Cellule hôte selon la revendication 9, **caractérisée en ce qu'**il s'agit d'une cellule de plante appartenant à la famille des cucurbitacées.

**11.** Plante appartenant à la famille des cucurbitacées, comprenant un vecteur recombinant selon la revendication 8.

**12.** Plante transformée comprenant une pluralité de cellules hôtes selon la revendication 9 ou 10

**13.** Acide nucléique, utilisable en tant que sonde ou amorce, choisie parmi :

- un acide nucléique comprenant au moins 60 nucléotides consécutifs d'un acide nucléique de séquence SEQ ID N°1 ou SEQ ID N°2 ou de sa séquence complémentaire,
- un acide nucléique comprenant au moins 15 nucléotides consécutifs de l'une des séquences 5907-6086, 6181-6467 et 7046-7915 de la séquence SEQ ID N°1, et
- un acide nucléique comprenant au moins 15 nucléotides consécutifs de l'une des séquences 3615-3830, 3924-4209 et 4790-5659 de la séquence SEQ ID N°2.

**14.** Procédé de détection de la présence d'un allèle (A) ou (a),

- ledit allèle dominant A consistant en un acide nucléique (NA) comprenant

(i) un polynucléotide régulateur (PA) fonctionnel dans une plante de la famille des cucurbitacées, et
(ii) un acide nucléique dont l'expression est régulée par le polynucléotide régulateur (PA), ledit acide nucléique codant pour la protéine aminocyclopropane carboxylate synthase (ACCS) de séquence SEQ ID N°3, et

- ledit allèle récessif (a), se distinguant de l'allèle dominant (A) par :

(i) un acide nucléique (NA) non présent dans la plante, ou
(ii) un polynucléotide régulateur (Pa) non fonctionnel dans une plante de la famille des cucurbitacées, ou
(iii) un acide nucléique codant pour une protéine ACCS non active, ou
(iv) un polynucléotide régulateur (Pa) non fonctionnel dans une plante de la famille des cucurbitacées, et un acide nucléique codant pour une protéine ACCS non active, et

ladite méthode comprenant les étapes suivantes :

1) mettre en contact une sonde ou une pluralité de sondes nucléotidiques selon la revendication 13 avec un échantillon à tester ;
2) détecter le complexe éventuellement formé entre la ou les sondes et l'acide nucléique présent dans l'échantillon.

**15.** Procédé selon la revendication 14, **caractérisé en ce qu'**il comprend le fait de déterminer la présence d'une séquence AGCT correspondant à l'allèle (A), ou la présence d'une séquence AGTT correspondant à la présence de l'allèle (a).

**16.** Procédé de détection de la présence d'un allèle (A) ou (a), ladite méthode comprenant les étapes suivantes :

1) amplifier par PCR l'ADN d'un échantillon à analyse en utilisant un couple d'amorces encadrant la région du polymorphisme nucléotidique simple (SNP) qui s'étend du nucléotide en position 6074 au nucléotide en position 6077 de la séquence SEQ ID N°1.
2) digérer le produit obtenu par l'enzyme de restriction Alu I, et
3) détecter les fragments de restriction obtenus.

**17.** Procédé de sélection du type floral d'une plante appartenant à la famille des cucurbitacées, **caractérisé en ce qu'**il comprend une étape consistant à :

a) détecter la présence des allèles (A) et (a), dans une plante d'intérêt appartenant à la famille des cucurbitacées selon un procédé de détection tel que défini dans l'une quelconque des revendications 14 à 16, et
b) sélectionner positivement la plante qui possède l'allèle (A) ou l'allèle (a) dans son génome.

**18.** Procédé pour l'obtention d'une plante transformée, appartenant à la famille des cucurbitacées, portant des fleurs femelles, **caractérisé en ce qu'**il comporte les étapes suivantes :

a) transformation d'au moins une cellule végétale d'une plante d'intérêt ne comprenant pas un acide nucléique selon l'une des revendications 1, 2, 4 et 5, dans son génome, par un vecteur recombinant comprenant un tel acide nucléique ;
b) sélection des cellules transformées obtenues à l'étape a) ayant intégré dans leur génome ledit acide nucléique;
c) régénération d'une plante transformée à partir des cellules transformées obtenues à l'étape b).

**Patentansprüche**

**1.** Nucleinsäure, umfassend in 5'→3'-Richtung wenigstens:

(i) eine Sequenz mit wenigstens 95% Identität mit dem Polynucleotid, das sich von Nucleotid 5907 bis zu Nucleotid 6086 der Sequenz SEQ ID Nr. 1 erstreckt;
(ii) eine Sequenz mit wenigstens 95% Identität mit dem Polynucleotid, das sich von Nucleotid 6181 bis zu Nucleotid 6467 der Sequenz SEQ ID Nr.1 erstreckt; und
(iii) eine Sequenz mit wenigstens 95% Identität mit dem Polynucleotid, das sich von Nucleotid 7046 bis zu Nucleotid 7915 der Sequenz SEQ ID Nr. 1 erstreckt.

**2.** Nucleinsäure gemäß Anspruch 1 mit der Sequenz SEQ ID Nr. 1.

**3.** Nucleinsäure in Form eines Allels (a) gemäß Anspruch 1 mit der Sequenz SEQ ID Nr. 2.

**4.** Nucleinsäure, die ein Polypeptid codiert, das wenigstens 95% Aminosäure-identität mit der Sequenz SEQ ID Nr. 3 besitzt.

**5.** Nucleinsäure, umfassend eine Nucleotidsequenz, die sich von Nucleotid 1 bis Nucleotid 5906 der Sequenz SEQ ID Nr. 1 erstreckt.

**6.** Nucleinsäure, umfassend in 5'→3'-Richtung wenigstens:

(i) eine Sequenz, die sich von Nucleotid 1 is zu Nucleotid 5906 der Sequenz SEQ ID Nr. 1 erstreckt;
(ii) eine Sequenz, die sich von Nucleotid 5907 bis zu Nucleotid 6086 der Sequenz SEQ ID Nr. 1 erstreckt;
(iii) ein Sequenz, die sich von Nucleotid 6181 bis zu Nucleotid 6467 der Sequenz SEQ ID Nr. 1 erstreckt; und
(iv) eine Sequenz, die sich von Nucleotid 7046 bis zu Nucleotid 7915 der Sequenz SEQ ID Nr. 1 erstreckt;

und tragend, in Bezug auf die Sequenz SEQ ID Nr. 1, eine Mutation der Art (i), die zu einer reduzierten Expression des ACCS-Proteins der Se-quenz SEQ ID Nr. 3 führt, oder (ii), die die Synthese eines inaktiven ACCS-Proteins induziert.

**7.** Nucleinsäure, umfassend eine Sequenz, die sich von Nucleotid 1 bis Nucleotid 3650 der Sequenz SEQ ID Nr. 2 erstreckt.

**8.** Rekombinanter Vektor, der eine Nucleinsäure gemäß einem der Ansprüche 1 bis 7 umfasst.

**9.** Wirtszelle, umfassend einen rekombinanten Vektor gemäß Anspruch 8.

**10.** Wirtszelle gemäß Anspruch 9, **dadurch gekennzeichnet, dass** es sich um eine Zelle einer zur Familie der Cucurbitaceae gehörenden Pflanze han-delt.

**11.** Pflanze, die zur Familie der Cucurbitaceae gehört und einen rekombinan-ten Vektor gemäß Anspruch 8 umfasst.

**12.** Transformierte Pflanze, die eine Vielzahl von Wirtszellen gemäß Anspruch 9 oder 10 umfasst.

**13.** Nucleinsäure, die als Sonde oder Primer verwendbar ist und ausgewählt ist aus:

- einer Nucleinsäure, die wenigstens 60 aufeinanderfolgende Nucleotide einer Nucleinsäure der Sequenz SEQ ID Nr. 1 oder SEQ ID Nr. 2 oder der dazu komplementären Sequenz umfasst;
- einer Nucleinsäure, die wenigstens 15 aufeinanderfolgende Nucleotide einer der Sequenzen 5907-6086, 6181-6467 und 7046-7915 der Sequenz SEQ ID Nr. 1 umfasst; und
- einer Nucleinsäure, die wenigstens 15 aufeinanderfolgende Nucleotide einer der Sequenzen 3615-3830, 3924-4209 und 4790-5659 der Sequenz SEQ ID Nr. 2 umfasst.

**14.** Verfahren zum Nachweis der Anwesenheit eines Allels (A) oder (a)

- wobei das dominante Allel A aus einer Nucleinsäure (NA) besteht, umfassend

  (i) ein regulatorisches Polynucleotid (PA), das in einer Pflanze der Familie der Cucurbitaceae funktionsfähig ist; und
  (ii) eine Nucleinsäure, deren Expression durch das regulatorische Polynucleotid (PA) reguliert wird, wobei die Nucleinsäure für das Protein Aminocyclopropancarboxylat-Synthase (ACCS)der Sequenz SEQ ID Nr. 3 codiert; und

- wobei sich das rezessive Allel (a) vom dominanten Allel (A) unterschei-det durch:

  (i) in Nucleinsäure (NA), die in der Pflanze nicht vorhanden ist; oder
  (ii) ein regulatorisches Polynucleotid (Pa), das in einer Pflanze der Familie der Cucurbitaceae nicht funktionsfähig ist; oder
  (iii) eine Nucleinsäure, die für ein inaktives ACCS-Protein codiert; oder
  (iv) ein regulatorisches Polynucleotid (Pa), das in einer Pflanze der Familie der Cucurbitaceae nicht funktionsfähig ist, und eine Nucleinsaure, die für ein inaktives ACCS-Protein codiert; und

wobei das Verfahren die folgenden Schritte umfasst:

1) In-Kontakt-Bringen einer Nucleotidsonde oder einer Vielzahl von Nucleotidsonden gemäß Anspruch 13 mit einer zu testenden Probe;
2) Nachweisen des Komplexes, der gegebenenfalls zwischen der oder den Sonden und der in der Probe vorhandenen Nucleinsäure gebildet wird.

**15.** Verfahren gemäß Anspruch 14, **dadurch gekennzeichnet, dass** es das Bestimmen der Anwesenheit einer Sequenz AGCT, die dem Allel (A) entspricht, oder der Anwesenheit einer Sequenz AGTT, die der Anwesenheit des Allels (a) entspricht, umfasst.

**16.** Verfahren zum Nachweis der Anwesenheit eines Allels (A) oder (a), wobei das Verfahren die folgenden Schritte umfasst:

1) Amplifizieren der DNA einer zu analysierenden Probe durch PCR unter Verwendung eines Primerpaars, die den Bereich des Einzelnukleotidpolymorphismus (SNP), der sich vom Nucleotid der Position 6074 bis zum Nucleotid der Position 6077 der Sequenz SEQ ID Nr. 1 erstreckt, einfassen;
2) Verdau des erhaltenen Produkts durch das Restriktionsenzym AluI; und
3) Nachweisen der erhaltenen Restriktionsfragmente.

**17.** Verfahren zur Selektion des Blütentyps einer zur Familie der Cucurbitaceae gehörenden Pflanze, **dadurch gekennzeichnet, dass** einen Schritt bestehend aus

a) Nachweisen der Anwesenheit der Allele (A) und (a) in einer interessierenden Pflanze, die zur Familie der Cucurbitaceae gehört, gemäß einem Nachweisverfahren, wie es in einem der Ansprüche 14 bis 16 definiert ist; und
b) positiv Selektieren der Pflanze, die das Allel (A) oder das Allel (a) in ihrem Genom aufweist;

umfasst.

**18.** Verfahren zur Gewinnung einer transformierten Pflanze, die zur Familie der Cucurbitaceae gehört und weibliche Blüten trägt, **dadurch gekennzeichnet, dass** es die folgenden Schritte umfasst:

a) Transformation wenigstens einer Pflanzenzelle einer interessierenden Pflanze, die keine Nucleinsäure gemäß einem der Ansprüche 1, 2, 4 und 5 in ihrem Genom umfasst, mit einem rekombinanten Vektor, der eine solche Nucleinsäure umfasst;
b) Selektieren der in Schrift a) erhaltenen transformierten Zellen, die die Nucleinsäure in ihn Genom integriert haben;
c) Regeneration einer transformierten Pflanze ausgehend von den in Schritt b) erhaltenen transformierten Zellen.

**Claims**

**1.** A nucleic acid comprising, from the 5' end to the 3' end, at least:

(i) a hazing at least 95% identity with the polynucleotide extending from nucleotide 5907 to nucleotide 6086 of the sequence SEQ ID No 1,
(ii) a sequence having at least 95% identity with the polynucleotide extending from nucleotide 6181 to nucleotide 6467 of the sequence SEQ ID No 1, and
(iii) a sequence having at least 95% identity with the polynucleotide extending from nucleotide 7046 to nucleotide 7915 of the sequence SEQ ID No 1.

**2.** A nucleic acid, such as defined in claim 1, of the sequence SEQ ID No 1.

**3.** A nucleic acid in the form of the allele (a), such as defined in claim 1, of the SEQ ID No 2.

**4.** A nucleic acid encoding a polypeptide having at least 95% amino acid identity with the sequence SEQ ID No 3.

**5.** A nucleic acid comprising a nucleotide sequence extending from nucleotide 1 to nucleotide 5906 of the sequence SEQ ID No 1.

**6.** A nucleic acid comprising, from the 5' end to the 3' end, at least:

(i) a sequence extending from nucleotide 1 to nucleotide 5906 of the sequence SEQ ID No 1,
(ii) a sequence extending from nucleotide 5907 to nucleotide 6086 of the sequence SEQ ID No 1,
(iii) a sequence extending from nucleotide 6181 to nucleotide 6467 of the sequence SEQ ID No 1, and
(iv) a sequence from nucleotide 7046 to nucleotide 7915 of the sequence SEQ ID No 1, and carrying as compared to the sequence SEQ ID No 1, a mutation which will (i) induce a reduced expression of the ACCS protein of sequence SEQ ID No 3 or (ii) induce the synthesis of a non-active ACCS protein.

**7.** A nucleic acid comprising a sequence extending from nucleotide 1 to nucleotide 3650 of the sequence SEQ ID No 2.

**8.** A recombinant vector comprising a nucleic acid as defined in anyone of claims 1 to 7.

**9.** A host cell comprising a recombinant vector according to claim 8.

**10.** A host cell according to claim 9, **characterized in that** it is a plant cell belonging to the cucurbitaceae family.

**11.** A plant belonging to the cucurbitaceae family comprising a recombinant vector according to claim 8.

**12.** A transformed plant comprising a plurality of host cells according to claim 9 or 10.

**13.** A nucleic acid, to be used as a probe or a primer, chosen among:

- a nucleic acid comprising at least 60 consecutive nucleic acids of a nucleic acid sequence of the sequence SEQ ID No 1, or SEQ ID No 2 or of its complementary sequence,
- a nucleic acid comprising at least 15 consecutive nucleic acids of one of the sequences 5907-6086, 6181-6467 et 7046-7915 of the sequence SEQ ID No 1, and

- a nucleic acid comprising at least 15 consecutive nucleic acids of one of the sequences 3615-3830, 3924-4209 et 4790-5659 of the sequence SEQ ID No 2.

**14.** A method for detecting the presence of an allele (A) or (a),

- said dominant allele (A) consisting in a nucleic acid (NA) comprising:

(i) a regulatory polynucleotide (PA) that is functional in a plant belonging to the cucurbitaceae family, and
(ii) a nucleic acid which expression is regulated by the regulatory polynucleotide (PA), the said nucleic acid encoding the aminocyclopropane carboxylate synthase (ACCS) protein of sequence SEQ ID No. 3, and

- the recessive allele (a) that differs from the dominant allele (A) through:

(i) a nucleic acid (NA) that is not present in the plant, or
(ii) a regulatory polynucleotide (Pa) that is not functional in a plant belonging to the cucurbitaceae family, or
(iii) a nucleic acid encoding a non active ACCS protein, or
(iv) a regulatory polynucleotide (Pa) that is not functional in a plant belonging to the cucurbitaceae family, and a nucleic acid encoding a non active ACCS protein, and

the said method comprising the following steps of:

1) bringing into contact a nucleotide probe or a plurality of nucleotide probes according to claim 13 with a test sample;
2) detecting the complex possibly formed between the probe(s) and the nucleic acid present in the sample.

**15.** A method according to claim 14, **characterized in that** it comprises the fact of determining the presence of an AGCT sequence corresponding to the allele (A), or the presence of an AGTT sequence corresponding to the allele (a).

**16.** A method for detecting the presence of an allele (A) or (a), said method comprising the following steps of:

1) PCR amplifying the DNA in a sample to be analyzed by using a pair of primers flanking the single nucleotide polymorphism (SNP) region extending from the nucleotide in position 6074 to the nucleotide in position 6077 of the sequence SEQ ID No 1,
2) digesting by the restriction enzyme Alu I the resulting product, and
3) detecting the resulting restriction fragments.

**17.** A method for selecting the floral type of a plant belonging to the cucurbitaceae family, **characterized in that** it comprises a step consisting in:

a) detecting the presence of the alleles (A) and (a), in a plant of interest belonging to the cucurbitaceae family according to a detection method such as defined in anyone of claims 14 to 16, and
b) positively selecting the plant which comprises the allela (A) or the allele (a) within its genome.

**18.** A method for producing a transformed plant, belonging to the cucurbitaceae family, comprising female flowers, **characterized in that** it comprises the following steps of:

a) transforming at least one plant cell of a plant of interest that does not comprise a nucleic acid according to one of claims 1, 2, 4 and 5, within its genome, with a recombinant vector comprising such a nucleic acid,
b) selecting the transformed cells obtained in step a) that have integrated in their genome the said nucleic acid;
c) regenerating a transformed plant from the transformed cells obtained in step b).

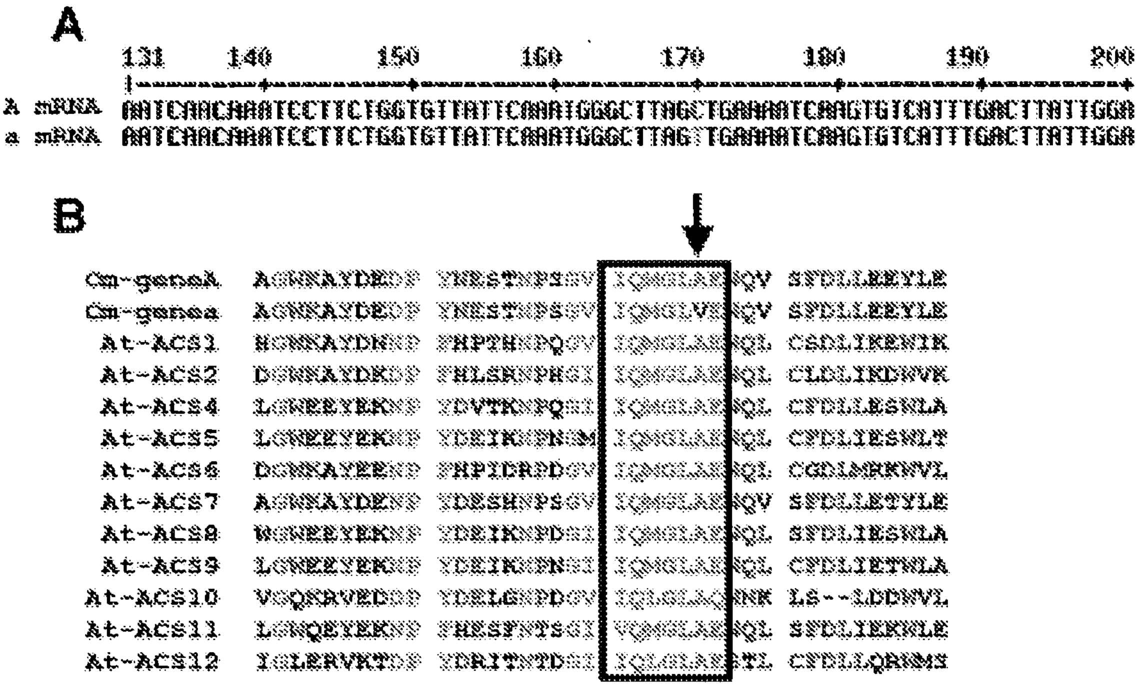
A
131
140
150
160
170
180
190
200
A mRNA
a mRNA
B
Cm-geneA
Cm-genea
At-ACS1
At-ACS2
At-ACS4
At-ACS5
At-ACS6
At-ACS7
At-ACS8
At-ACS9
At-ACS10
At-ACS11
At-ACS12

FIGURE 1

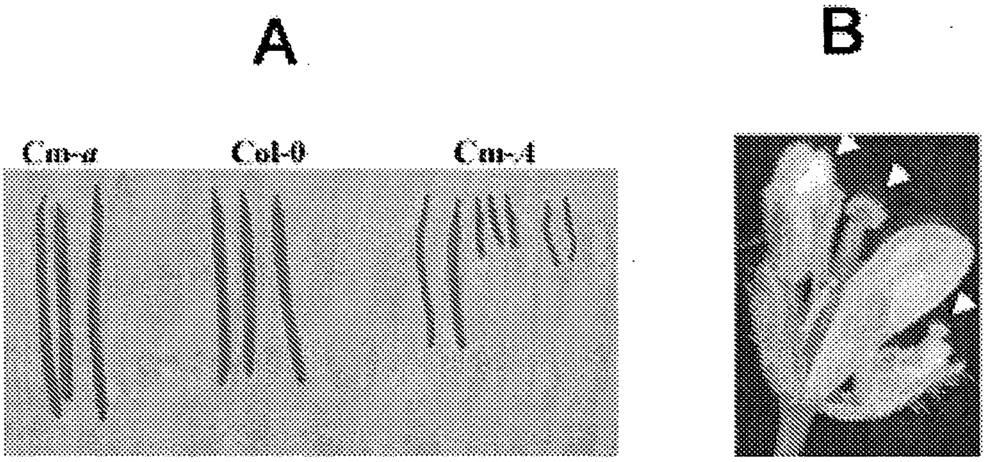


Figure 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9002172 A
- US 4946778 A
- WO 9504064 A
- EP 0707592 A
- FR 7810975
- EP 0225807 A, Chiron
- EP 672752 A, Ishida


### Littérature non-brevet citée dans la description


- **PAPADOPOULOU et al.** *Sexual Plant reproduction,* 2005, vol. 18 (3), 131-142
- **SILBERSTEIN et al.** *Genome,* 2003, vol. 46 (5), 761-73
- **NOGUERA et al.** *Theor Appl Genet.,* 2005, vol. 110 (4), 714-20
- **AN et al.** *Plant Physiol.,* 1986, vol. 81, 86-91
- **AOYAMA T et al.** *The Plant Journal,* 1997, vol. 11 (3), 605-612
- **AUSUBEL et al.** *Current protocols in molecular biology,* 1997
- **BAI et al.** *Planta,* 2004, vol. 220, 230-240
- **BEAUCAGE et al.** *Tetrahedron Lett.,* 1981, vol. 22, 1859-1862
- **BEVAN et al.** *Nucleic Acids Research,* vol. 12, 8711-8721
- **BROWN et al.** *Methods Enzymol.,* 1979, vol. 68, 109-151
- **CAUSSE et al.** *Molecular Breeding,* 1995, vol. 1, 259-272
- **CAPITANI et al.** *J. Biol. Chem,* 2002, vol. 51, 49735-49742
- **CHRISTENSEN et al.** *Transgenic. Res.,* 1996, vol. 5, 213
- **FINER et al.** *Plant Cell Report,* 1992, vol. 11, 323-328
- **FROMM M. et al.** *Biotechnology,* 1990, vol. 8, 833-839
- Nucleic Acid Hybridization. 1984
- **GORLACH J ; VOLRATH S ; KNAUF-BEITER G ; HENGY G ; BECKHOVE U ; KOGEL KH ; OOSTENDORP M ; STAUB T ; WARD E ; KESSMANN H.** Benzothiadiazole, a novel class of inducers of systemic acquired résistance, activates gene expression and disease résistance in wheat. *Plant Cell,* 1996, vol. 8, 629-43
- DNA Cloning: A Practical Approach. Oligonucleotide Synthesis. MRL Press, Ltd, 1985, vol. I, II
- **GUERCHE et al.** *Mol Gen. Genet,* 1987, vol. 206, 382
- **HAMES ; HIGGINS.** Nucleic Acid Hybridization: a practical approach. IRL Press, 1985
- **HAJDUKIEWICZ, P. ; SVAB. Z. ; MALIGA P.** *Plant Mol. Biol.,* 1994, vol. 25 (6), 989-994
- **HUAI et al.** *J ; Biol. Chem,* 2001, vol. 41, 38210-38216
- **ISHIDA et al.** *Nature biotechnology,* 1996, vol. 14, 745-750
- **JEFFERSON.** *Plant Molecular Biology Reporter,* 1987, vol. 5, 387-405
- **KAY et al.** *Science,* 1987, vol. 236, 4805
- **KAHANA, A. ; SILBERSTEIN, L. ; KESSLER, N. ; GOLDSTEIN, R. S. ; PERL-TREVES, R.** expression of ACC oxidase genes differs among sex genotypes and sex phases in cucumber. *Plant Mol Biol.,* Novembre 2000, vol. 41 (4), 517-528
- **KAMACHI, S. ; SEKIMOTO, H. ; KONDO, N. ; SAKAI, S.** Cloning of a cDNA for a 1-aminocyclopropane-1-carboxylate synthase that is expressed durig development of female flowers at the apices of Cucumis sativus L. *The Plant Cell Physiol.,* 1997, vol. 38, 1197-206
- **KOHLER G ; MILSTEIN C.** *Nature,* 1975, vol. 256, 495
- **KOOTER, JM. ; MATZKE, MA. ; MEYER, P.** Listening to the silent genes: transgene silencing, gene regulation and pathogen control. *Trends Plant Sci.,* 1999, vol. 4, 430-437
- **KOZBOR et al.** *Hybridoma,* 1983, vol. 2 (1), 7-16
- **LEGER OJ et al.** *Hum Antibodies,* 1997, vol. 8 (1), 3-16
- **MARTINEAU P et al.** *J. Mol. Biol.,* 1998, vol. 280 (1), 117-127
- **MARTINEZ, A. ; SPARKS, C. ; HART, CA ; TOMPSON, J. ; JEPSON, I.** Ecdysone agonist inducible transcription in transgenic tobacco plants. *Plant J.,* 1999, vol. 19, 97-106
- **MCNELLIS T W.** *The Plant Journal,* 1998, vol. 14 (2), 247-257

- **MOLINA A ; HUNT MD ; RYALS JA.** Impaired fungicide activity in plants blocked in disease résistance signal transduction. *Plant Cell,* 1998, vol. 10, 1903-14
- **NARANG et al.** *Methods Enzymol.,* 1979, vol. 68, 90-98
- **NEUHAUS et al.** *Theor. Appl. Genet.,* 1987, vol. 75 (1), 30-36
- **NG PC ; HENIKOFF S.** *Genome Res.,* 2001, vol. 11 (5), 863-874
- **REINMANN KA et al.** *Aids Res. Hum retroviruses,* 1997, vol. 13 (11), 933-943
- **RIDDER R. et al.** *Biotechnology (NY,* 1995, vol. 13 (3), 255-260
- Induction par le nitrate d'argent de fleurs staminées chez des plantes gynoïques de melon (Cucumis melo L. **RISSER, G. ; RODE, J.C.** Annales de l'Amélioration des Plantes. 1979, vol. 29, 349-352
- **RUDICH, J. ; HALEVY, A.H. ; KEDAR, N.** Increase of femaleness of three cucurbits by treatment with Ethrel, an ethylene-releasing compound. *Planta,* 1969, vol. 86, 69-76
- **SAMBROOK et al.** Molecular Cloning A-laboratory manual. 2001
- **SANCHEZ PESCADOR.** *J. Clin. Microbiol.,* 1988, vol. 26 (10), 1934-1938
- **URDEA et al.** *Nucleic Acids Research,* 1988, vol. 11, 4937-4957
- **WASSENEGGER, M. ; PÉLISSIER, T.** A model for RNA-mediated gene silencing in higher plants. *Plant Mol. Biol.,* 1998, vol. 37, 349-362
- **WATSON et al.** ADN recombinant. 1994, 273-292
- **YANG F. ; MOSS LG ; PHILIPS GN JR.** *Nat. Biotechnol.,* Octobre 1996, vol. 14 (10), 1246-51
- **YANG TT ; CHENG L. ; KAIN SR.** *Nucleic acids Res.,* 15 Novembre 1996, vol. 24 (22), 4592-3

SEQUENCE LISTING

<110> INSTITUT NATIONAL DE LA RECHERCHE AGRONOMIQUE INRA

<120> système génétique pour le contrôle du développement du type floral d'une plante dicotylédone, et mise en oeuvre dans des procédés de détection et de sélection.

<130> U144FR

<160> 10

<170> PatentIn version 3.1

<210> 1
<211> 13380
<212> DNA
<213> Cucumis melo

<400> 1

```
tatacacatt ttgtaatgat aaaattagaa gactagttga ttaattgttt aggctttatt     60
atatattcat cataagtctt ttttgtagcc atttaggttt gttttcgtcg aattaatctt    120
ataaacacta tttttattcg taaattccgt tgctttctta tttactttat atcaatgctt    180
taaaacatca atctagtttt taaaaatcaa tatatatgtt tgcacacacc attattatcg    240
tatgttactc tatctattac tgacaaacgt tatgaaattt tattatattt gtaattatct    300
tttgcagttt tgtcatttaa aatcgttttt cttaaaagaa ttatgttgtt attttaaaat    360
tttggctaaa gaatcacgtg gagaattaga tatatcaaac ctttcatctt tgagatgaaa    420
gattacatca attactatta actaagctta ctttgataaa ttaaaatcat attaaaacaa    480
atagtccgta aaagaatata attttgaaaa actaaacagt catcaaacaa cgcgtgttag    540
cttttaatat atattatgat atgttaagtg aaaataaagt tgaagtgtat gaagccaaaa    600
gagaagtcgt tttcacttgt tgagttctaa tttctaggat ggttctatgt aaagtacttc    660
ctcttccaaa attggaatcc aactcactac ttataaacat catttattcg tcatcttaat    720
tacaatacca actcttattt ttgtctcatc tatcatcaca ctcactaatt aacattacca    780
ttatcttata tcattttatg aactcattat ttaacaaata aatcacttaa aagtttaact    840
tcaaaaaaaa aaaggaagaa agaaagaagg tttgaaatta cactatttgc aattaattat    900
gttttatgaa aactttctaa tactttaatt ttatgtcgaa tcgtttgtcg aatcgtttct    960
```

```
cttttatcct actacaaaaa tattataaaa tgattataaa tggctaaaat atatagtatg  1020
tgtatttcat aaatttaaga aaatgttttc gaatacagtc aaatgaacta aaatatttac  1080
aaaaatataa caaaatttca tatgtatatc gaataaaatt taaaaatttg aagactaaat  1140
ttgtaatata attacatatt aaagtaattt ttagatgtgt gggtattata taataataat  1200
gttgggaagg tgagggcatg aggcagctgg agggataagg actagggatt gttttatatc  1260
ctttttcaca tttaattttt gatgctaatt aatttgttgc caatcatttc atcacttttt  1320
tttttttttt tggttctaat ttatttactt tatatggaaa ataaataaaa gaaaaatgaa  1380
agaaagaaaa aagtggtttt caaatcaata gaaaaaacaa acaactccaa ctttaatggc  1440
ttgaaaacaa atgcattcta aaattaaacc ttatgattga tttgattttt attccccttt  1500
tttacacttt tcattttcat cataattata tcttcagtta cctgtccacc aattacacca  1560
tcaaatgtgg attattggga ttcttttttt ttttttaag attatcttac ggctttcatt  1620
tttttcgtat ctttatgacg gtttgataga cgtaaaagtg gttattgtgt tatagagatt  1680
tgtattattt tgatattatg gaaggattcg tttgagtaaa attataaaaa tcagaggggt  1740
gtcgtttaaa aatgtaagta atccaacaca aaaaaataat tatcataaaa tgtaaaaaaa  1800
agggttagat tgaaaacaaa cgaaacaaat gagttttgta ttataaatcg acctaaaatg  1860
ttcaacccaa acatggatta cgatacgacc gattcatctc attacagctc atcgatccta  1920
aaaatgtgaa gagaagtatt ggatataatt attacttaaa aagataatag aaaaaggaaa  1980
tcagcaaaat tagggttctt taataagtta taaaactcat ttatatacaa aattaattac  2040
attacaaaag gtgggaatgt ggatttagac atacaaccta taataattaa ttaaaaacaa  2100
tacacatgtt tcacaatttg agataattaa attttaatcc ccatttgata agtaatgatt  2160
ttatcttata aattagtttg ttaggtctat actttatttg tttatttatt tattcttact  2220
ctttttaat tatattttta cttatatccc aagcttcatt aacgattaat ctaagtttga  2280
aatgattaat tacaaaatag tagtctattt tgatctatca cggactattg tgggtatttt  2340
ataatatttt gttatatttt ataaatattt ttagttcatt ttgctatatt tgaaaataac  2400
catatattat ttatatttta tttttctaga aaatattttc cataaactca agttctatat  2460
ttaaaatata tattcaaaag tttcctatta caaccctaag ttgaatactt atagaattgt  2520
aataaaataa gataattaac taaataagtc taattaaaca ctaataattt gaattaacaa  2580
cactaaacaa atattgtcaa caaaacttag ttcaattgac atctatatga agaatcgagt  2640
tccaaatctt cacacctgaa cattaacaaa attatatagt aattatctta attaattctc  2700
ctcatcgata aagtgaatat ctaattaaaa atttaaagtc aaaagtgtga atttcttgaa  2760
atatcaaatt aagacaaaat tcaaatcaat ttgaaaacat ataaacaaaa tggtaaatta  2820
gacaaaaaaa aaaaatccta aaaactacat atgaaaaggt tcattaccaa agaagttttt  2880
ccatgaaaaa aaaaaagaaa gaaagagaat aaaatattat atatagttaa taattatgaa  2940
atttttgtat aatcccataa agtttgcaac taaacttaag catatagttt atgacataat  3000
```

taaggtcact aataatagag aacagttaga gcaaaggtca aacatccact ttattcactc 3060
tctctcaatc atacaaagag atttaattga atctactcat tacaaaatcc ccaatcttat 3120
aataatatta atatcattaa tctcttatat atatatataa tatatataca tatattatct 3180
catgcacatg gattttcatg atcttcaaac cccacgtcgt tgattttcca taaaacctat 3240
atattccact aatcatttat attcattttt ttttttgggt ctaattttaa actatatgtt 3300
ttaaaactcc atagtttgat caattcaaaa aaaaaaaaaa aaaaaagtga gttatacaat 3360
ttttaaaatt tttaggacat aatcttgaca agtatcttta tctctcctac atgaaagagg 3420
gagcataaga ttagcttgac attgtctaaa attggaagtg tatatatata tatatatata 3480
tctataaatt tagaaattaa aataatgggg tttttttcatg aaatatatat taatagcttg 3540
attaaggaag gtttagaggg tgattaaagt gcaataatat tgttgattaa ttgtttttttt 3600
tttcttatgt gtatcttagt ttcaaggact catgtttttt ttttcttttt tctttttggt 3660
cccatggaag agaacttttt ttcaattata ggatttgggt ttttagtttt tgggaattat 3720
tgaaaagtta taatttctgt tgctaatgat gggaaaatta tgaaaaatta tatatgcatg 3780
ggttggtggg gtcataagat ctcaaagaag cttttatttt gtcattattt ttctttagaa 3840
aatcagaatc ttaatctttt ttttttaca cattggtatt ttggtcccct ctcgtccaac 3900
ccaaatttaa aaaagatcaa aaaagaaaaa aaaaaaaaaa agaaacagaa acctaatctt 3960
aaatcaattt ccactatgca atccttaatt gtcatgttga tataaaaaaa aatagtaacg 4020
aggcaggaga ttgaaccata aaacttagct ttgtggttat taatacactt agatgatgct 4080
aattgagtta aactcttgat tgacaattaa aagaaaagtt aaatcattag ttaaataatt 4140
aaagtttaat gatcataagt taatatttga tgttgggtat taataaagga gatgcatttg 4200
actaaaaaaa tgattaggta gagactaggg taattaataa ccaatattaa taaagtatgg 4260
ttatgggga attcatgaca aactcaagag gggatgttca tttgggtctt aatgaagtgt 4320
aggaattcaa ataatttaaa aagttattaa taattattat gattttatta ttattatttc 4380
atttgggtct acataagtat aaagaattga ttaaagaggt tgattatgca gaaagaaggg 4440
tgattagaga agtacaatta tgaagggatt ttggataaac acataggaac gaatgatttt 4500
cattgggggc cttaacaaat aatattcaat tttaaaaaaa ttgactattt gcaattaggt 4560
cttgatcatg aagatcctcg agataaatta tagtttttc ttttttcttt cgcatatgaa 4620
tttgttcgat ataacgaatt ttccgacata tcttacgtac actgataaga tattgtctgc 4680
ttaggatcta tacttgtgat ttattctatt atctaatcaa tgtgagattt tggtctcatt 4740
cctaacaatt ctctgctaat taattgaaca aaggacgatc actgaggctc cattcaaata 4800
ggaactctta tatctaggtt aattactatg ctacattaga acatatcacc tatctgatag 4860
agttcaaaca catatcacac catgagtact actttttgag gctaagctcc actacatctt 4920
tgtttgacac ccaaatactc tatctacacg actaggttag gagcataaac tttgatacca 4980
tctctttgag acataaactc ccgtcacttt atttttcatt tcattgatct aaaacgtctt 5040

ataccaatag agatagttgt tttcacatat atatacttat attatcctat tgcctagtga 5100
atctttatac aaagcaacat actttaattt tgattaaaca aagagtgatt acacatggag 5160
atcatagcct aattaaataa ttaaagtata attataggga gggattttga gagaaatgta 5220
attcaacaag gattttgcat aagggtctta gataaggaac taaacaacta gaaaaaaaaa 5280
tataatatat atatatataa aagggaaatg aaatcaaaga aagcatccat tctccatata 5340
tataaaaata catatatata tatggggaag agagaagaga ttacaaaact aatttaataa 5400
taaggtagtt gagggggcaa aaagcaaaat acaagagatt ttgatttttg agagaagccc 5460
tttttagcaa aaaaaataaa atagattaat ataacacaca aacacacacc tactcctttt 5520
cttcaaccac cagattcgat tttgcctctc tctctctctc tctctctctc tctctctgtg 5580
gatcttaaac cccaattcaa aatatgatga caaattatta attattattc ctccaaaaat 5640
attttcccta ttaaaaaaaa taccaagaga gagaaaattc aatgattgtt ttttctcttt 5700
tacattattt ttcttttaaa gaaaaaaact tgctataaat agaggtgccc attgtaagag 5760
caacattcaa ttcaacaaat cttcagttca atttctctct ttttggctct caaaaaggga 5820
aagaaaaaaa aatcattatt attattattt cattttcttt ctttccctta aatttgagct 5880
gaaggaaaaa aaaaaaaaaa aaatcaatgg cgattgagat tgatattgag caaaatccaa 5940
cggttgaact ttcgcgaatc ggaacatcag aaacacacgg cgaagattcg ccgtattttg 6000
ctggctggaa agcgtatgat gaagatcctt ataatgaatc aacaaatcct tctggtgtta 6060
ttcaaatggg cttagctgaa aatcaagtaa gaatatataa cttttttttg ttttgttttg 6120
ctttgtaagg agattgggtt ttttttttta attgggtttg tgttggaatt tatgaaacag 6180
gtgtcatttg acttattgga ggaatatttg gaggaaaatt gtgagggaga agggaattat 6240
ttaaattctg ggtttagaga aaatgcttta tttcaagact atcatggtct tttctcattt 6300
agaagtgcaa tgggaagttt tatggaagag attagaggtg gaagagcaaa atttgaccca 6360
aatcgagttg ttttaactgc tggtgccact gctgccaatg agcttctcac tttcattctt 6420
gcaaatcctg gcgatgcttt gcttgtcccc actccttact atcctgggta agtttatcat 6480
cacctctacg ttttcgtatt tcatttcaaa aaccactctt tactgtaatt actataccct 6540
cagacattaa aattttaact ttcaaactat tcttaaagta tgagtttgag ggtatttcat 6600
atggggtttt taaatgtaaa tttatttaca tttttccact acttaagtgt cctatatttc 6660
tactaatttc ttcttgtgtt gtactcatat tttctatcgt ggggtggact acgtattttt 6720
acgagactat tcgtataaca tacgaatgag tgctttttaa accaaattct tcaaaatcca 6780
agtttaattt tggaaactag aaaatgggta gttttttaaa atgttaccaa acgtgatctt 6840
tatccttaca atcaaacatt accaaggata attgcaacta ccgttagact ttatgagtgc 6900
tttttttttcc aactgttcta tatttttaca acattttgag ttgtattcat catttctgtt 6960
aaagatattt atatgtaact aagtattttt ataagacact gttggtataa tttcatgcac 7020
taataatata gtttcttttt ccagatttga cagagatttg agatggagaa caggagtgaa 7080

aattgtacca attcattgtg acagttcaaa caattttcaa ataactccaa aagcattaga 7140
agaagcttat aattcagcaa tggaaatgaa aatcaaagta agaggagttt taatcacaaa 7200
tccatcaaat ccactcggag caacgatcca acgctccaca atcgaagaca ttctagattt 7260
cgttacacgc aaaaacatcc acctcgtatc cgacgaaatc tattccggtt ccgttttctc 7320
ctccgccgag ttcacaagcg tcgctgaggt tttggaatcc cgcagctaca aaaacgccga 7380
acgtgtccac atcgtttaca gcctctccaa agatctcggc cttcccgggt ttagaatcgg 7440
cacgatctac tcatacaacg ataaagtcgt cacaaccgct cgccggatgt ctagctttac 7500
gcttatctct tcacaaacgc aacgattttt agcgtccatg ttgtcgaacc ggaagtttac 7560
ggagaaatat attaaaatga accgggacag gctcaagaaa cggtatgaaa tgattattga 7620
agggctgcga accgccggga ttgaatgttt ggaagggaat gccggtttgt tttgttggat 7680
gaatttgagc ccgttgttga aagataaaaa aaccaaagaa ggtgagattg agatatggaa 7740
gaggattttg aaggaagtga aattgaatat ttcgcccggt tcgtcgtgtc attgctctga 7800
acccggttgg ttcagggttt gttttgctaa tatgagtgaa aagactctgc atgttgccct 7860
tgatagaata cgtcggttca tggaacggat gaagaaggaa aacgaagcta attaaatata 7920
tatctatata taaatatatg aaaagaaaaa aaacatatgt agcttatttt attttatttt 7980
tttttacaat ggttgtgaga aaaaagaaaa aagaaaaaaa aaagaaaaaa aagccattgt 8040
gattcttttg tgtggacact gcccaatatt tgttagaaat ttggggtttt ttgtcttcat 8100
ttatacgtca tattttgatg atttaaactg aggaaaaaga aaaagaaatc cttgttttct 8160
tgcttttagc aaagcaagtt ttatttctca gttttatata tatatatata taaagtttct 8220
atttgtattg tcatttttat gtgatatgga atataattag tataattcgt tcttgcaatt 8280
aattacctcg aaaataaacg aaatacaaga aaaagaaaaa aaaaatctca tggagtattt 8340
tagggacaag tgtcaactca gggagagaga aaaaaatatg gtttaaattt aatagtattg 8400
gttattttca taacatgctc taaaaaggaa tataactaat aatttgactt taattaagaa 8460
aagaaaagct aataatatat aattaaaatc acttttagca acgaataaca ctttgccgac 8520
ttgtgtaatt aaccacctaa ctatccatct gacgtggaat gcaagtaatt aattaattga 8580
tttttcgtt ttcaaatttt ggtcaacttg attcttggta ctaatttaat gtttccatct 8640
gtcagaaagc tacaacgttt ttcctccttc tttcttttt ttaagaatta ttttaaaaag 8700
tcaatacggt gctataatta gatttttatt tttcctcttt tttagtgtat atatatattt 8760
atataagtag agattaggaa ctaattgatt gaaaattaaa tatgctgtga cgctcaaaag 8820
atattaatcc cgcgttggtt ttatgtattt aaaaaatgta tttttcttt tttgatattt 8880
ttaaataata aaatatttaa attatttaca aaatataaca aaatttgctc gtttacattt 8940
ttattttgtg aaggacttat gcgatgtggt tcgatctaga attcttgtat tttcaaaata 9000
gatggagctt ctttttggat gaattctctc taggcttctg aagtcaaaaa ttttcaaccc 9060
aagaaaaaac tagagtttcc ttgtggtatg aggtgtatga aattgactca ttgactcaat 9120

tacatggact tttatcatat ttaactcagc taaattaagt ttattttttg gaattaatct 9180
aagtaaataa tatttaattg aaccaaaata tttaatttga tcagtcataa taaagacatg 9240
tgacatcatt ggaatcagtc aatttgtgtt taaatttaat ttgggataca tgtcaacttt 9300
tagttaatct caaatgcaat ttgtgattag ttacaaaatt tcttattcaa catacttcaa 9360
atctaaattt ggtaaattat gtttttttta aagaaattag atcaacacaa aaatataata 9420
tgttttgtga aaatgaaaat tttggtttaa tgggaggaga caaatttgaa cgacaaattt 9480
tcttagtaac ttacgatatg atcactaact aatttattat aggttggggg tttgaacctc 9540
tctcaacttt gtgctcatta tataatatat ctttaaaaga ccgaccttgc attaatgttg 9600
ttggttagtc tagtggtaga atcgtcattc tctagctctc tctaaattgt tccagcttca 9660
gtttttatat acttttttat attatttta ctgaactata aaaaattact gtcgacaaaa 9720
tttatgcttt tatcacttaa cataataatt gaactatgtt tgctttgttt tttcttttt 9780
aacgtatact atctcaaagt tttggataat gtacgtgttt gaaattttgt aacaaacaag 9840
gatacataaa tacgtaattg ttgttaatta tttcaaaatg taaatagatg atatgatgta 9900
ggggttgcta atattattgt ctaattattt ttgtaaagaa taaaataaaa taaaaatcca 9960
tatgatgcct aaggacgtgt ttagtattca atatggcgat tattatgtct ttttttcaaa 10020
tggattactt tttggatgat atgatatctt ttattttaat taaaactttt tgatgacttt 10080
taaaatttaa agggcataaa atatagcttt cttttgtaca tatatatggt tgtgcttgga 10140
ttttgtatct gcttgtcttt gtcccgagtt tctcgtcggt ttgaccttcg atctactttt 10200
tttgatatat atgttccaat atcgatttct aagctaacat atgtaaaaga tgattgcact 10260
cgtgtggtgc tgagactatc acacttgata cttgatataa gattgtatta ttcatctgaa 10320
ccaataaaaa ttaagatgga tattactcac tcttctcttt taggatcatc aaacaaggct 10380
ctttttttga caatcacctg aagtcacgag taactctaca gtgttatagt tgtttatagt 10440
atttgagact ggaaatatca aattcagcca attcaattaa attttataat tgatatacat 10500
tcatatgctc gaaaagttgg cttccaaccg tccaacgctc taactttggg acatgtgtta 10560
caaaacacat aggtttaaaa aaatacctaa aaacataaaa aatacaactt tgatcatcct 10620
ttagttcaat tttattccca atttgatact tttgaattcc ctaactagag aattgtaatt 10680
gtgattgaac gtttttatag tcaaaatttg gatttgatag cttataggac cccatgtgcc 10740
aaacaaaata aaaattgcat atatatatat agagagagag ggggagtttt atattttaaa 10800
aaacaaaatt agaaaaaggc aattaatttt gttctttaaa cgacatggtt tctcacgtgt 10860
taaacgtcat tgttatcaac tgcgtcttgt cgctgaccaa ttcattgaca gcacagcaag 10920
caaaaaagaa aaaagaaaaa aaaaagtcaa aaagttaatg tggttaaaga aagcgctttt 10980
tgtgataact caaaaaaaca aaccaaccaa tggaagccct ccaggtcact ctcatgtggc 11040
tcccgtctcc acgtcatcaa tgaacacgga cgtgtttaca gctcaacaaa caacaactat 11100
tcctctgacg tggcaatttc ttattaatga atttccaact ctgccctcct attacatttt 11160

cttaaggtcg gtgaatgccg tacaatggaa gctcctagac atactttcta gttgtgatat 11220
atatatatat atattattca tccatcatat attaaactcc aatgtatgtt tgtggatttt 11280
acaaaagtta atattatttg gtagatgttg agatttcttt tttttccaat gtctagtctt 11340
tttccaagtt tggagaaagt tttttatgat gttgggagtt atttaatttc ctagtggggc 11400
cacgtagtta aataaatata ggttaaattt tacgaaatat ccatatatcg cgtgtgtggt 11460
atgatttcag ttacgtcact attttgaaaa catagttttc gtgttcttat taatgcttat 11520
agttgtaaat aacataatta aaagtatcat ttgttaaaat tgatgtcaca ccgtatgtac 11580
ataatttatt tattgattga tgttataagg ggcgttggag atatcgttgg aaaaaaatga 11640
tttgtaagga tgatcttaat tttctataat tgactcacgt atattatatt gtatacgttt 11700
ttcaaaattt acacaccaat catctcactt tcgttttcat ttttcatttt agtggaaaac 11760
aattcaataa aaaaaaattc tgacaacttt ttaaaattta aggcacagtt gaatcaatcc 11820
aaccgttcaa gatttaaaga agaaaaaaac taatttggtt gctccacttt ttgtttttgt 11880
tcgttttggt ccattaattc taaaaatgtt taatttattt gttatacttt caaatcttca 11940
caactttacc gtattgatcc cttaaaaatg aagtaaaaac aataatgaac gaactaagac 12000
aatcaccatt tgaaagttta aggaccaaat gaaccaaagt taaaagtata gaaacaaaaa 12060
taaacatcgc taaataaacc aaataaaact agaattactt aattgaaaca aaataatatg 12120
aaatggatca aatctttaga ctttagtgta tgggaaagtt ctatgaaaat gaccaccgac 12180
tatcgagaga ccaattttgg ggccaagtca atgattggta atttcaacct acatttatga 12240
tgtatgacaa tgacaatagc ttaggtcact ttgaaaatga ctataagatt ttctagttag 12300
agatatacac ttgatattag acttggtcgt tgtaataaaa actatgtgtc acggatgata 12360
tatgctaagt acatgtttta gtctttaatg tttgcgtata tttctttacg taatttaatc 12420
ttcgttaatt atattttta aactatgttt taatctttta attcttttgt tgtgaaattg 12480
acaaataaag agaaacacga caatgtagat ggtaaacaat gaagtttgtg tagtttattg 12540
acaatatgtt ggcaatgttt acgagtagag agataattgt tcaaattaca gaacaatagg 12600
tgacaatacg tgagtttttg ttttaattta cttttgaaac tttattttga ttttcaaaac 12660
ttaaagaagt tgatactatt attgttttga gctatgaaga tgtgtgatcg aacctttcac 12720
acgtttagaa tgaaagagca tgtcaattaa ttttgagcta aacttgttta aaaaaattga 12780
ccttttgtct ttgttttaag ttttaacaaa ttaatgatgt cattgcgtaa ttttaagtca 12840
gttaggtatg aaaagccacc atcgaagaaa gaaaatttca agaagaaaag caatgtagta 12900
aatcacaaat aattgttttt ctttcccata ggttataact ataaaaaaaa aacttctttt 12960
ttagtataat aacggtaaag aaggatgatc aaaccttcta actcagtcaa ttgcaaatat 13020
gataaattca ctttgacaaa ctaaaataaa tttgaagatt tatgaaacaa aatgtacatt 13080
ttaaaagttt atattttcac acaatgttag cttcctttta aaaaaaaatt aaattttaaa 13140
agttcagaga acaaaacata catttcaact ttgctaactt caatatagaa cttatataaa 13200

atcgtgccac atagggttca aaagaactat aggattttaa aatgaaaaca tatattttaa 13260
ttgagtttta gaactaagtt aatatattta tttataaaga ataacacttc agtaaaatta 13320
agaacaacac agacatagtt caataacata aaactagtac cttttacatc aatattagaa 13380

<210> 2
<211> 11137
<212> DNA
<213> cucumis melo

<400> 2

tagtctattt tgatctatca cgaactattg taggtatttt ataatatttt gttatatttt 60
ataaatattt ttagttcatt ttgctatatt taaaaataac catatattat ttatatttta 120
tttttctaaa aaatattttc cataaactca agttctatat ttaaaatata tattcaaaac 180
tttcttatta caaccctaag ttgaatactt atagaattgt aataaaataa gataattaac 240
taaataagtc taattaaaca ttaataattt gaattaacaa cactaaacaa atattgtcaa 300
caaaacttag ttcaattgac atctatatga agaatcgact tccaaatctt cgcacctgaa 360
cattaacaaa attatatagt aattatctta attaattatc ctcatcgata aagtgaatat 420
ctaattaaaa atttaaagtc aaaagtgtga atttcttgaa atatcaaatt aagacaaaat 480
tcaaatctct ttagagtaaa attatagaat ttgaaaacat ataaacgaaa aatggtaaat 540
tagacaaaaa aaaaatatat tttttctaaa aactacatat gaaaaggttc attaccaaag 600
aagtttttcc atgaaaaaaa aaagaaagaa agaaagaaag agaataaaat attatatata 660
gttaataatt atgaaatttt tgtataatcc cataaagttt gcaactaaac ttaagcatat 720
agtttatgac ataattaagg tcactaataa tagagaacag ttagagcaaa ggtcaaacat 780
ccactttatt cactctctct caatcataca aagagattta attgaatcta ctcattacaa 840
aatccccaat cttataataa tattaatatc attaatctct tatatatata tataatatat 900
atacatatat tatctcatgc acatggattt tcatgatctt caaaccccac gtcgttgatt 960
ttccataaaa cctatatatt ccactaatca tttatattca tttttttttt tgggtctaat 1020
tttaaactat atgttttaaa actccatagt ttgatcaatt caaaaaaaaa aaaaaaaaaa 1080
aaagtgagtt atacaatttt taaaattttt aggacataat cttgacaagt atctttatct 1140
ctcctacatg aaagagggag cataagatta gcttgacatt gtctaaaatt ggaagtgtat 1200
atatatatat atatatatct ataaatttag aaattaaaat aatggggttt tttcatgaaa 1260
tatatattaa tagcttaatt aaggaaggtt tagagggtga ttaaagtgca ataatattgt 1320
tgattaattg ttttttttc ttatgtgtat cttagtttca aggactcatg ttttttttt 1380
cttttttctt tttggtccca tggaagagaa cttttttca attataggat ttgggttttt 1440
agtttttggg aattattgaa aagttataat ttctgttgct aatgatggga aaattatgaa 1500

aaattatata tgcatgggtt ggtggggtca taagatctca aagaagcttt tattttgtca 1560
ttatttttct ttagaaaatc agaatcttaa tctttttttt ttacacattg gtattttggt 1620
cccctctcgt ccaacccaaa tttaaaaaag atcaaaaaag aaaaaagaaa aaaaaaaaaa 1680
gaagaagaag aaacctaatc ttaaattaat ttccactatg caatccttaa ttgtcatgtt 1740
gatataaaaa aaaatagtaa cgaggtagga gattcaacca taaaacttag ctttgtggtt 1800
attaatacac ttagatgatg ctaattgagt taaactcttg attgacaatt aaaagaaaag 1860
ttaaatcatt agttaaataa ttaaagttta atgatcataa gttaatattt gatgttgggt 1920
attaataaag gagatgcatt tgactaaaaa aatgattagg tagagactag ggtaattaat 1980
aacaaatatt aataaagtat ggttatgggg gaattcatga caaactcaag aggggatgtt 2040
catttgggtc ttaatgaagt gtaggaattc aaataattta aaaagttatt aataattatt 2100
atgattttat tattattatt tcatttgggt ctacataagt ataaagaatt gattaaagag 2160
gttgattatg cagaaagaag ggtgattaga gaagtacaat tatgaaggga ttttggataa 2220
acacatagga acgaatgatt ttcattgggg gccttaacaa ataatattca atttttaaaa 2280
aattgactat ttgcaattag gtcttgatca tgaagatcct cgagataaat tatagttttt 2340
tcttgttttc ttcgcatatg aatttgttcg atataacgaa ttttccgaca tatcttacgt 2400
acactgataa gatattgtct gcttaagatc tatacttgtg atttattcta ttatctaatc 2460
aatgtgagat tttggtctca ttcctaacaa ttctctgcta attaattgaa caaaggacga 2520
tcactgaggc tccattcaaa taggaactct tatatctagg ttaattacta tgctacatta 2580
gaacatatca cctatctgat agagttcaaa cacatatcac accatgagta ctacttttcg 2640
aggctaagct ccactacatc tttgtttgac acccaaatac tctatctaca cgactaggtt 2700
aggagcataa actttgatac catctctttg agacataaac tcccgtcact ttatttttca 2760
tttcactgat ctaaaacgtc ttataccaat agagatagtt gttttcacat atatatactt 2820
atattatcct attacctagt gaatctttct gcaaagcaca tactttaatt ttgattaaac 2880
aaaaagtgat tacacatgga tatcatagcc taattaaata attaaagtat aattatgggg 2940
gggggggat tttgagagaa atgtaattca acaaggattt tgcataaggg tcttagataa 3000
ggaactaaac aactagaaaa aaaatataat atatatatat ataaaaaagg gaaatgaaat 3060
caaagaaagc atccattctc catatatata aaaatacata tatatatatg gggaagagag 3120
aagagattac aaaactaatt taataataag gtagttgagg gggcaaaaag caaaatacaa 3180
gagattttga tttttgagag aagcctttt tagcaaaaaa aataaaatag attaatataa 3240
cacacaaaca cacacctact cctttcttc aaccaccaga ttcgattttg cctctctctc 3300
tctctctctc tctctctctc tctctctgga tcttaaaccc caattcaaaa tatgatgaca 3360
aattattaat tattattcct ccaaaaatat tttccctatt aaaaaaaata ccaagagaga 3420
gaaaattcaa tgattgtttt ttctctttta cattattttt tttttaaaga aaaaaacttg 3480
ctataaatag aggtgcccat tgtaagagca acattcaatt caacaaatct tcagttcaat 3540

```
ttctctcttt ttggctctca aaaagggaaa gaaaaaaaaa tcattattat tattatttca 3600
ttttctttct ttcccttaaa tttgagctga aggaaaaaaa aaaaaaatca atggcgattg 3660
agattgatat tgagcaaaat ccaacggttg aactttcgcg aatcggaaca tcagaaacac 3720
acggcgaaga ttcgccgtat tttgctggct ggaaagcgta tgatgaagat ccttataatg 3780
aatcaacaaa tccttctggt gttattcaaa tgggcttagt tgaaaatcaa gtaagaatat 3840
ataacttttt tttgttttgt tttgctttgt aaggagattg gggttttttt ttaattgggt 3900
ttgtgttgga atttatgaaa caggtgtcat ttgacttatt ggaggaatat ttggaggaaa 3960
attgtgaggg agaagggaat tatttaaatt ctgggtttag agaaaatgct ttatttcaag 4020
actatcatgg tcttttctca tttagaagtg caatgggaag ttttatggaa gagattagag 4080
gtggaagagc aaaatttgac ccaaatcgag ttgttttaac tgctggtgcc actgctgcca 4140
atgagcttct cactttcatt cttgcaaatc ctggcgatgc tttgcttgtc cccactcctt 4200
actatcctgg gtaagtttat catcacctct acgttttcgt atttcatttc aaaaaccact 4260
ctttactgta attactatac cctcagacat taaaatttta actttcaaac tattcttaaa 4320
gtatgagttt gagggtattt catatggggt ttttaaatgt aaatttattt acatttttcc 4380
actacttaag tgtcctatat ttctactcat ttcttcttgt gttgtactca tattttctat 4440
cgtggggtgg actacgtatt tttacgagac tattcgtata acatacgaat gagtgctttt 4500
taaaccaaat tcttcaaaat ccaagtttaa ttttggaaac tagaaaatgg gtagtttttt 4560
aaaatgttac caaacgtgat ctttatcctt acaatcaaac attatcaagg ataattgcaa 4620
ctatcattag actttatgag tgcttttttt ttccaactgt tctatatttt tacaacattt 4680
tgagttatat tcatcacttc tgttaaagat atttatatgt aactaagtat ttttataaga 4740
cactgttggt ataatttcat gcactaataa tatagtttct ttttccagat ttgacagaga 4800
tttgagatgg agaacaggag tgaaaattgt accaattcat tgtgacagtt caaacaattt 4860
tcaaataact ccaaaagcat tagaagaagc ttataattca gcaatggaaa tgaaaatcaa 4920
agtaagagga gttttaatca caaatccatc aaatccactc ggagcaacga tccaacgctc 4980
cacaatcgaa gacattctag atttcgttac acgcaaaaac atccacctcg tatccgacga 5040
aatctattcc ggttccgttt tctcctccgc cgagttcaca agcgtcgctg aggttttgga 5100
atcccgcagc tacaaaaacg ccgaacgtgt ccacatcgtt tacagcctct ccaaagatct 5160
cggccttccc gggtttagaa tcggcacgat ctactcatac aacgataaag tcgtcacaac 5220
cgctcgccgg atgtctagct ttacgcttat ctcttcacaa acgcaacgat ttttagcgtc 5280
catgttgtcg aaccggaagt ttacggagaa atatattaaa atgaaccggg acaggctcaa 5340
gaaacggtat gaaatgatta ttgaagggct gcgaaccgct gggattgaat gtttggaagg 5400
gaatgccggt ttgttttgtt ggatgaattt gagcccgttg ttgaaagata aaaaaaccaa 5460
agaaggtgag attgagatat ggaagaggat tttgaaggaa gtgaaattga atatttcgcc 5520
cggttcgtcg tgtcattgct ctgaacccgg ttggttcagg gtttgttttg ctaatatgag 5580
```

tgaaaagact ctgcatgttg cccttgatag aatacgtcgg ttcatggaac ggatgaagaa 5640
ggaaaacgaa gctaattaaa tatatatata tatatatata aatatatgaa aagaaaaaaa 5700
acatatgtag cttattttat tttatttttt ttttacaatg gttgtgagaa aaaagaaaaa 5760
agaaaaaaga aaaaaaaaaa gaaaaaaaag ccattgtgat tcttttgtgt ggacactgcc 5820
caatatttgt tagaaatttg gggtttttg tcttcattta tacgtcatat tttgatgatt 5880
taaactgagg aaaaagaaaa agaaatcctt gttttcttgc ttttagcaaa gcaagtttta 5940
tttctcagtt ttatatatat atataaagtt tctatttgta ttgtcatttt tatgtgatat 6000
ggaatataat tagtataatt cgttcttgca attaattacc tcgaaaataa acgaaataca 6060
agaaaaagaa aaaaaaaatc tcatggagta ttttagggac aagtgtcaac tcagggagag 6120
agaaaaaaat atggtttaaa tttaatagta ttggttattt tcataacatg ctctaaaaag 6180
gaatataact aataatttga ctttaattaa gaaaagaaaa gctaataata tataattaaa 6240
atcactttta gcaacgaata acactttgcc gacttgtgta attaaccacc taactatcca 6300
tctgacgtgg aatgcaagta attaattaat tgatttttc gttttcaaat tttggtcaac 6360
ttgattcttg gtactaattt aatgtttcca tctgtcagaa agctacaacg tttttcctcc 6420
ttctttcttt tttttaagaa ttattttaaa aagtcaatac ggtgctataa ttagattttt 6480
atttttcctc ttttttagtg tatatatata tttatataag tagagattag gaactaattg 6540
attgaaaatt aaatatgctg tgacgctcaa aagatattaa tcccgcgttg gttttatgta 6600
tttaaaaaat gtatttttc tttttgata tttttaaata ataaaatatt taaattattt 6660
acaaaatata acaaaatttg ctcgtttaca tttttatttt gtgaaggact tatgcgatgt 6720
ggttcgatct agaattcttg tattttcaaa atagatggag cttcttttg gatgaattct 6780
ctctaggctt ctgaagtcaa aaattttcaa cccaagaaaa aactagagtt tccttgtggt 6840
atgaggtgta tgaaattgac tcattgactc aattacatgg acttttatca tatttaactc 6900
agctaaatta agtttatttt ttggaattaa tctaagtaaa taatatttaa ttgaaccaaa 6960
atatttaatt tgatcagtca taataaagac atgtgacatc attggaatca gtcaatttgt 7020
gtttaaattt aatttgggat acatgtcaac ttttagttaa tctcaaatgc aatttgtgat 7080
tagttacaaa atttcttatt caacatactt caaatctaaa tttggtaaat tatgtttttt 7140
ttaaagaaat tagatcaaca caaaaatata atatgttttg tgaaaatgaa aattttggtt 7200
taatgggagg agacaaattt gaacgacaaa ttttcttagt aacttacgat atgatcacta 7260
actaatttat tctaggttgg gggtttgaac ctctctcaac tttgtgctca ttatataata 7320
tatctttaaa agaccgacct tgcattaatg ttgttggtta gtctagtggt agaatcgtca 7380
ttctctagct ctctctaaat tgttccagct tcagttttta tatacttttt tatattattt 7440
ttactgaact ataaaaaatt actgtcgaca aaatttatgc ttttatcact taacataata 7500
attgaactat gtttgctttg ttttttcttt tttaacgtat actatctcaa agttttggat 7560
aatgtacgtg tttgaaattt tgtaacaaac aaggatacat aaatacgtaa ttgttgttaa 7620

ttatttcaaa atgtaaatag atgatatgat gtaggggttg ctaatattat tgtctaatta 7680
tttttgtaaa gaataaaata aaataaaaat ccatatgatg cctaaggacg tgtttagtat 7740
tcaatatggc gattattatg tcttttttc aaatggatta ctttttggat gatatgatat 7800
cttttatttt aattaaaact ttttgatggc ttttaaaatt taaagggcat aaaatatagc 7860
tttcttttgt acatatatat ggttgtgctt ggattttgta tctgcctgtc tttgtcccga 7920
gtttctcgtc ggtttgacct ccgatctact tttcttgata tatatgttcc aatatcgatt 7980
tctaagctaa catatgtaag agatgattgc actcgtgtgg tgtcgagact atcacacttg 8040
atacttgata taagattgta ttattcatct gaaccaataa aaattaagat ggatattact 8100
cactcttctc ttttaggatc atcaaacaat gctcttttt tgacaatcac ctgaagtcat 8160
gagtaactct acagtgttat agttgtttat agtatttgag actagaaata acaaattcag 8220
ccaattcaat taaattttat aattgatata cattcatatg ctcgaaaagt tggcttccaa 8280
ccgtccaacg ccctaacttt gggacatgtg ttacaaaaca cataggttta aaaaaatacc 8340
taaaaacata aaaaaaaata caattttgat catcctttag ttcaatttta ttcccaattt 8400
gatacttttg aattccctaa ctagagaatt gtaattgtga ttgaacgttt ttatagtcaa 8460
aatttggatt tgatagctta tagaacccca tgtgccaaac aaaataaaaa ttgcatacat 8520
atatatatat atatatatat atatatatat atatatatat atatatatat atatagaggg 8580
agttttatat tttcaaaaac aaaattagaa aaaggcaatt aattttgttc tttaaacgac 8640
atggtttctc acgtgttaaa cgtcattgtt atcaactgcg tcttgtcgct gaccaattca 8700
ttgacagcac agcaagcaaa aaagaaaaaa aaaaaaaaaa aagtcaaaaa gttaatgtgg 8760
ttaaagaaag cgctttttgt gataactcaa aaaaacaaac caaccaatgg aagccctcca 8820
ggtcactctc atgtggctcc cgtctccacg tcatcaatga acacggacgt gtttacagct 8880
caacaaacaa caaccattcc tctgacgtgg caatttctta ttaatgaatt tccaactctg 8940
ccctcctatt acattttctt aaggtcggtg aatgccgtac aatggaagct cctagacata 9000
ctttctagtt gtgatatata tatatatata tatatattat tcatccatca tatattaaac 9060
tccaatgtat gtttgtggat tttacaaaag ttaatattat ttggtagatg ttgagatttc 9120
ttttttttcc aatgtctagt ctttttccaa gtttggagaa agttttttat gatgttggga 9180
gttatttaat ttcctagtgg ggccacgtag ttaaataaat ataggttaaa ttttacgaaa 9240
tatccatata tcgcgtgtgt ggtatgattt cagttacgtc actattttga aaacatagtt 9300
ttcgtgttct tattaatgct tatagttgta aataacataa ttaaaagtat catttgttaa 9360
aattgatgtc acaccgtatg tacataattt atttattgat tgatgttata aggggcgttg 9420
gagatatcgt tggaaaaaaa tgatttgtaa ggatgatctt aattttctat aattgactca 9480
cgtatattat attgtatacg tttttcaaaa tttacacacc aatcatctca ctttcgtttt 9540
catttttcat tttagtggaa aacaattcaa aaaaaaaaaa aattctgaca actttttaaa 9600
atttaaggca cagttgaatc aatccaaccg ttcaagattt aaagaagaaa aaaactaatt 9660

tggttgctcc actttttgtt tttgttcgtt ttggtccatt aattctaaaa atgtttaatt 9720
tatttgttat actttcaaat cttcacaact ttaccgtatt gatcccttaa aaatgaagta 9780
agtcaatcac catttgaaag tttaaggacc aaatgaacca aagttaaaag tataaaaaca 9840
aaaataaaca tcgctaaata aaccaaataa aactagaatt acttaattga aacaaaataa 9900
tatgaaatgg atcaaatctt tagactttag tgtatgggaa agttctatga aaatgaccac 9960
cgactatcga gagaccaatt ttggggccaa gtcaatgatt ggtaatttca acctacattt 10020
atgatgtatg acaatgacaa tagcttaggt cactttgaaa atgactataa gattttctag 10080
ttagagatat acacttgata ttagacttgg tcgttgtaat aaaaactatg tgtcacggat 10140
gatatatgct aagtacatgt tttagtcttt aatgtttgcg tatatttctt tacgtaattt 10200
aatcttcgtt aattatattt tttaaactat gttttaatct tttaattctt ttgttgtgaa 10260
attgatacaa ataaagaggt ttctctttat tgacaatatg ttgcgtagtt tattgacaat 10320
atgttggcaa tgtttacgag tagagagata attgttcaaa ttacagaaca ataggtgaca 10380
atacgtgagt ttttgtttta atttagtttt gaaactttat tttgattttc aaaacttaaa 10440
gaagttgata ctattattgt tttgagctat gaagatgtgt gatcgaacct ttcacacgtt 10500
tagaatgaaa gagcatgtca attaattttg agctaaactt gtttaaaaaa attgaccttt 10560
tgtctttgtt ttaagtttta acaaattaat gatgtcattg cgtaatttta agtcagttag 10620
gtatgaaaag ccaccatcga agaaagaaaa tttcaagaag aaaagcaatg tagtaaatca 10680
caaataattg tttttctttc ccataggtta taactataaa aaaaaacttc tttttagta 10740
taataacggt aaagaaggat gatcaaacct tctaactcag tcaattgcaa atatgataaa 10800
ttcactttga caaactaaaa taaatttgaa gatttatgaa acaaaatgta cattttaaaa 10860
gtttatattt tcacacaatg ttagcttcct tttaaaaaaa atttaaattt taaaagttca 10920
gagaacaaaa catacatttc aactttgcta acttcaatat agaacttata taaaatcgtg 10980
ccacataggg ttcaaaagaa ctataggatt ttaaaatgaa aacatatatt ttaattgagt 11040
tttagaacta agttaatata tttatttata aagaataaca cttcagtaaa attaagaaca 11100
acacagacat agttcaataa cataaaacta gaggatc 11137

<210> 3
<211> 445
<212> PRT
<213> Cucumis melo

<400> 3

Met Ala Ile Glu Ile Asp Ile Glu Gln Asn Pro Thr Val Glu Leu Ser
1 5 10 15

Arg Ile Gly Thr Ser Glu Thr His Gly Glu Asp Ser Pro Tyr Phe Ala
20 25 30

Gly Trp Lys Ala Tyr Asp Glu Asp Pro Tyr Asn Glu Ser Thr Asn Pro
35 40 45

Ser Gly Val Ile Gln Met Gly Leu Ala Glu Asn Gln Val Ser Phe Asp
50 55 60

Leu Leu Glu Glu Tyr Leu Glu Glu Asn Cys Glu Gly Glu Gly Asn Tyr
65 70 75 80

Leu Asn Ser Gly Phe Arg Glu Asn Ala Leu Phe Gln Asp Tyr His Gly
85 90 95

Leu Phe Ser Phe Arg Ser Ala Met Gly Ser Phe Met Glu Glu Ile Arg
100 105 110

Gly Gly Arg Ala Lys Phe Asp Pro Asn Arg Val Val Leu Thr Ala Gly
115 120 125

Ala Thr Ala Ala Asn Glu Leu Leu Thr Phe Ile Leu Ala Asn Pro Gly
130 135 140

Asp Ala Leu Leu Val Pro Thr Pro Tyr Tyr Pro Gly Phe Asp Arg Asp
145 150 155 160

Leu Arg Trp Arg Thr Gly Val Lys Ile Val Pro Ile His Cys Asp Ser
165 170 175

Ser Asn Asn Phe Gln Ile Thr Pro Lys Ala Leu Glu Glu Ala Tyr Asn
180 185 190

Ser Ala Met Glu Met Lys Ile Lys Val Arg Gly Val Leu Ile Thr Asn
195 200 205

Pro Ser Asn Pro Leu Gly Ala Thr Ile Gln Arg Ser Thr Ile Glu Asp
210 215 220

Ile Leu Asp Phe Val Thr Arg Lys Asn Ile His Leu Val Ser Asp Glu
225 230 235 240

Ile Tyr Ser Gly Ser Val Phe Ser Ser Ala Glu Phe Thr Ser Val Ala
245 250 255

Glu Val Leu Glu Ser Arg Ser Tyr Lys Asn Ala Glu Arg Val His Ile
260 265 270

Val Tyr Ser Leu Ser Lys Asp Leu Gly Leu Pro Gly Phe Arg Ile Gly
275 280 285

Thr Ile Tyr Ser Tyr Asn Asp Lys Val Val Thr Thr Ala Arg Arg Met
290 295 300

Ser Ser Phe Thr Leu Ile Ser Ser Gln Thr Gln Arg Phe Leu Ala Ser
305 310 315 320

Met Leu Ser Asn Arg Lys Phe Thr Glu Lys Tyr Ile Lys Met Asn Arg
325 330 335

Asp Arg Leu Lys Lys Arg Tyr Glu Met Ile Ile Glu Gly Leu Arg Thr
340 345 350

Ala Gly Ile Glu Cys Leu Glu Gly Asn Ala Gly Leu Phe Cys Trp Met
355 360 365

Asn Leu Ser Pro Leu Leu Lys Asp Lys Lys Thr Lys Glu Gly Glu Ile
370 375 380

Glu Ile Trp Lys Arg Ile Leu Lys Glu Val Lys Leu Asn Ile Ser Pro
385 390 395 400

Gly Ser Ser Cys His Cys Ser Glu Pro Gly Trp Phe Arg Val Cys Phe
405 410 415

Ala Asn Met Ser Glu Lys Thr Leu His Val Ala Leu Asp Arg Ile Arg
420 425 430

Arg Phe Met Glu Arg Met Lys Lys Glu Asn Glu Ala Asn
435 440 445

<210> 4
<211> 18
<212> DNA
<213> Cucumis melo

<400> 4
gactgcgtac atgcagca 18

<210> 5
<211> 27
<212> DNA
<213> Cucumis melo

<400> 5
cgtattttgc tggctggaaa gcgtatg 27

<210> 6
<211> 27
<212> DIA
<213> cucumis melo

<400> 6
cgatagaaaa tatgagtaca acacaag 27

<210> 7
<211> 16177
<212> DNA

<213> séquence artificielle

<400> 7

```
ggaaacagct atgaccatga ttacgccaag ctcggaatta accctcacta aagggaacaa     60
aagctggagc tccaccgcgg tggccggggc cgctctagcc acagacagct ccgtagccct    120
cgttctcctt ggagttcttc gggaaatgga tctttcgatt cccgatgatg tctctcttat    180
ctgctttgac gacgccgact ggacatccgc tataacgccg ccattgaccg tgatttcgca    240
acctgtcagg gatctcgcga cggctgccac agaagacctg atcgcccgct taaagggcga    300
gacttcagcc ccacccaagg aaactcttct cccggcggtt ctcatagagc gcggttccgt    360
aagcggttct tcgcaaggtc ggggttgcat accgaactcg cgaaacgtcg gcgactgagc    420
tcccgaggcg cgttgacaag atgccacgaa gggaatggaa gacagccgat attgcaattg    480
tcttcgtgga ctgctttcgg gacgtaaggc gcaagccatc atcaccgccg tcctaaacaa    540
acatacctcc acacaaattt atctacctga ccacaagata tatcctgtca cacgatttat    600
taaacgctgc acttggctag aactagtgga tccgcggccg catgcctgca ggtcgactct    660
agttgattaa ttgtttaggc tttattatat attcatcata agtctttttt gtagccattt    720
aggtttgttt tcgtcgaatt aatcttataa acactatttt tattcgtaaa ttccgttgct    780
ttcttattta ctttatatca atgctttaaa acatcaatct agtttttaaa aatcaatata    840
tatgtttgca cacaccatta ttatcgtatg ttactctatc tattactgac aaacgttatg    900
aaattttatt atatttgtaa ttatcttttg cagttttgtc atttaaaatc gtttttctta    960
aaagaattat gttgttattt taaaattttg gctaaagaat cacgtggaga attagatata   1020
tcaaaccttt catctttgag atgaaagatt acatcaatta ctattaacta agcttacttt   1080
gataaattaa aatcatatta aaacaaatag tccgtaaaag aatataattt tgaaaaacta   1140
aacagtcatc aaacaacgcg tgttagcttt taatatatat tatgatatgt taagtgaaaa   1200
taaagttgaa gtgtatgaag ccaaaagaga agtcgttttc acttgttgag ttctaatttc   1260
taggatggtt ctatgtaaag tacttcctct tccaaaattg gaatccaact cactacttat   1320
```

aaacatcatt tattcgtcat cttaattaca ataccaactc ttatttttgt ctcatctatc 1380
atcacactca ctaattaaca ttaccattat cttatatcat tttatgaact cattatttaa 1440
caaataaatc acttaaaagt ttaacttcaa aaaaaaaaag gaagaaagaa agaaggtttg 1500
aaattacact atttgcaatt aattatgttt tatgaaaact ttctaatact ttaattttat 1560
gtcgaatcgt ttgtcgaatc gtttctcttt tatcctacta caaaaatatt ataaaatgat 1620
tataaatggc taaaatatat agtatgtgta tttcataaat ttaagaaaat gttttcgaat 1680
acagtcaaat gaactaaaat atttacaaaa atataacaaa atttcatatg tatatcgaat 1740
aaaatttaaa aatttgaaga ctaaatttgt aatataatta catattaaag taatttttag 1800
atgtgtgggt attatataat aataatgttg ggaaggtgag ggcatgaggc agctggaggg 1860
ataaggacta gggattgttt tatatccttt ttcacattta atttttgatg ctaattaatt 1920
tgttgccaat catttcatca cttttttttt tttttttggt tctaatttat ttactttata 1980
tggaaaataa ataaaagaaa aatgaaagaa agaaaaaagt ggttttcaaa tcaatagaaa 2040
aaacaaacaa ctccaacttt aatggcttga aaacaaatgc attctaaaat taaaccttat 2100
gattgatttg atttttattc cccttttta cacttttcat tttcatcata attatatctt 2160
cagttacctg tccaccaatt acaccatcaa atgtggatta ttgggattct tttttttttt 2220
tttaagatta tcttacggct ttcatttttt tcgtatcttt atgacggttt gatagacgta 2280
aaagtggtta ttgtgttata gagatttgta ttattttgat attatggaag gattcgtttg 2340
agtaaaatta taaaaatcag aggggtgtcg tttaaaaatg taagtaatcc aacacaaaaa 2400
aataattatc ataaaatgta aaaaaaaggg ttagattgaa aacaaacgaa acaaatgagt 2460
tttgtattat aaatcgacct aaaatgttca acccaaacat ggattacgat acgaccgatt 2520
catctcatta cagctcatcg atcctaaaaa tgtgaagaga agtattggat ataattatta 2580
cttaaaaaga taatagaaaa aggaaatcag caaaattagg gttctttaat aagttataaa 2640
actcatttat atacaaaatt aattacatta caaaaggtgg gaatgtggat ttagacatac 2700
aacctataat aattaattaa aaacaataca catgtttcac aatttgagat aattaaattt 2760
taatccccat ttgataagta atgattttat cttataaatt agtttgttag gtctatactt 2820
tatttgttta tttatttatt cttactcttt tttaattata tttttactta tatcccaagc 2880
ttcattaacg attaatctaa gtttgaaatg attaattaca aaatagtagt ctattttgat 2940
ctatcacgga ctattgtggg tattttataa tattttgtta tattttataa atatttttag 3000
ttcattttgc tatatttgaa aataaccata tattatttat attttatttt tctagaaaat 3060
attttccata aactcaagtt ctatatttaa aatatatatt caaaagtttc ctattacaac 3120
cctaagttga atacttatag aattgtaata aaataagata attaactaaa taagtctaat 3180
taaacactaa taatttgaat taacaacact aaacaaatat tgtcaacaaa acttagttca 3240
attgacatct atatgaagaa tcgagttcca aatcttcaca cctgaacatt aacaaaatta 3300
tatagtaatt atcttaatta attctcctca tcgataaagt gaatatctaa ttaaaaattt 3360

aaagtcaaaa gtgtgaattt cttgaaatat caaattaaga caaaattcaa atcaatttga 3420
aaacatataa acaaaatggt aaattagaca aaaaaaaaaa atcctaaaaa ctacatatga 3480
aaaggttcat taccaaagaa gtttttccat gaaaaaaaaa aagaaagaaa gagaataaaa 3540
tattatatat agttaataat tatgaaattt ttgtataatc ccataaagtt tgcaactaaa 3600
cttaagcata tagtttatga cataattaag gtcactaata atagagaaca gttagagcaa 3660
aggtcaaaca tccactttat tcactctctc tcaatcatac aaagagattt aattgaatct 3720
actcattaca aaatccccaa tcttataata atattaatat cattaatctc ttatatatat 3780
atataatata tatacatata ttatctcatg cacatggatt ttcatgatct tcaaacccca 3840
cgtcgttgat tttccataaa acctatatat tccactaatc atttatattc attttttttt 3900
ttgggtctaa ttttaaacta tatgttttaa aactccatag tttgatcaat tcaaaaaaaa 3960
aaaaaaaaaa aagtgagtta tacaattttt aaaattttta ggacataatc ttgacaagta 4020
tctttatctc tcctacatga aagagggagc ataagattag cttgacattg tctaaaattg 4080
gaagtgtata tatatatata tatatatcta taaatttaga aattaaaata atggggtttt 4140
ttcatgaaat atatattaat agcttgatta aggaaggttt agagggtgat taaagtgcaa 4200
taatattgtt gattaattgt ttttttttc ttatgtgtat cttagtttca aggactcatg 4260
tttttttttt ctttttcttt tttggtccca tggaagagaa cttttttca attataggat 4320
ttgggttttt agtttttggg aattattgaa aagttataat ttctgttgct aatgatggga 4380
aaattatgaa aaattatata tgcatgggtt ggtggggtca taagatctca aagaagcttt 4440
tattttgtca ttattttct ttagaaaatc agaatcttaa tcttttttt tttacacatt 4500
ggtattttgg tcccctctcg tccaacccaa atttaaaaaa gatcaaaaaa gaaaaaaaaa 4560
aaaaaaagaa acagaaacct aatcttaaat caatttccac tatgcaatcc ttaattgtca 4620
tgttgatata aaaaaaaata gtaacgaggc aggagattga accataaaac ttagctttgt 4680
ggttattaat acacttagat gatgctaatt gagttaaact cttgattgac aattaaaaga 4740
aaagttaaat cattagttaa ataattaaag tttaatgatc ataagttaat atttgatgtt 4800
gggtattaat aaaggagatg catttgacta aaaaaatgat taggtagaga ctagggtaat 4860
taataaccaa tattaataaa gtatggttat gggggaattc atgacaaact caagagggga 4920
tgttcatttg ggtcttaatg aagtgtagga attcaaataa tttaaaaagt tattaataat 4980
tattatgatt ttattattat tatttcattt gggtctacat aagtataaag aattgattaa 5040
agaggttgat tatgcagaaa gaagggtgat tagagaagta caattatgaa gggattttgg 5100
ataaacacat aggaacgaat gattttcatt gggggcctta acaaataata ttcaatttta 5160
aaaaaattga ctatttgcaa ttaggtcttg atcatgaaga tcctcgagat aaattatagt 5220
tttttctttt tttcttcgca tatgaatttg ttcgatataa cgaattttcc gacatatctt 5280
acgtacactg ataagatatt gtctgcttag gatctatact tgtgatttat tctattatct 5340
aatcaatgtg agattttggt ctcattccta acaattctct gctaattaat tgaacaaagg 5400

acgatcactg aggctccatt caaataggaa ctcttatatc taggttaatt actatgctac 5460
attagaacat atcacctatc tgatagagtt caaacacata tcacaccatg agtactactt 5520
tttgaggcta agctccacta catctttgtt tgacacccaa atactctatc tacacgacta 5580
ggttaggagc ataaactttg ataccatctc tttgagacat aaactcccgt cactttattt 5640
ttcatttcat tgatctaaaa cgtcttatac caatagagat agttgttttc acatatatat 5700
acttatatta tcctattgcc tagtgaatct ttatacaaag caacatactt taattttgat 5760
taaacaaaga gtgattacac atggagatca tagcctaatt aaataattaa agtataatta 5820
tagggaggga ttttgagaga aatgtaattc aacaaggatt ttgcataagg gtcttagata 5880
aggaactaaa caactagaaa aaaaaatata atatatatat atataaaagg gaaatgaaat 5940
caaagaaagc atccattctc catatatata aaaatacata tatatatatg gggaagagag 6000
aagagattac aaaactaatt taataataag gtagttgagg gggcaaaaag caaaatacaa 6060
gagattttga tttttgagag aagccctttt tagcaaaaaa aataaaatag attaatataa 6120
cacacaaaca cacacctact ccttttcttc aaccaccaga ttcgattttg cctctctctc 6180
tctctctctc tctctctctc tctgtggatc ttaaacccca attcaaaata tgatgacaaa 6240
ttattaatta ttattcctcc aaaaatattt tccctattaa aaaaaatacc aagagagaga 6300
aaattcaatg attgtttttt ctcttttaca ttatttttct tttaaagaaa aaaacttgct 6360
ataaatagag gtgcccattg taagagcaac attcaattca acaaatcttc agttcaattt 6420
ctctcttttt ggctctcaaa aagggaaaga aaaaaaaatc attattatta ttatttcatt 6480
ttctttcttt cccttaaatt tgagctgaag gaaaaaaaaa aaaaaaaaat caatggcgat 6540
tgagattgat attgagcaaa atccaacggt tgaactttcg cgaatcggaa catcagaaac 6600
acacggcgaa gattcgccgt attttgctgg ctggaaagcg tatgatgaag atccttataa 6660
tgaatcaaca aatccttctg gtgttattca aatgggctta gctgaaaatc aagtaagaat 6720
atataacttt tttttgtttt gttttgcttt gtaaggagat tgggtttttt tttttaattg 6780
ggtttgtgtt ggaatttatg aaacaggtgt catttgactt attggaggaa tatttggagg 6840
aaaattgtga gggagaaggg aattatttaa attctgggtt tagagaaaat gctttatttc 6900
aagactatca tggtcttttc tcatttagaa gtgcaatggg aagttttatg gaagagatta 6960
gaggtggaag agcaaaattt gacccaaatc gagttgtttt aactgctggt gccactgctg 7020
ccaatgagct tctcactttc attcttgcaa atcctggcga tgctttgctt gtccccactc 7080
cttactatcc tgggtaagtt tatcatcacc tctacgtttt cgtatttcat ttcaaaaacc 7140
actctttact gtaattacta taccctcaga cattaaaatt ttaactttca aactattctt 7200
aaagtatgag tttgagggta tttcatatgg ggtttttaaa tgtaaattta tttacatttt 7260
tccactactt aagtgtccta tatttctact aatttcttct tgtgttgtac tcatattttc 7320
tatcgtgggg tggactacgt atttttacga gactattcgt ataacatacg aatgagtgct 7380
ttttaaacca aattcttcaa aatccaagtt taattttgga aactagaaaa tgggtagttt 7440

tttaaaatgt taccaaacgt gatctttatc cttacaatca aacattacca aggataattg 7500
caactaccgt tagactttat gagtgctttt ttttccaact gttctatatt tttacaacat 7560
tttgagttgt attcatcatt tctgttaaag atatttatat gtaactaagt atttttataa 7620
gacactgttg gtataatttc atgcactaat aatatagttt ctttttccag atttgacaga 7680
gatttgagat ggagaacagg agtgaaaatt gtaccaattc attgtgacag ttcaaacaat 7740
tttcaaataa ctccaaaagc attagaagaa gcttataatt cagcaatgga aatgaaaatc 7800
aaagtaagag gagttttaat cacaaatcca tcaaatccac tcggagcaac gatccaacgc 7860
tccacaatcg aagacattct agatttcgtt acacgcaaaa acatccacct cgtatccgac 7920
gaaatctatt ccggttccgt tttctcctcc gccgagttca caagcgtcgc tgaggttttg 7980
gaatcccgca gctacaaaaa cgccgaacgt gtccacatcg tttacagcct ctccaaagat 8040
ctcggccttc ccgggtttag aatcggcacg atctactcat acaacgataa agtcgtcaca 8100
accgctcgcc ggatgtctag ctttacgctt atctcttcac aaacgcaacg atttttagcg 8160
tccatgttgt cgaaccggaa gtttacggag aaatatatta aaatgaaccg ggacaggctc 8220
aagaaacggt atgaaatgat tattgaaggg ctgcgaaccg ccgggattga atgtttggaa 8280
gggaatgccg gtttgttttg ttggatgaat ttgagcccgt tgttgaaaga taaaaaaacc 8340
aaagaaggtg agattgagat atggaagagg attttgaagg aagtgaaatt gaatatttcg 8400
cccggttcgt cgtgtcattg ctctgaaccc ggttggttca gggtttgttt tgctaatatg 8460
agtgaaaaga ctctgcatgt tgcccttgat agaatacgtc ggttcatgga acggatgaag 8520
aaggaaaacg aagctaatta aatatatatc tatatataaa tatatgaaaa gaaaaaaaac 8580
atatgtagct tattttattt tattttttt tacaatggtt gtgagaaaaa agaaaaaaga 8640
aaaaaaaaag aaaaaaaagc cattgtgatt cttttgtgtg gacactgccc aatatttgtt 8700
agaaatttgg ggttttttgt cttcatttat acgtcatatt ttgatgattt aaactgagga 8760
aaaagaaaaa gaaatccttg ttttcttgct tttagcaaag caagttttat ttctcagttt 8820
tatatatata tatatataaa gtttctattt gtattgtcat ttttatgtga tatggaatat 8880
aattagtata attcgttctt gcaattaatt acctcgaaaa taaacgaaat acaagaaaaa 8940
gaaaaaaaaa atctcatgga gtattttagg gacaagtgtc aactcaggga gagagaaaaa 9000
aatatggttt aaatttaata gtattggtta ttttcataac atgctctaaa aaggaatata 9060
actaataatt tgactttaat taagaaaaga aaagctaata atatataatt aaaatcactt 9120
ttagcaacga ataacacttt gccgacttgt gtaattaacc acctaactat ccatctgacg 9180
tggaatgcaa gtaattaatt aattgatttt ttcgttttca aattttggtc aacttgattc 9240
ttggtactaa tttaatgttt ccatctgtca gaaagctaca acgtttttcc tccttctttc 9300
tttttttaa gaattatttt aaaaagtcaa tacggtgcta taattagatt tttatttttc 9360
ctctttttta gtgtatatat atatttatat aagtagagat taggaactaa ttgattgaaa 9420
attaaatatg ctgtgacgct caaaagatat taatcccgcg ttggttttat gtatttaaaa 9480

aatgtatttt ttctttttg atatttttaa ataataaaat atttaaatta tttacaaaat 9540
ataacaaaat ttgctcgttt acatttttat tttgtgaagg acttatgcga tgtggttcga 9600
tctagaattc ttgtattttc aaaatagatg gagcttcttt ttggatgaat tctctctagg 9660
cttctgaagt caaaaatttt caacccaaga aaaaactaga gtttccttgt ggtatgaggt 9720
gtatgaaatt gactcattga ctcaattaca tggactttta tcatatttaa ctcagctaaa 9780
ttaagtttat tttttggaat taatctaagt aaataatatt taattgaacc aaaatattta 9840
atttgatcag tcataataaa gacatgtgac atcattggaa tcagtcaatt tgtgtttaaa 9900
tttaatttgg gatacatgtc aacttttagt taatctcaaa tgcaatttgt gattagttac 9960
aaaatttctt attcaacata cttcaaatct aaatttggta aattatgttt tttttaaaga 10020
aattagatca acacaaaaat ataatatgtt ttgtgaaaat gaaaattttg gtttaatggg 10080
aggagacaaa tttgaacgac aaattttctt agtaacttac gatatgatca ctaactaatt 10140
tattataggt tgggggtttg aacctctctc aactttgtgc tcattatata atatatcttt 10200
aaaagaccga ccttgcatta atgttgttgg ttagtctagt ggtagaatcg tcattctcta 10260
gctctctcta aattgttcca gcttcagttt ttatatactt ttttatatta tttttactga 10320
actataaaaa attactgtcg acaaaattta tgcttttatc acttaacata ataattgaac 10380
tatgtttgct ttgtttttc tttttaacg tatactatct caaagttttg gataatgtac 10440
gtgtttgaaa ttttgtaaca aacaaggata cataaatacg taattgttgt taattatttc 10500
aaaatgtaaa tagatgatat gatgtagggg ttgctaatat tattgtctaa ttatttttgt 10560
aaagaataaa ataaaataaa aatccatatg atgcctaagg acgtgtttag tattcaatat 10620
ggcgattatt atgtcttttt ttcaaatgga ttactttttg gatgatatga tatcttttat 10680
tttaattaaa actttttgat gacttttaaa atttaaaggg cataaaatat agctttcttt 10740
tgtacatata tatggttgtg cttggatttt gtatctgctt gtctttgtcc cgagtttctc 10800
gtcggtttga ccttcgatct actttttttg atatatatgt tccaatatcg atttctaagc 10860
taacatatgt aaaagatgat tgcactcgtg tggtgctgag actatcacac ttgatacttg 10920
atataagatt gtattattca tctgaaccaa taaaaattaa gatggatatt actcactctt 10980
ctcttttagg atcatcaaac aaggctcttt ttttgacaat cacctgaagt cacgagtaac 11040
tctacagtgt tatagttgtt tatagtattt gagactggaa atatcaaatt cagccaattc 11100
aattaaattt tataattgat atacattcat atgctcgaaa agttggcttc caaccgtcca 11160
acgctctaac tttgggacat gtgttacaaa acacataggt ttaaaaaaat acctaaaaac 11220
ataaaaaata caactttgat catcctttag ttcaatttta ttcccaattt gatacttttg 11280
aattccctaa ctagagaatt gtaattgtga ttgaacgttt ttatagtcaa aatttggatt 11340
tgatagctta taggacccca tgtgccaaac aaaataaaaa ttgcatatat atatatagag 11400
agagaggggg agttttatat tttaaaaaac aaaattagaa aaaggcaatt aattttgttc 11460
tttaaacgac atggtttctc acgtgttaaa cgtcattgtt atcaactgcg tcttgtcgct 11520

gaccaattca ttgacagcac agcaagcaaa aaagaaaaaa gaaaaaaaaa agtcaaaaag 11580
ttaatgtggt taaagaaagc gctttttgtg ataactcaaa aaaacaaacc aaccaatgga 11640
agccctccag gtcactctca tgtggctccc gtctccacgt catcaatgaa cacggacgtg 11700
tttacagctc aacaaacaac aactattcct ctgacgtggc aatttcttat taatgaattt 11760
ccaactctgc cctcctatta cattttctta aggtcggtga atgccgtaca atggaagctc 11820
ctagacatac tttctagttg tgatatatat atatatatat tattcatcca tcatatatta 11880
aactccaatg tatgtttgtg gattttacaa aagttaatat tatttggtag atgttgagat 11940
ttcttttttt tccaatgtct agtctttttc caagtttgga gaaagttttt tatgatgttg 12000
ggagttattt aatttcctag tggggccacg tagttaaata aatataggtt aaattttacg 12060
aaatatccat atatcgcgtg tgtggtatga tttcagttac gtcactattt tgaaaacata 12120
gttttcgtgt tcttattaat gcttatagtt gtaaataaca taattaaaag tatcatttgt 12180
taaaattgat gtcacaccgt atgtacataa tttatttatt gattgatgtt ataaggggcg 12240
ttggagatat cgttggaaaa aaatgatttg taaggatgat cttaattttc tataattgac 12300
tcacgtatat tatattgtat acgtttttca aaatttacac accaatcatc tcactttcgt 12360
tttcattttt cattttagtg gaaaacaatt caataaaaaa aaattctgac aactttttaa 12420
aatttaaggc acagttgaat caatccaacc gttcaagatt taaagaagaa aaaaactaat 12480
ttggttgctc cactttttgt ttttgttcgt tttggtccat taattctaaa aatgtttaat 12540
ttatttgtta tactttcaaa tcttcacaac tttaccgtat tgatccctta aaaatgaagt 12600
aaaaacaata atgaacgaac taagacaatc accattgaaa agtttaagga ccaaatgaac 12660
caaagttaaa agtatagaaa caaaaataaa catcgctaaa taaaccaaat aaaactagaa 12720
ttacttaatt gaaacaaaat aatatgaaat ggatcaaatc tttagacttt agtgtatggg 12780
aaagttctat gaaaatgacc accgactatc gagagaccaa ttttggggcc aagtcaatga 12840
ttggtaattt caacctacat ttatgatgta tgacaatgac aatagcttag gtcactttga 12900
aaatgactat aagattttct agttagagat atacacttga tattagactt ggtcgttgta 12960
ataaaaacta tgtgtcacgg atgatatatg ctaagtacat gttttagtct ttaatgtttg 13020
cgtatatttc tttacgtaat ttaatcttcg ttaattatat tttttaaact atgttttaat 13080
cttttaattc ttttgttgtg aaattgacaa ataaagagaa acacgacaat gtagatggta 13140
aacaatgaag tttgtgtagt ttattgacaa tatgttggca atgtttacga gtagagagat 13200
aattgttcaa attacagaac aataggtgac aatacgtgag tttttgtttt aatttacttt 13260
tgaaacttta ttttgatttt caaaacttaa agaagttgat actattattg ttttgagcta 13320
tgaagatgtg tgatcgaacc tttcacacgt ttagaatgaa agagcatgtc aattaatttt 13380
gagctaaact tgtttaaaaa aattgacctt ttgtctttgt tttaagtttt aacaaattaa 13440
tgatgtcatt gcgtaatttt aagtcagtta ggtatgaaaa gccaccatcg aagaaagaaa 13500
atttcaagaa gaaaagcaat gtagtaaatc acaaataatt gtttttcttt cccataggtt 13560

ataactataa aaaaaaaact tcttttttag tataataacg gtaaagaagg atgatcaaac 13620
cttctaactc agtcaattgc aaatatgata aattcacttt gacaaactaa aataaatttg 13680
aagatttatg aaacaaaatg tacattttaa aagtttatat tttcacacaa tgttagcttc 13740
cttttaaaaa aaaattaaat tttaaaagtt cagagaacaa aacatacatt tcaactttgc 13800
taacttcaat atagaactta tataaaatcg tgccacatag ggttcaaaag aactatagga 13860
ttttaaaatg aaaacatata ttttaattga gttttagaac taagttaata tatttattta 13920
taaagaataa cacttcagta aaattaagaa caacacagac atagttcaat aacataaaac 13980
tagctagagg atccccgggt accatggata tctagaattc gcggccgcaa gcttgatccc 14040
ccgggctgca ggaattcgat atcaagctag agatctagta acatagatga caccgcgcgc 14100
gataatttat cctagtttgc gcgctatatt ttgttttcta tcgcgtatta aatgtataat 14160
tgcgggactc taatcataaa aacccatctc ataaataacg tcatgcatta catgttaatt 14220
attacatgct taacgtaatt caacagaaat tagatgataa tcatcgcaag accggcaaca 14280
ggattcaatc ttaagaaact ttattgccaa atgtttgaac gatctgcagg tcgatcctag 14340
acgcgtgaga tcagatctcg gtgacgggca ggaccggacg gggcggtacc ggcaggctga 14400
agtccagctg ccagaaaccc acgtcatgcc agttcccgtg cttgaagccg gccgcccgca 14460
gcatgccgcg gggggcatat ccgagcgcct cgtgcatgcg cacgctcggg tcgttgggca 14520
gcccgatgac agcgaccacg ctcttgaagc cctgtgcctc cagggacttc agcaggtggg 14580
tgtagagcgt ggagcccagt cccgtccgct ggtggcgggg ggagacgtac acggtcgact 14640
cggccgtcca gtcgtaggcg ttgcgtgcct tccaggggcc cgcgtaggcg atgccggcga 14700
cctcgccgtc cacctcggcg acgagccagg gatagcgctc ccgcagacgg acgaggtcgt 14760
ccgtccactc ctgcggttcc tgcggctcgg tacggaagtt gaccgtgctt gtctcgatgt 14820
agtggttgac gatggtgcag accgccggca tgtccgcctc ggtggcacgg cggatgtcgg 14880
ccgggcgtcg ttctgggctc atggatcgac ctgcaggtct gtcctctcca aatgaaatga 14940
acttccttat atagaggaag ggtcttgcga aggatagtgg gattgtgcgt catcccttac 15000
gtcagtggag atatcacatc aatccacttg ctttgaagac gtggttggaa cgtcttcttt 15060
ttccacgatg ctcctcgtgg gtgggggtcc atctttggga ccactgtcgg cagaggcatc 15120
ttcaacgatg gcctttcctt tatcgcaatg atggcatttg taggagccac cttccttttc 15180
cactatcttc acaataaagt gacagatagc tgggcaatgg aatccgagga ggtttccgga 15240
tattaccctt tgttgaaaag tctcaattgc cctttggtct tctgagactg tatctttgat 15300
atttttggag tagacaagcg tgtcgtgctc caccatgttg acggatctct agcttatcga 15360
taccgtcggc tattggtaat aggacactgg gattcgtctt ggacaacttt ccttctcatc 15420
taagcgtaga caaccctcaa ctggaaacgg gccggactcc agggcgtgtg ccaggtgccc 15480
acggaatagt tttggccaga cccttgaaaa tccgattcag tacaatcgat tgccctcatt 15540
tttacgttgg catatatcct gccaaacagc caacaacgcg cgtgcggtga ataggaaagc 15600

gtttgagttg cttgctcata tcgtgacggt tgacagcaca ggttgaccgc ttgatgattc 15660
gtacgagccg ccaaacattg gctgtcgtaa tgatatacca tgtcagaaca gcaatccgat 15720
ggggcggaaa gcattatctt aatgcacacg gaaatggcgc gtcggtgggt ggaatacacc 15780
gacatagagg ccgtaagttc tgcatggtca tcgtcggaaa ggtggcagca ggcgcacggc 15840
tgtggcctct tgctctttca gcgtgaaatg cgtgttgaaa gaataatcga agagagcgtc 15900
cgctcgacac cttcaattat gccgatttga tcgatgaact gatcgagctc tgaaatcgaa 15960
ggggcttcga taatcgcaat caaatcaaaa gtgccactca cagaatgaag agcgataacg 16020
gccgtgacct tcccaaggga ggccgtcacc tgtgaaagcg ccttcgtaat ggtgatcaga 16080
atatgggctc gaaccaagct cgagacctcg aggggggccc cggtacccaa ttcgccctat 16140
agtgagtcgt attacaattc actggccgtc gttttac 16177

<210> 8
<211> 445
<212> PRT
<213> cucumis melo

<400> 8

Met Ala Ile Glu Ile Asp Ile Glu Gln Asn Pro Thr Val Glu Leu Ser
1 5 10 15

Arg Ile Gly Thr Ser Glu Thr His Gly Glu Asp Ser Pro Tyr Phe Ala
20 25 30

Gly Trp Lys Ala Tyr Asp Glu Asp Pro Tyr Asn Glu Ser Thr Asn Pro
35 40 45

Ser Gly Val Ile Gln Met Gly Leu Val Glu Asn Gln Val Ser Phe Asp
50 55 60

Leu Leu Glu Glu Tyr Leu Glu Glu Asn Cys Glu Gly Glu Gly Asn Tyr
65 70 75 80

Leu Asn Ser Gly Phe Arg Glu Asn Ala Leu Phe Gln Asp Tyr His Gly
85 90 95

Leu Phe Ser Phe Arg Ser Ala Met Gly Ser Phe Met Glu Glu Ile Arg
100 105 110

Gly Gly Arg Ala Lys Phe Asp Pro Asn Arg Val Val Leu Thr Ala Gly
115 120 125

Ala Thr Ala Ala Asn Glu Leu Leu Thr Phe Ile Leu Ala Asn Pro Gly
130 135 140

Asp Ala Leu Leu Val Pro Thr Pro Tyr Tyr Pro Gly Phe Asp Arg Asp
145 150 155 160

Leu Arg Trp Arg Thr Gly Val Lys Ile Val Pro Ile His Cys Asp Ser
165 170 175

Ser Asn Asn Phe Gln Ile Thr Pro Lys Ala Leu Glu Glu Ala Tyr Asn
180 185 190

Ser Ala Met Glu Met Lys Ile Lys Val Arg Gly Val Leu Ile Thr Asn
195 200 205

Pro Ser Asn Pro Leu Gly Ala Thr Ile Gln Arg Ser Thr Ile Glu Asp
210 215 220

Ile Leu Asp Phe Val Thr Arg Lys Asn Ile His Leu Val Ser Asp Glu
225 230 235 240

Ile Tyr Ser Gly Ser Val Phe Ser Ser Ala Glu Phe Thr Ser Val Ala
245 250 255

Glu Val Leu Glu Ser Arg Ser Tyr Lys Asn Ala Glu Arg Val His Ile
260 265 270

Val Tyr Ser Leu Ser Lys Asp Leu Gly Leu Pro Gly Phe Arg Ile Gly
275 280 285

Thr Ile Tyr Ser Tyr Asn Asp Lys Val Val Thr Thr Ala Arg Arg Met
290 295 300

Ser Ser Phe Thr Leu Ile Ser Ser Gln Thr Gln Arg Phe Leu Ala Ser
305 310 315 320

Met Leu Ser Asn Arg Lys Phe Thr Glu Lys Tyr Ile Lys Met Asn Arg
325 330 335

Asp Arg Leu Lys Lys Arg Tyr Glu Met Ile Ile Glu Gly Leu Arg Thr
340 345 350

Ala Gly Ile Glu Cys Leu Glu Gly Asn Ala Gly Leu Phe Cys Trp Met
355 360 365

Asn Leu Ser Pro Leu Leu Lys Asp Lys Lys Thr Lys Glu Gly Glu Ile
370 375 380

Glu Ile Trp Lys Arg Ile Leu Lys Glu Val Lys Leu Asn Ile Ser Pro
385 390 395 400

Gly Ser Ser Cys His Cys Ser Glu Pro Gly Trp Phe Arg Val Cys Phe
405 410 415

Ala Asn Met Ser Glu Lys Thr Leu His Val Ala Leu Asp Arg Ile Arg
420 425 430

Arg Phe Met Glu Arg Met Lys Lys Glu Asn Glu Ala Asn
435 440 445

<210> 9
<211> 27
<212> DNA
<213> Cucumis melo

<400> 9
acattcaatt caacaaatct tcagttc 27

<210> 10
<211> 27
<212> DNA
<213> Cucumis melo

<400> 10
gggtatagta attacagtaa agagtgg 27